# EUROPEAN PATENT SPECIFICATION

(11) **EP 1 285 908 B1**
(45) Date of publication and mention of the grant of the patent: **10.09.2008**
(21) Application number: 01932262.7
(22) Date of filing: 25.05.2001
(51) Int. Cl.: C07C 69/734, C07C 69/738, C07C 229/34, C07C 233/55, C07C 233/81, C07C 235/44, C07C 235/78, A61K 31/192, A61P 43/00, A61P 3/06, A61P 9/10

(54) **SUBSTITUTED PHENYLPROPIONIC ACID DERIVATIVES**
SUBSTITUIERTE PHENYLPROPIONSÄURE-DERIVATE
DERIVES SUBSTITUES D'ACIDE PHENYLPROPIONIQUE

(30) Priority: 29.05.2000 JP 2000158424
(43) Date of publication of application: 26.02.2003
(73) Proprietor: Kyorin Pharmaceutical Co., Ltd., Chiyoda-ku, Tokyo 101-0062 (JP)
(72) Inventor: MIYACHI, Hiroyuki, Kazo-shi, Saitama 347-0063 (JP); NOMURA, Masahiro, Shimotsuga-gun, Tochigi 329-0101 (JP); TAKAHASHI, Yukie, Fukushima-shi, Fukushima 960-8056 (JP); TANASE, Takahiro, Shimotuga-gun, Tochigi 329-0101 (JP); MURAKAMI, Kouji, Oyama-shi, Tochigi 329-0207 (JP); SUZUKI, Masahiro, Kasukabe-shi, Saitama 329-0067 (JP)
(74) Representative: ter Meer, Nicolaus
(86) International application number: PCT/JP2001/004385
(87) International publication number: WO 2001/092201

(56) References cited:
- EP-A- 0 930 299
- WO-A1-00/75103
- WO-A1-01/25181
- WO-A1-97/36862
- ZHANG JUAN ET AL.: 'Polyphosphoric acid catalyzed conversion of 3-(methoxyphenyl) propionic acids to derivatives of (3.3) metacyclophane-1, 10-diones and 1-indanones' J. ORG. CHEM. vol. 58, no. 3, 1993, pages 556 - 559, XP002945092

## Description

### Technical field

The present invention relates to substituted phenylpropanoic acid derivatives, effective for the therapy of abnormality of lipometabolism as agonists of human peroxisome proliferator-activated receptor (abbreviated as PPAR), in particular, as agonists for human PPARα isoform, their addition salts, processes for preparing them, and medicinal compositions containing these compounds.

### Background technologies

The peroxisome proliferator-activated receptor(PPAR) is a ligand-dependent transcription factor that belongs to nuclear receptor superfamily such as steroid receptor, retinoid receptor, thyroid receptor, etc. Three isoforms (α type, β(or δ) type and γ type) with different histological distribution have been identified hitherto in human and various animal species (Proc. Natl. Acad. Sci., 1992, 89, 4653). Thereamong, the PPARα is distributed in the liver, kidney, etc. with high catabolic capacity for fatty acids and, in particular high expression is recognized in the liver(Endocrinology, 1995, 137, 354), positively or negatively controlling the expressions of genes relevant to the metabolism and the intracellular transport of fatty acids (e.g. acyl-CoA synthetic enzyme, fatty acid-binding protein and lipoprotein lipase) and apolipoprotein (AI, AII, CIII) genes relevant to the metabolisms of cholesterol and triglyceride. The PPARβ is expressed ubiquitously in the tissues of organisms, including nerve cells. At present, the physiological significance of PPARβ is unclear. The PPARγ is highly expressed in the adipocytes and involved the differentiation of adipocytes (J. Lipid Res., 1996, 37, 907). In this way, each isoform of PPAR plays specific functions in the particular organs and tissues.

Moreover, it is reported that a knock-out mouse of PPARα exhibits hypertriglyceridemia with ageing and becomes obesity mainly by increasing the white adipose tissues (J. Biol. Chem., 1998, 273, 29577), hence the relevance between activation of PPARα and decreasing action of lipids (cholesterol and triglyceride) in blood is suggested strongly.

On the other hand, fibrates and statins are widely used so far as the therapeutic drugs for hyperlipidemia. However, the fibrates have only weak decreasing effect of cholesterol, while the statins have weak decreasing effect of free fatty acids and triglycerides. Moreover, with respect to the fibrates, various adverse effects such as gastrointestinal injury, anthema, headache, hepatic disorder, renal disorder and biliary calculus are reported. The reason is considered to be due to that the fibrates exhibit extensive pharmacological function, hence the development of a therapeutic drug for hyperlipidemia with specific mechanism is desired.

When considering the present situation of such conventional therapeutic drugs for hyperlipidemia, and the role on the adjusting mechanism of lipid metabolism and the connection to the pathology of hyperlipidemia of transcription factor called PPARα, which has become clear until now, if a compound that binds directly as a ligand of PPARα, in particular, human PPARα and is capable of activating human PPARα could be created, the medicinal use thereof would be expected as a compound that exhibits the decreasing effect of lipids (both of cholesterol and triglyceride) in blood due to very specific mechanism.

For compounds having an affinity to PPARα as ligands of PPARα, eicosanoids in HETE (hydroxyeicosatetraenoic acid) group produced via oxidation with cytochrome P-450, in particular, 8-HETE, 8-HEPE, etc. are reported in addition to LTB4 being a metabolite of arachidonic acid (Proc. Natl. Acad. Sci., 1997, 94, 312). However, these endogenous unsaturated fatty acid derivatives are unstable metabolically and chemically and cannot be offered as medicinal drugs.

On the other hand, as compounds with similar structure to the inventive substituted phenylpropanoic acid derivatives, a group of compounds shown below, etc. are reported.

As α-substituted phenylpropanoic acid derivatives with blood glucose-decreasing action and lipid-decreasing action, in Japanese Unexamined Patent Publication No. Hei 11-158144 (SS Pharmaceutical Co., Ltd.), compounds represented by a general formula (A) 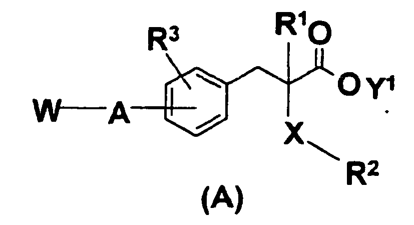 (wherein W denotes a (substituted) lactam ring, A denotes an alkylene group or alkyleneoxy group, X denotes O, S, NH or CH₂, Y¹ denotes an amino group, hydroxyl group or alkoxy group, R¹ denotes H, alkyl group, etc., R² denotes an alkyl group, phenyl group, etc., and R3 denotes an alkyl group, alkoxy group, etc.) are reported.

However, these compounds have different structure from that of the inventive compounds in the points that carbonyl group or amide group is not contained in A being connecting portion and that lactam ring is contained in W being end substituent, and it is not described that these compounds have the binding activity to human PPARα and the transcription-activating function.

As compounds with blood glucose-lowering action, in International Publication Number WO98/28254 (Nippon Chemiphar Co., Ltd.), compounds represented by a general formula (B) 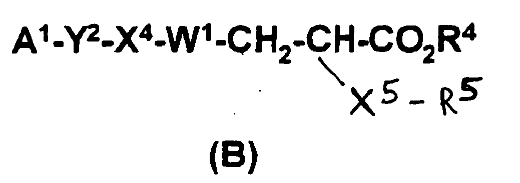 (wherein A¹ denotes an aryl group which may have substituents or heterocycle group, Y2 denotes an alkylene chain with carbon atoms of 1 to 5, X4 denotes bond hand, oxygen atom or sulfur atom, W¹ denotes a naphthalene ring which may have substituents, quinoline ring, indole ring, benzisoxazole ring or benzo[b]thiophene ring, R⁴ denotes a hydrogen atom or alkyl group with carbon atoms of 1 to 8, X⁵ denotes an oxygen atom or sulfur atom, and R⁵ denotes an alkyl group with carbon atoms of 1 to 8 which may have substituents, aralkyl group or aryl group), are reported.

However, these compounds have different structure from that of the inventive compounds in the points that carbonyl group or amide group is not contained in Y² and X⁴ being connecting portions and that W¹ to bind to 3-position of propionic acid is heterocycle, and it is not described that these compounds have the binding activity to human PPARα and the transcription-activating function.

As propanoic acid derivatives with blood glucose-lowering action and lipid-decreasing action, in International Publication Number WO98/07699 (Japan Tobacco Inc.), compounds represented by a general formula (C) 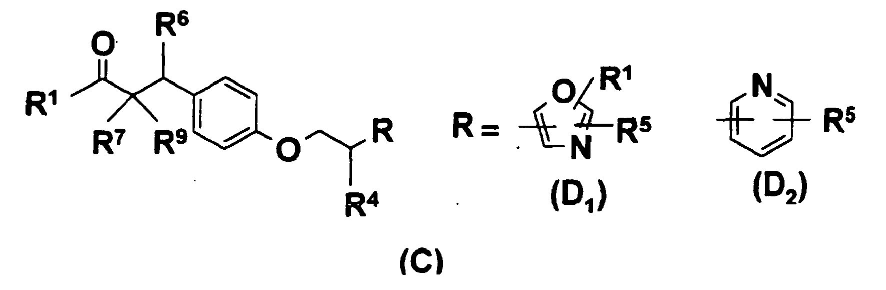 (wherein R denotes a substituent represented by D₁ or D₂, R¹ denotes an aromatic ring, cycloalkyl group or heteroaromatic ring, R⁵ denotes an alkyl group, R⁴ denotes a hydrogen atom or alkyl group, R⁶ denotes a hydrogen atom or it may be connected to R⁹ to form double bond, R⁷ denotes a carboxyl group, acyl group, alkoxycarbonyl group which may have substituents, alkyl group, aryloxycarbonyl group, aralkyloxycarbonyl group, carbamoyl group, NHR⁸ group or OR⁸ group, R⁸ denotes an acyl group which may have substituents or alkoxycarbonyl group, R⁹ denotes a hydrogen atom, alkyl group or alkoxycarbonyl group, and R¹⁰ denotes a hydrogen atom, amino group, alkoxy group, alkyl group, aryloxy group or aralkyloxy group), are reported.

However, these compounds also have different structure from that of the inventive compounds in the point that substituents on benzene ring are of disubstituted form at 1-position and 4-position, and it is not described that these compounds have the binding activity to human PPARα and the transcription-activating function.

As carboxylic acid derivatives with working function on leukotriene receptor, in Japanese Unexamined Patent Publication No. Sho 63-91354 (Yamanouchi Pharmaceutical Co., Ltd.), compounds represented by a general formula (E) 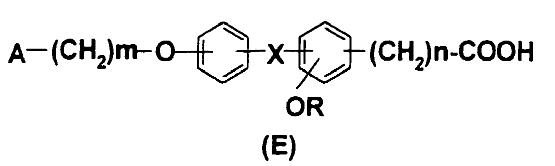 (wherein A denotes a hydrogen atom or phenyl group, m denotes an integer of 3 to 10, n denotes an integer of 1 to 6, X denotes CONH group or NHCO group, and R denotes a carboxy lower alkyl group or carboxy lower alkylcarbamoyl group (however, when A is phenyl group, R is carboxy lower alkylcarbamoyl lower alkyl group)), are reported.

However, since these compounds have no substituent at 2-position of propanoic acid and carbonyl groups exist in all of R group portions, the structure differs from that of the inventive compounds, and it is not described that these compounds have the binding activity to human PPARα and the transcription-activating function.

As carboxylic acid derivatives with antagonism against fibrinogen receptor, in US5227490 (Merck & Co., Inc.), compounds represented by a general formula (F) 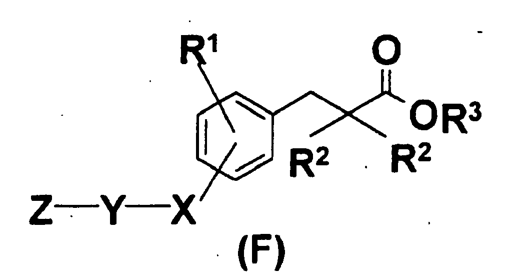 (wherein R¹ denotes a hydrogen atom, C₁₋₆ alkyl group, aryl C₄₋₁₀ alkyl group, aryl group, carboxyl group, C₁₋₆ alkoxy group, carboxy C₀₋₆ alkyl group, carboxy C₀₋₆ alkoxy group, hydroxy C₁₋₆ alkyl group, C₁₋₄ alkylsulfonyl C₀₋₆ alkyl group, C₀₋₄ alkylamino C₀₋₆ alkyl group, aryl C₀₋₁₀ alkylamino C₀₋₆ alkyl group, C₂₋₁₀ acylamino C₀₋₆ alkyl group, C₁₋₄ carboalkoxy C₀₋₆ alkyl group or halogen atom, R²s denote identically or differently hydrogen atoms, halogen atoms, hydroxyl groups, C₁₋₆ alkoxy groups, aryl C₀₋₄ alkyl groups, aryl C₀₋₆ alkoxy groups or C₁₋₆ alkyl groups which may have substituents, R³ denotes a hydrogen atom, C₁₋₆ alkyl group or aryl C₁₋₁₀ alkyl group, X denotes an oxygen atom, sulfur atom, SO group, SO₂ group, CO group, NR⁴CO group, C0NR⁴ group, CH₂ group, CH=CH group or NR⁴CS group, Y denotes a C₁₋₁₀ alkyl group which is unsubstituted or which may have substituents, C₄₋₈ cycloalkyl group, aryl group, C₀₋₃ alkylaryl C₀₋₃ alkyl group, C₀₋₃ alkylaryl C₀₋₃ alkylcarbonyl group, C₀₋₃ alkylaryl C₀₋₃ alkylcarboxyamide group, C₀₋₃ alkylaryloxy C₀₋₃ alkyl group, CONH group, NHCO group or (CH₂)m-Q-(CH₂)n group (however, Q denotes a C₃₋₈ membered heterocycle containing 1 to 3 kinds of heteroatoms selected from oxygen and sulfur, and m and n denote 0 to 4), and Z denotes a NR⁴R⁵ group (however, R⁴ and R⁵ denote identically or differently hydrogen atoms, C₁₋₆ alkyl groups, aryl C₁₋₁₀ alkyl groups in which alkyl group is unsubstituted or may be substituted with C₁₋₄ alkoxy group, carboxy C₀₋₆ alkyl group, hydroxyl group, halogen atom, or 4-9 membered monocyclic or bicyclic ring containing 1 to 3 heteroatoms selected from nitrogen, oxygen and sulfur) or guanidino group which may have substituents), are reported.

However, from the fact that these compounds are amino acid derivatives inevitably containing amino group which may have substituents, in all of Z group portions, the structure is different from that of the inventive compounds, and it is not described that these compounds have the binding activity to human PPARα and the transcription-activating function.

With respect to patents that report the working function on PPAR α, compounds represented by a general formula (G) 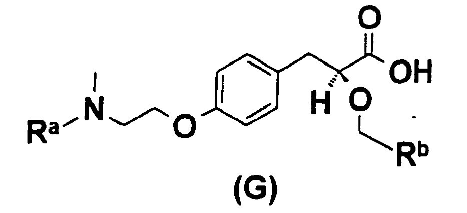 (wherein R^{a} denotes a 2-benzoxazolyl group or 2-pyridyl group, and R^{b} denotes a methoxymethyl group or trifluoromethyl group), are reported in International Open Number WO97/25042 (SmithKline Beecham plc.) as compounds with working functions on PPARα and PPARγ. However, the structure of these compounds is different from that of the inventive compounds in the point that substituents on benzene ring are of disubstituted derivatives at 1-position and 4-position, and further it is not described that they have the binding activity to human PPARα and the transcription-activating function.

As compounds with working function on PPARα, in International Publication Number WO97/36579 (Glaxo Welcome Corp.), compounds represented by a general formula (H) 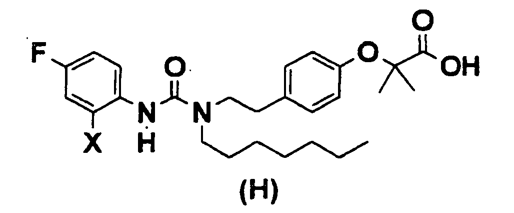 (wherein X denotes a hydrogen atom or fluorine atom), are reported.

However, the structure is different from that of the inventive compounds in the points that these compounds are phenoxyacetic acid derivatives and that the position relationship of substituents on benzene ring is of disubstituted form at 1-position and 4-position. Also, the transcription-activating function of PPARα is never satisfied in strength.

The hyperlipidemia is a risk factor of arterial sclerosis and, from a viewpoint of the prevention of arteriosclerotic diseases, in particular, coronary arteriosclerotic disease, the development of a therapeutic drug for hyperlipidemia with effectiveness and high safety is desired clinically.

In addition, a polyphosphoric acid catalyzed conversion of 3-(methoxyphenyl)propionic acids to derivatives of (3,3)metacyclophane-1.10-diones and 1-indanones were known in the art, see Zhang et al., J. Org. Chem. 1993, 58, 556. Furthermore, WO 01/25181 A1 discloses carboxylic acid compounds useful for prevention or treatment of hyperglycemia and hyperlipemia.

### Disclosure of the invention

As a result of diligent studies paying an attention to such specific role on the lipid metabolism of human PPARα, aiming at the creation of structurally novel drug with effectiveness and high safety as a therapeutic drug for hyperlipidemia, the inventors have found that novel substituted phenylpropanoic acid derivatives represented by a following general formula (1) have excellent binding activity to human PPARα and transcriptional activation and exhibit the lipid-decreasing action of lipids (cholesterol and triglyceride) in blood, leading to the completion of the invention.

Namely, the invention relates to substituted phenylpropanoic acid derivatives represented by a general formula (1) 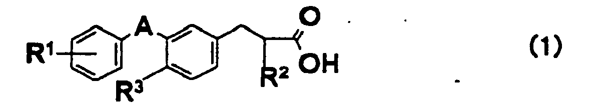 [wherein R¹ denotes a alkyl group with carbon atoms of 1 to 4, alkoxy group with carbon atoms of 1 to 3, trifluoromethyl group, trifluoromethoxy group, phenyl group which is unsubstituted or may have substituents, phenoxy group which is unsubstituted or may have substituents or benzyloxy group which is unsubstituted or may have substituents, R² denotes a hydrogen atom, alkyl group with carbon atoms of 1 to 4 or alkoxy group with carbon atoms of 1 to 3, R³ denotes a alkoxy group with carbon atoms of 1 to 3, and the binding mode of A portion denotes -CH₂NHCH₂-, -COCH₂O-, -OCH₂CO-, -COCH₂NH- or -NHCH_{2C}O-), their pharmaceutically acceptable salts and their hydrates.

The salts of the compounds represented by the general formula (1) in the invention are of common use and metal salts, for example, alkali metal salts (e.g. sodium salt, potassium salt, lithium salt, etc.), alkaline earth metal salts (e.g. calcium salt, magnesium salt, etc.), aluminum salt, and other pharmacologically acceptable salts are mentioned. Moreover, in the compounds represented by the general formula (1) in the invention, compounds capable of making acid addition salts can exist. As the acids in this case, pharmacologically acceptable inorganic acids, for example, hydrochloric acid, sulfuric acid, hydrobromic acid and phosphoric acid, and organic acids, for example, maleic acid, fumaric acid, acetic acid, oxalic acid, tartaric acid and benzenesulfonic acid, and the like are mentioned.

Moreover, the compounds represented by the general formula (1) in the invention sometimes include optical isomers based on the propanoic acid portion. Furthermore, compounds obtainable in the process of synthesizing the compounds represented by the general formula (1) sometimes include a mixture of geometrical isomers. Such isomers and their mixtures are all included in the scope of the invention.

Respective optical isomers can be prepared through stereoselective synthetic process. Moreover, they can also be prepared by separating diastereomeric ester derivatives or oxazolidinone derivatives obtainable by reacting with optically active alcohol derivatives or optically active oxazolidinone derivatives by a technique of fractional crystallization or chromatography, followed by hydrolysis. Furthermore, they can also be prepared by a technique of chromatography that uses chiral support.

In the general formula (1) of the invention, for "lower alkyl group with carbon atoms of 1 to 4", straight chain or branched ones with carbon atoms of 1 to 4 such as methyl, ethyl, propyl, isopropyl and butyl are mentioned.

For "lower alkoxy group with carbon atoms of 1 to 3", straight chain or branched ones with carbon atoms of 1 to 3 such as methoxy, ethoxy, isopropoxy and propoxy are mentioned.

For "halogen atoms", fluorine atom, chlorine atom, bromine atom and iodine atom are mentioned.

For substituents acceptable in "phenyl group which is unsubstituted or may have substituents, phenoxy group which is unsubstituted or may have substituents or benzyloxy group which is unsubstituted or may have substituents", lower alkyl group with carbon atoms of 1 to 4, lower alkoxy group with carbon atoms of 1 to 3, halogen atom, etc. are mentioned.

Generally, compounds of a general formula (1b), the binding mode of A portion being -CH₂CONH-, in the compounds of said general formula (1) can be prepared, for example, through following processes (Scheme 1).

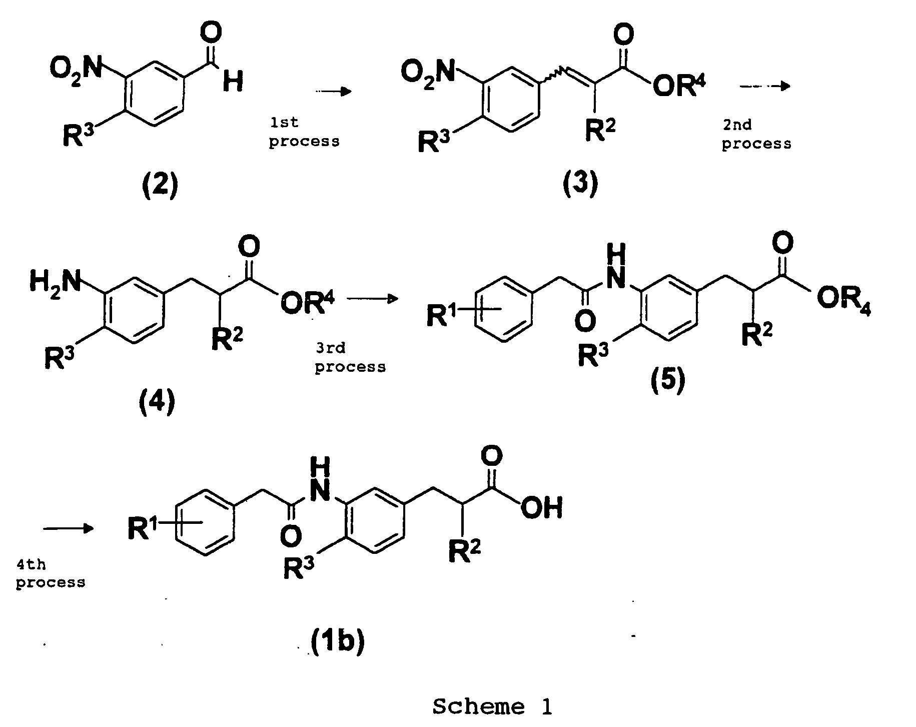

Namely, compounds represented by the general formula (1b) 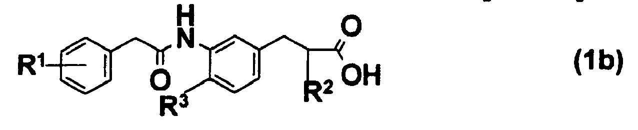 [wherein R¹ denotes a lower alkyl group with carbon atoms of 1 to 4, lower alkoxy group with carbon atoms of 1 to 3, trifluoromethyl group, trifluoromethoxy group, phenyl group which is unsubstituted or may have substituents, phenoxy group which is unsubstituted or may have substituents or benzyloxy group which is unsubstituted or may have substituents, R² denotes a hydrogen atom, lower alkyl group with carbon atoms of 1 to 4 or lower alkoxy group with carbon atoms of 1 to 3, and R³ denotes a lower alkoxy group with carbon atoms of 1 to 3], can be prepared by reacting (Wittig reaction or Horner-Emmons reaction; first process) compounds represented by a general formula (2) 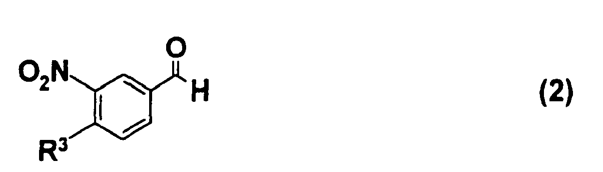 [wherein R³ denotes a lower alkoxy group with carbon atoms of 1 to 3], with compounds represented by a general formula (6) 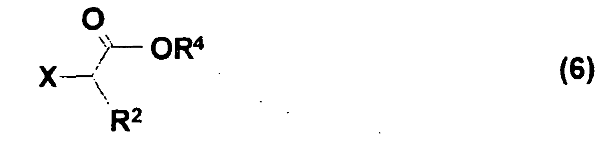 [wherein R² is as described above, R⁴ is a lower alkyl group with carbon atoms of 1 to 4, and X denotes a PPh₃ group or PO(OC₂H₅)₂ group], in the presence of base, to synthesize compounds represented by a general formula (3) 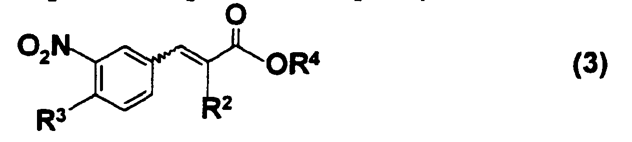 [wherein R², R³ and R⁴ are as described above], by reducing and hydrogenolyzing (second process) these compounds, to obtain compounds represented by a general formula (4) 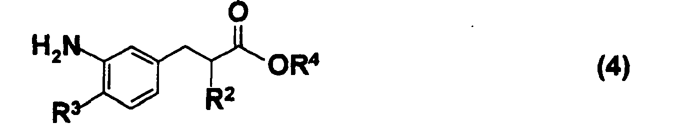 [wherein R², R³ and R⁴ are as described above], by reacting (third process) these compounds with compounds represented by a general formula (7) 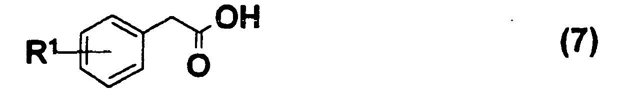 [wherein R¹ denotes a lower alkyl group with carbon atoms of 1 to 4, lower alkoxy group with carbon atoms of 1 to 3, trifluoromethyl group, trifluoromethoxy group, phenyl group which is unsubstituted or may have substituents, phenoxy group which is unsubstituted or may have substituents or benzyloxy group which is unsubstituted or may have substituents], to obtain compounds represented by a general formula (5) 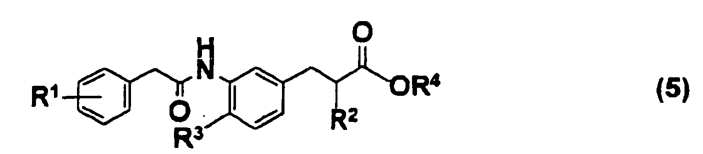 [wherein R¹, R², R³ and R⁴ are as described above] and by hydrolyzing (fourth process) COOR⁴ position of these compounds.

In the reaction of the first process, as a base, for example, alkali metal hydride such as sodium hydride, organometallic compound such as butyl lithium, metal amide such as lithium diisopropylamide, or metal alkoxide such as sodium methoxide or potassium t-butoxide can be used in a solvent such as tetrahydrofuran, toluene, dioxane or N,N-dimethylformamide. The reaction can be performed at a reaction temperature of -20°C to 150°C, preferably 0°C to 50°C.

The reduction reaction of the second process can be performed at a hydrogen pressure of 98.1kPa to 491kPa in a solvent such as ethanol, methanol, tetrahydrofuran, ethyl acetate or N,N-dimethylformamide in the presence of metallic catalyst such as palladium on carbon, platinum on carbon, platinum oxide or rhodium on alumina. The reaction can be performed at a reaction temperature of 0°C to 100°C, preferably room temperature to 80°C.

The condensation reaction of the third process can be performed by leaving carboxyl group as it is or converting it to reactive derivatives.

As the "reactive derivative group of carboxyl group", acid chloride, acid bromide, acid anhydride, carbonylimidazole or the like is mentioned. In the case of the reaction using reactive derivative, the reaction can be performed in a solvent such as dioxane or N,N-dimethylformamide in the presence or absence of, for example, alkali metal hydride such as sodium hydride, alkali metal hydroxide such as sodium hydroxide, alkali metal carbonate such as potassium carbonate, or organic base such as pyridine or triethylamine as a base.

In the case of conducting the reaction by leaving carboxylic acid form as it is, the reaction can be performed in a solvent such as methylene chloride, chloroform, dioxane or N,N-dimethylformamide in the presence of condensing agent in the presence or absence of base, and further in the presence or absence of additive.

As the condensing agent, for example, dicyclohexylcarbodiimide, 1-[3-(dimethylamino)propyl]-3-ethylcarbodiimide hydrochloride, diethyl cyanophosphate, diphenylphosphoric azide, carbonyldiimidazole or the like can be mentioned. As the base, for example, alkali metal hydroxide such as sodium hydroxide, alkali metal carbonate such as potassium carbonate, or organic base such as pyridine or triethylamine can be mentioned. As the additive, N-hydroxybenzotriazole, N-hydroxysuccinimide, 3,4-dihydro-3-hydroxy-4-oxo-1,2,3-benzotriazine or the like can be mentioned. The reaction can be performed at a reaction temperature of -20°C to 100°C, preferably 0°C to 50°C.

The hydrolysis reaction of the fourth process can be performed under alkaline condition. For the alkaline condition, lithium hydroxide, sodium hydroxide, potassium hydroxide or the like is used. The reaction can be performed at a reaction temperature of 0°C to 80°C, preferably room temperature to 60°C.

Moreover, compounds of the general formula (1b), the binding mode of A portion being -CH₂CONH-, in the compounds of said general formula (1) can be prepared, for example, through following processes (Scheme 2).

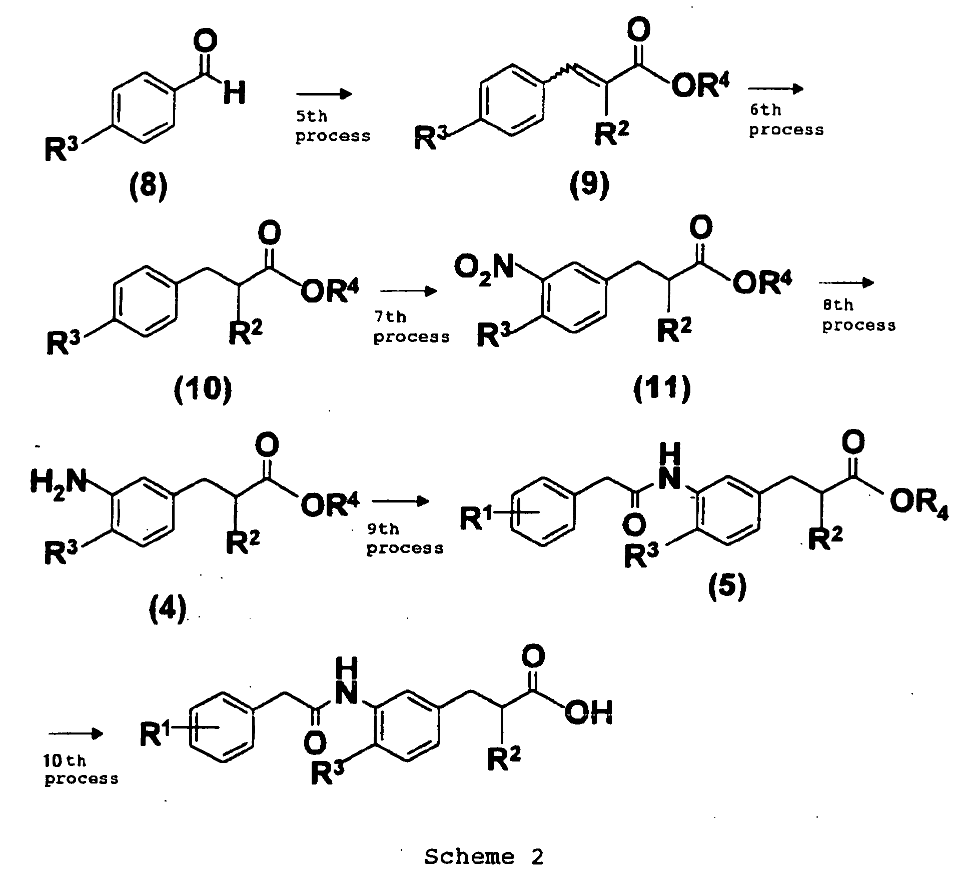

Namely, compounds represented by the general formula (1b) 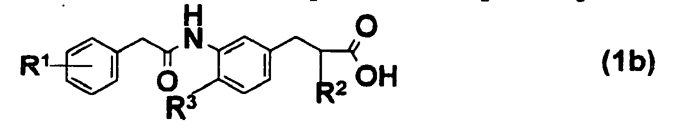 [wherein R¹ denotes a lower alkyl group with carbon atoms of 1 to 4, lower alkoxy group with carbon atoms of 1 to 3, trifluoromethyl group, trifluoromethoxy group, phenyl group which is unsubstituted or may have substituents, phenoxy group which is unsubstituted or may have substituents or benzyloxy group which is unsubstituted or may have substituents, R² denotes a hydrogen atom, lower alkyl group with carbon atoms of 1 to 4 or lower alkoxy group with carbon atoms of 1 to 3, and R³ denotes a lower alkoxy group with carbon atoms of 1 to 3], can be prepared by reacting (fifth process) compounds represented by a general formula (8) 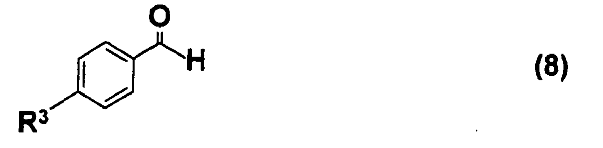 [wherein R³ denotes a lower alkoxy group with carbon atoms of 1 to 3], with compounds represented by the general formula (6) 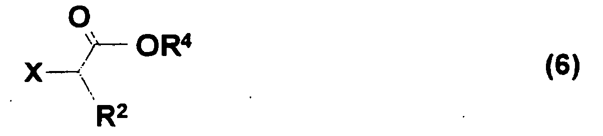 [wherein R² is as described above, R⁴ is a lower alkyl group with carbon atoms of 1 to 4, and X denotes PPh₃ or PO(OC₂H₅)₂], in the presence of base, to synthesize compounds represented by a general formula (9) 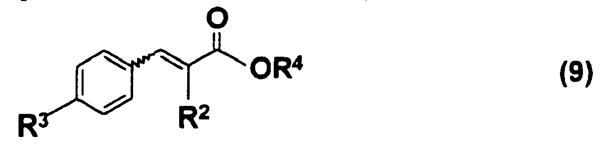 [wherein R², R³ and R⁴ are as described above], by reducing (sixth process) these compounds, to synthesize compounds represented by a general formula (10) 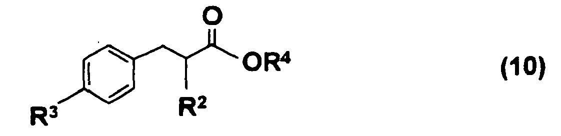 [wherein R², R³ and R⁴ are as described above], by nitrating (seventh process) these compounds to synthesize compounds represented by a general formula (11) 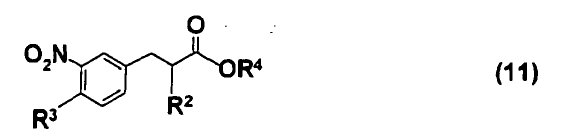 [wherein R², R³ and R⁴ are as described above], by reducing these compounds to synthesize compounds represented by the general formula (4) 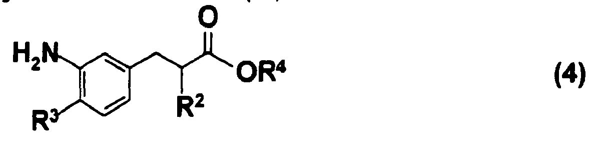 [wherein R², R³ and R⁴ are as described above], by reacting these compounds with compounds represented by the general formula (7) 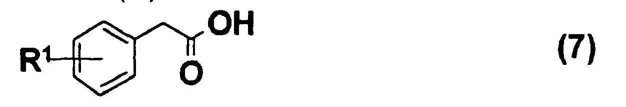 [wherein R¹ denotes a lower alkyl group with carbon atoms of 1 to 4, lower alkoxy group with carbon atoms of 1 to 3, trifluoromethyl group, trifluoromethoxy group, phenyl group which is unsubstituted or may have substituents, phenoxy group which is unsubstituted or may have substituents or benzyloxy group which is unsubstituted or may have substituents], to synthesize compounds represented by the general formula (5) 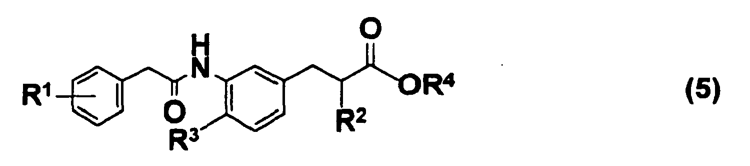 [wherein R¹, R², R³ and R⁴ are as described above], and by hydrolyzing (tenth process) COOR⁴ portion of these compounds.

In the reaction of the fifth process, as a base, for example, alkali metal hydride such as sodium hydride, organometallic compound such as butyl lithium, metal amide such as lithium diisopropylamide, or metal alkoxide such as sodium methoxide or potassium t-butoxide can be used in a solvent such as tetrahydrofuran, toluene, dioxane or N,N-dimethylformamide. The reaction can be performed at a reaction temperature of -20°C to 150°C, preferably 0°C to 50°C.

The reaction of the sixth process can be performed at a hydrogen pressure of 98.1kPa to 491kPa in a solvent such as ethanol, methanol, tetrahydrofuran, ethyl acetate or N,N-dimethylformamide in the presence of metallic catalyst such as palladium on carbon, platinum on carbon, platinum oxide or rhodium on alumina. The reaction can be performed at a reaction temperature of 0°C to 100°C, preferably room temperature to 80°C.

The reaction of the seventh process can be performed in nitric acid or a mixed solvent such as nitric acid and sulfuric acid or nitric acid and acetic anhydride. The reaction can be implemented at a reaction temperature of -20°C to 150°C, preferably 0°C to 100°C.

The reduction reaction of the eighth process can be performed at a hydrogen pressure of 98.1kPa to 491kPa in a solvent such as ethanol, methanol, tetrahydrofuran, ethyl acetate or N,N-dimethylformamide in the presence of metallic catalyst such as palladium on carbon, platinum on carbon, platinum oxide or rhodium on alumina. The reaction can be performed at a reaction temperature of 0°C to 100°C, preferably room temperature to 80°C.

The condensation reaction of the ninth process can be performed by leaving carboxyl group as it is or converting it to reactive derivatives.

As the "reactive derivative group of carboxyl group", acid chloride, acid bromide, acid anhydride, carbonylimidazole or the like is mentioned. In the case of the reaction using reactive derivative, the reaction can be performed in a solvent such as dioxane or N,N-dimethylformamide in the presence or absence of, for example, alkali metal hydride such as sodium hydride, alkali metal hydroxide such as sodium hydroxide, alkali metal carbonate such as potassium carbonate, or organic base such as pyridine or triethylamine as a base.

In the case of conducting the reaction by leaving carboxylic acid form as it is, the reaction can be performed in a solvent such as methylene chloride, chloroform, dioxane or N,N-dimethylformamide in the presence of condensing agent in the presence or absence of base, and further in the presence or absence of additive.

As the condensing agent, for example, dicyclohexylcarbodiimide, 1-[3-(dimethylamino)propyl]-3-ethylcarbodiimide hydrochloride, diethyl cyanophosphate, diphenylphosphoric azide, carbonyldiimidazole or the like can be mentioned. As the base, for example, alkali metal hydroxide such as sodium hydroxide, alkali metal carbonate such as potassium carbonate, or organic base such as pyridine or triethylamine can be mentioned. As the additive, N-hydroxybenzotriazole, N-hydroxysuccinimide, 3,4-dihydro-3-hydroxy-4-oxo-1,2,3-benzotriazine or the like can be mentioned. The reaction can be performed at a reaction temperature of -20°C to 100°C, preferably 0°C to 50°C.

The hydrolysis reaction of the tenth process can be performed under alkaline condition. For the alkaline condition, lithium hydroxide, sodium hydroxide, potassium hydroxide or the like is used. The reaction can be performed at a reaction temperature of 0°C to 80°C, preferably room temperature to 60°C.

Next, compounds of a general formula (1c), the binding mode of A portion being -NHCOCH₂-, in the compounds of said general formula (1) can be prepared through following processes (Scheme 3).

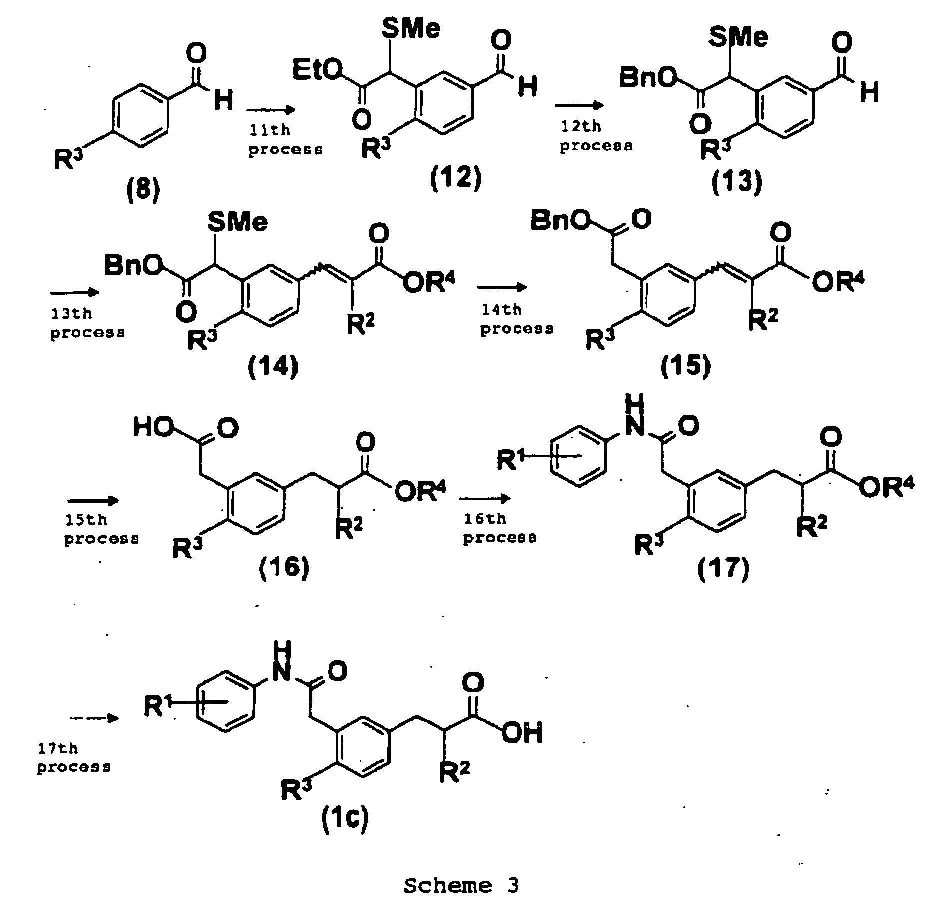

Namely, compounds represented by the general formula (1c) 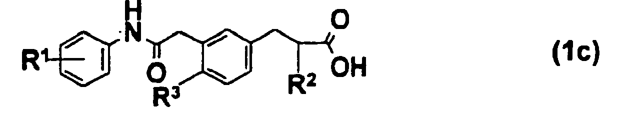 [wherein R¹ denotes a lower alkyl group with carbon atoms of 1 to 4, lower alkoxy group with carbon atoms of 1 to 3, trifluoromethyl group, trifluoromethoxy group, phenyl group which is unsubstituted or may have substituents, phenoxy group which
is unsubstituted or may have substituents or benzyloxy group which is unsubstituted or may have substituents, R² denotes a hydrogen atom, lower alkyl group with carbon atoms of 1 to 4 or lower alkoxy group with carbon atoms of 1 to 3, and R³ denotes a lower alkoxy group with carbon atoms of 1 to 3], can be prepared by reacting (eleventh process) compounds represented by the general formula (8) 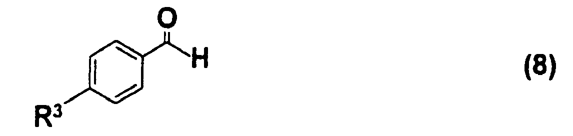 [wherein R³ denotes a lower alkoxy group with carbon atoms of 1 to 3], with known ethyl 2-chloro-2-methylthioacetate (e.g. Chem. Pharm. Bull., 1982, 30, 915), to synthesize compounds represented by a general formula (12) 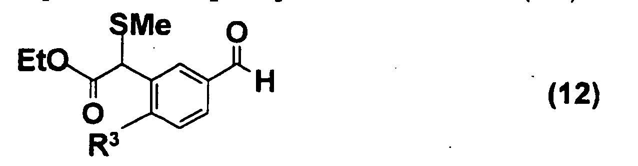 [wherein R³ is as described above], by hydrolyzing these compounds and then reacting (twelfth process) carboxylic acid derivatives obtained with benzyl bromide, to synthesize compounds represented by a general formula (13) 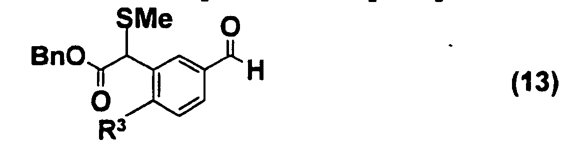 [wherein R³ is as described above], by reacting (thirteenth process) these compounds with compounds represented by the general formula (6) 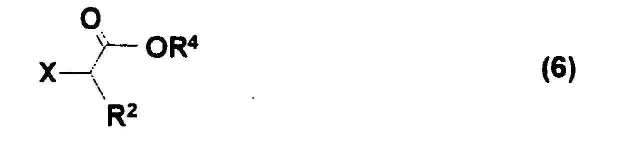 [wherein R² is as described above, R⁴ denotes a lower alkyl group with carbon atoms of 1 to 4, and X denotes PPh₃ or PO(OC₂H₅)₂], in the presence of base, to synthesize compounds represented by a general formula (14) 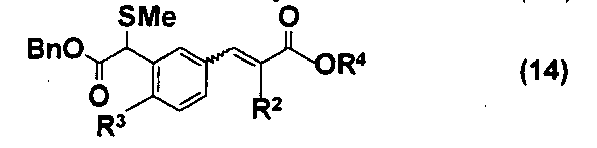 [wherein R², R³ and R⁴ are as described above], by removing (fourteenth process) methylthio group of these compounds, to synthesize compounds represented by a general formula (15) 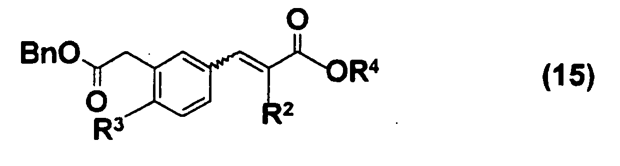 [wherein R², R³ and R⁴ are as described above], by reducing and hydrogenating (fifteenth process) these compounds, to synthesize compounds represented by a general formula (16) 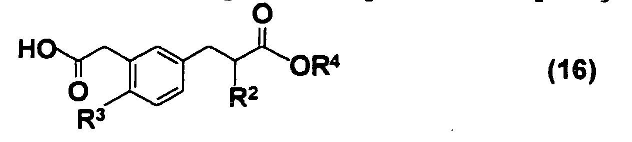 [wherein R², R³ and R⁴ are as described above], by reacting (sixteenth process) these compounds with compounds represented by a general formula (18) 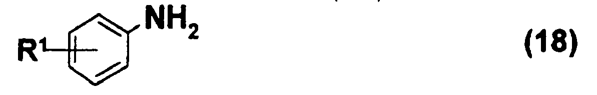 [wherein R¹ denotes a lower alkyl group with carbon atoms of 1 to 4, lower alkoxy group with carbon atoms of 1 to 3, trifluoromethyl group, trifluoromethoxy group, phenyl group which is unsubstituted or may have substituents, phenoxy group which is unsubstituted or may have substituents or benzyloxy group which is unsubstituted or may have substituents], to synthesize compounds represented by a general formula (17) 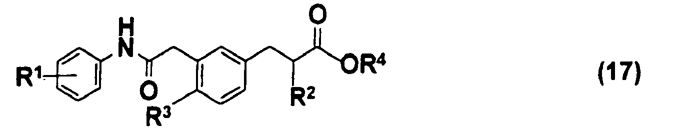 [wherein R¹, R², R³ and R⁴ are as described above], and by hydrolyzing (seventeenth process) COOR⁴ portion of these compounds.

The reaction of the eleventh process can be performed in a solvent such as methylene chloride, chloroform or nitrobenzene. As a Lewis acid, aluminum chloride, tin chloride, boron trifluoride or the like can be used. The reaction can be performed at a reaction temperature of -20°C to 150°C, preferably refluxing temperature of solvent.

In the reaction of the twelfth process, first, the hydrolysis reaction can be performed under alkaline condition. For the alkaline condition, lithium hydroxide, sodium hydroxide, potassium hydroxide or the like is used. The reaction can be performed at a reaction temperature of 0°C to 80°C, preferably room temperature to 60°C. Next benzyl esterification reaction can be performed in a solvent such as dioxane, N,N-dimethylformamide or dimethyl sulfoxide in the presence or absence of, for example, alkali metal hydride such as sodium hydride, alkali metal hydroxide such as sodium hydroxide, alkali metal carbonate such as potassium carbonate, or organic base such as pyridine or triethylamine as a base. The reaction can be performed at a reaction temperature of 0°C to 150°C, preferably room temperature to 60°C.

In the reaction of the thirteenth process, as a base, for example, alkali metal hydride such as sodium hydride, organometallic compound such as butyl lithium, metal amide such as lithium diisopropylamide, or metal alkoxide such as sodium methoxide or potassium t-butoxide can be used in a solvent such as tetrahydrofuran, toluene, dioxane or N,N-dimethylformamide. The reaction can be performed at a reaction temperature of -20°C to 150°C, preferably 0°C to 50°C.

The reaction of the fourteenth process can be performed in a solvent such as acetic acid or hydrochloric acid by reacting metallic zinc, zinc amalgam or zinc-copper alloy. The reaction can be performed at a reaction temperature of -10°C to 100°C, preferably 0°C to room temperature.

The reduction reaction of the fifteenth process can be performed at a hydrogen pressure of 98.1kPa to 491kPa in a solvent such as ethanol, methanol, tetrahydrofuran, ethyl acetate or N,N-dimethylformamide in the presence of metallic catalyst such as palladium on carbon, platinum on carbon, platinum oxide or rhodium on alumina. The reaction can be performed at a reaction temperature of 0°C to 100°C, preferably room temperature to 80°C.

The condensation reaction of the sixteenth process can be performed by leaving carboxyl group as it is or converting it to reactive derivatives.

As the "reactive derivative group of carboxyl group", acid chloride, acid bromide, acid anhydride, carbonylimidazole or the like is mentioned. In the case of the reaction using reactive derivative, the reaction can be performed in a solvent such as dioxane or N,N-dimethylformamide in the presence or absence of, for example, alkali metal hydride such as sodium hydride, alkali metal hydroxide such as sodium hydroxide, alkali metal carbonate such as potassium carbonate, or organic base such as pyridine or triethylamine as a base.

In the case of conducting the reaction by leaving carboxylic acid form as it is, the reaction can be performed in a solvent such as methylene chloride, chloroform, dioxane or N,N-dimethylformamide in the presence of condensing agent in the presence or absence of base, and further in the presence or absence of additive.

As the condensing agent, for example, dicyclohexylcarbodiimide,1-[3-(dimethylamino)propyl]-3-ethylcarbodiimide hydrochloride, diethyl cyanophosphate, diphenylphosphoric azide, carbonyldiimidazole or the like can be mentioned. As the base, for example, alkali metal hydroxide such as sodium hydroxide, alkali metal carbonate such as potassium carbonate, or organic base such as pyridine or triethylamine can be mentioned. As the additive, N-hydroxybenzotriazole, N-hydroxysuccinimide, 3,4-dihydro-3-hydroxy-4-oxo-1,2,3-benzotriazine or the like can be mentioned. The reaction can be performed at a reaction temperature of -20°C to 100°C, preferably 0°C to 50°C.

The hydrolysis reaction of the seventeenth process can be performed under alkaline condition. For the alkaline condition, lithium hydroxide, sodium hydroxide, potassium hydroxide or the like is used. The reaction can be performed at a reaction temperature of 0°C to 80°C, preferably room temperature to 60°C.

Moreover, compounds of general formulae (1d) and (1e), the binding modes of A portion being -CH₂CH₂CO- and -CH₂CH₂CH₂-, in the compounds of said general formula (1) can be prepared, for example, through following processes (Scheme 4).

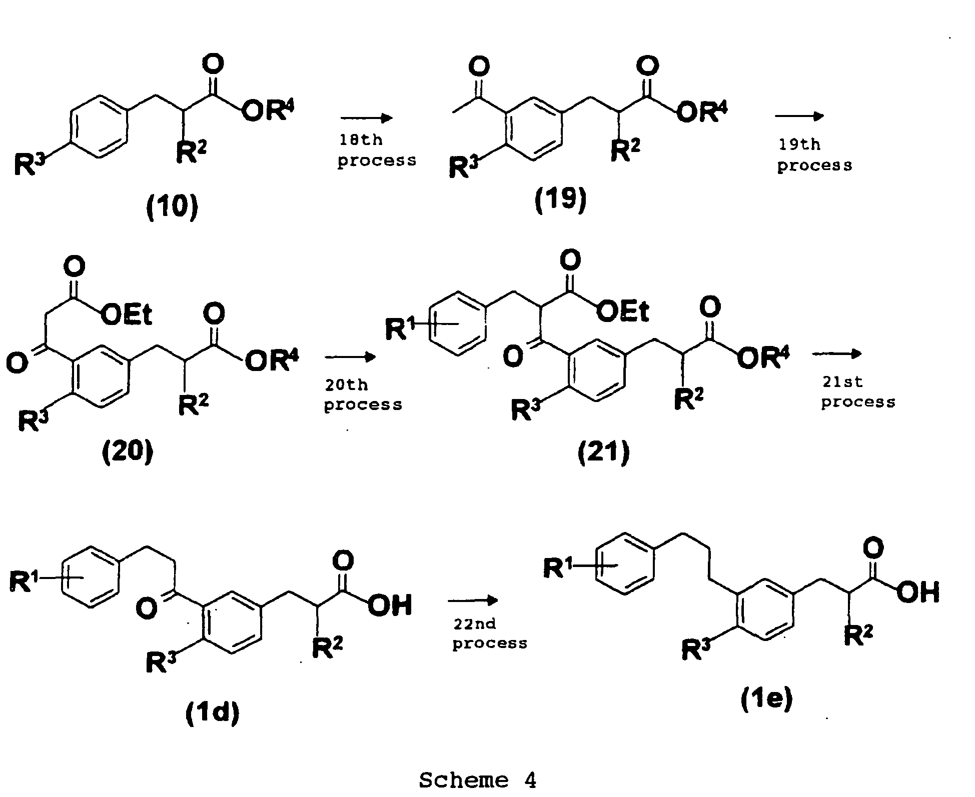

Namely, compounds represented by the general formula (1d) 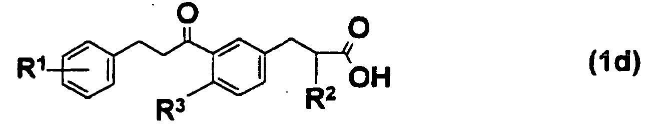 [wherein R¹ denotes a lower alkyl group with carbon atoms of 1 to 4, lower alkoxy group with carbon atoms of 1 to 3, trifluoromethyl group, trifluoromethoxy group, phenyl group which is unsubstituted or may have substituents, phenoxy group which is unsubstituted or may have substituents or benzyloxy group which is unsubstituted or may have substituents, R² denotes a hydrogen atom, lower alkyl group with carbon atoms of 1 to 4 or lower alkoxy group with carbon atoms of 1 to 3, and R³ denotes a lower alkoxy group with carbon atoms of 1 to 3], and compounds represented by the general formula (1e) 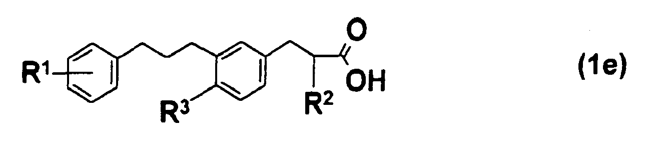 [wherein R¹ denotes a lower alkyl group with carbon atoms of 1 to 4, lower alkoxy group with carbon atoms of 1 to 3, trifluoromethyl group, trifluoromethoxy group, phenyl group which is unsubstituted or may have substituents, phenoxy group which is unsubstituted or may have substituents or benzyloxy group which is unsubstituted or may have substituents, R² denotes a hydrogen atom, lower alkyl group with carbon atoms of 1 to 4 or lower alkoxy group with carbon atoms of 1 to 3, and R³ denotes a lower alkoxy group with carbon atoms of 1 to 3], can be prepared by reacting (eighteenth process) compounds represented by the general formula (10) 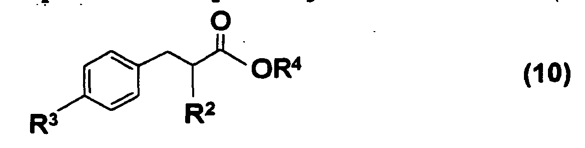 [wherein R² denotes a hydrogen atom, lower alkyl group with carbon atoms of 1 to 4 or lower alkoxy group with carbon atoms of 1 to 3, R³ denotes a lower alkoxy group with carbon atoms of 1 to 3, and R⁴ denotes a lower alkyl group with carbon atoms of 1 to 4], with acetyl chloride in the presence of Lewis acid, to synthesize compounds represented by a general formula (19) 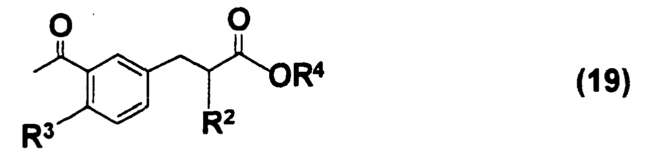 [wherein R², R³ and R⁴ are as described above], by reacting (nineteenth process) these compounds with diethyl carbonate in the presence of base, to synthesize compounds represented by a general formula (20) 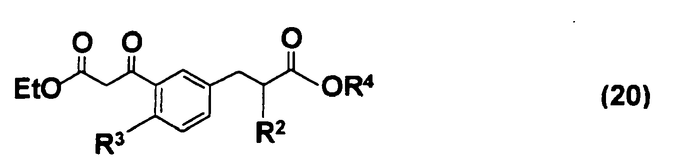 [wherein R², R³ and R⁴ are as described above], by reacting (twentieth process) these compounds with compounds represented by a general formula (22) 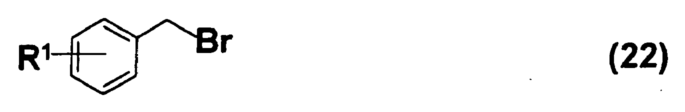 [wherein R¹ denotes a lower alkyl group with carbon atoms of 1 to 4, lower alkoxy group with carbon atoms of 1 to 3, trifluoromethyl group, trifluoromethoxy group, phenyl group which is unsubstituted or may have substituents, phenoxy group which is unsubstituted or may have substituents or benzyloxy group which is unsubstituted or may have substituents], in the presence of base, to synthesize compounds represented by a general formula (21) 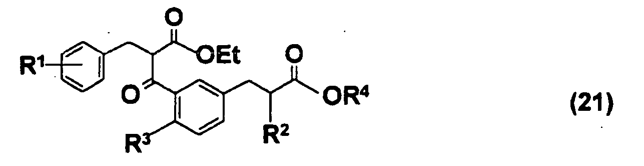 [wherein R¹, R², R³ and R⁴ are as described above], and by hydrolyzing and decarboxylating (twenty-first process) these compounds, thereby preparing compounds represented by the general formula (1d), and further, by hydrogenating (twenty-second process) compounds represented by the general formula (1d), thereby preparing compounds represented by the general formula (1e).

The reaction of the eighteenth process can be performed in a solvent such as methylene chloride, chloroform or nitrobenzene. As a Lewis acid, aluminum chloride, tin chloride, boron trifluoride or the like can be used. The reaction can be performed at a reaction temperature of -20°C to 150°C, preferably 0°C to refluxing temperature of solvent.

In the reaction of the nineteenth process, as a base, for example, alkali metal hydride such as sodium hydride, organometallic compound such as butyl lithium, metal amide such as lithium diisopropylamide, or metal alkoxide such as sodium methoxide or potassium t-butoxide can be used in a solvent such as tetrahydrofuran, toluene, dioxane, ethanol or N,N-dimethylformamide, or a mixed solvent thereof. The reaction can be performed at a reaction temperature of -20°C to 150°C, preferably 0°C to refluxing temperature of solvent.

In the reaction of the twentieth process, as a base, for example, alkali metal hydride such as sodium hydride, organometallic compound such as butyl lithium, metal amide such as lithium diisopropylamide, or metal alkoxide such as sodium methoxide or potassium t-butoxide can be used in a solvent such as tetrahydrofuran, toluene, dioxane or N,N-dimethylformamide. The reaction can be performed at a reaction temperature of -20°C to 150°C, preferably 0°C to refluxing temperature of solvent.

The reaction of the twenty-first process can be performed in a solvent such as acetic acid, hydrochloric acid or sulfuric acid, or a mixed solvent thereof, or a mixed solvent with organic solvent such as ethanol. The reaction can be performed at a reaction temperature of room temperature to 150°C.

The reduction reaction of the twenty-second process can be performed at a hydrogen pressure of 98.1kPa to 491kPa in a solvent such as ethanol, methanol, tetrahydrofuran, ethyl acetate or N,N-dimethylformamide in the presence of metallic catalyst such as palladium on carbon, platinum on carbon, platinum oxide or rhodium on alumina. The reaction can be performed at a reaction temperature of 0°C to 100°C, preferably room temperature to 80°C.

Moreover, compounds of general formula (1f), the binding mode of A portion being -CH₂CH₂O-, in the compounds of said general formula (1) can be prepared, for example, through following processes (Scheme 5).

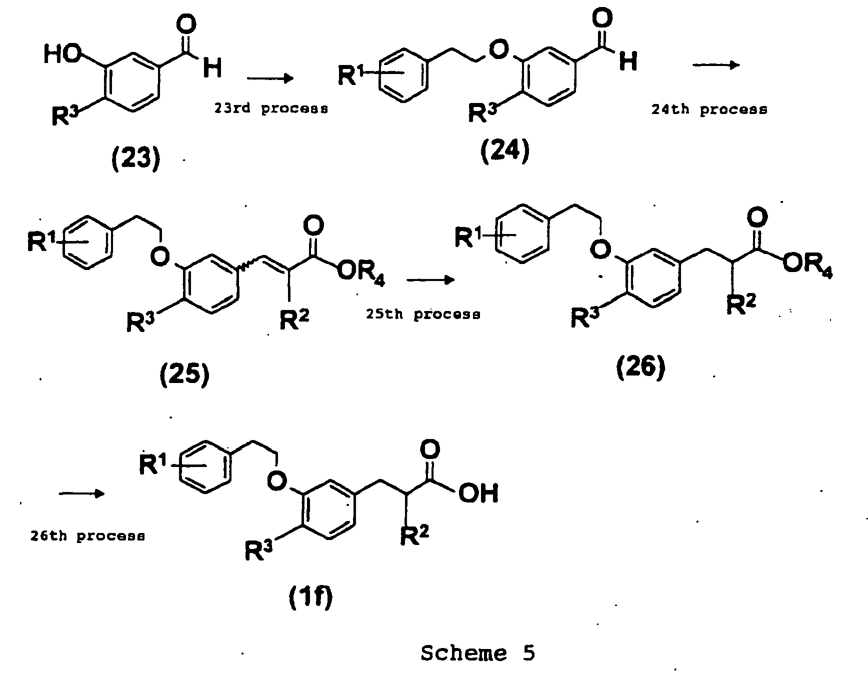

Namely, compounds represented by the general formula (1f) 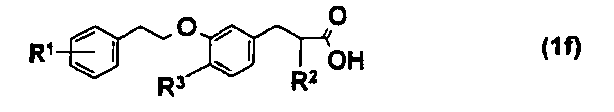 [wherein R¹ denotes a lower alkyl group with carbon atoms of 1 to 4, lower alkoxy group with carbon atoms of 1 to 3,
trifluoromethyl group, trifluoromethoxy group, phenyl group which is unsubstituted or may have substituents, phenoxy group which is unsubstituted or may have substituents or benzyloxy group which is unsubstituted or may have substituents, R² denotes a hydrogen atom, lower alkyl group with carbon atoms of 1 to 4 or lower alkoxy group with carbon atoms of 1 to 3, and R³ denotes a lower alkoxy group with carbon atoms of 1 to 3], can be prepared by reacting (twenty-third process) compounds represented by a general formula (23) 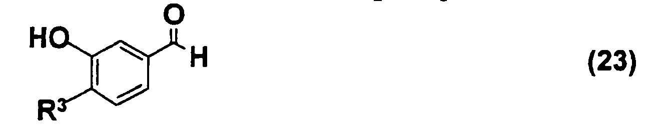 [wherein R³ denotes a lower alkoxy group with carbon atoms of 1 to 3], with compounds represented by a general formula (27) 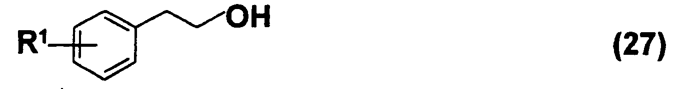 [wherein R¹ denotes a lower alkyl group with carbon atoms of 1 to 4, lower alkoxy group with carbon atoms of 1 to 3, trifluoromethyl group, trifluoromethoxy group, phenyl group which is unsubstituted or may have substituents, phenoxy group which is unsubstituted or may have substituents or benzyloxy group which is unsubstituted or may have substituents], to synthesize compounds represented by a general formula (24) 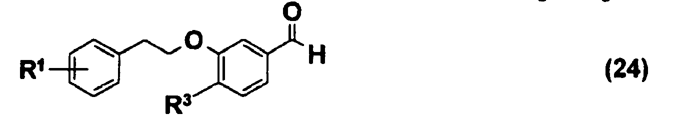 [wherein R¹ and R³ are as described above], by reacting
(twenty-fourth process) these compounds with compounds represented by the general formula (6) 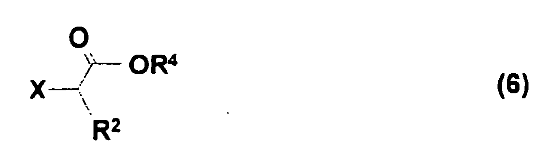 [wherein R² is as described above, R⁴ denotes a lower alkyl group with carbon atoms of 1 to 4, and X denotes PPh₃ or PO(OC₂H₅)₂], in the presence of base, to synthesize compounds represented by a general formula (25) 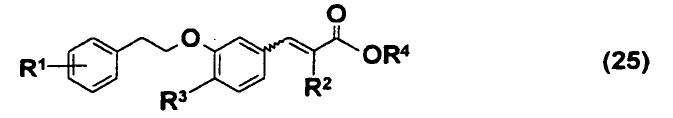 [wherein R¹, R², R³ and R⁴ are as described above], by reducing (twenty-fifth process) the double bond of these compounds, to synthesize compounds represented by a general formula (26) 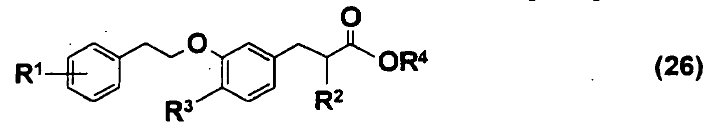 [wherein R¹, R², R³ and R⁴ are as described above], and by hydrolyzing (twenty-sixth process) COOR⁴ portion of these compounds.

The reaction of the twenty-third process can be performed in a solvent such as ether, tetrahydrofuran, dioxane, benzene or toluene, in the presence of base, triphenylphosphine, tri(o-tolylphosphine) and diethyl azodicarboxylate or diisopropyl azodicarboxylate (Mitsunobu reaction). The reaction can be performed at a reaction temperature of -20°C to 150°C, preferably 0°C to 100°C.

The reaction of the twenty-fourth process can be performed in a solvent such as ether, tetrahydrofuran or dioxane in the presence of base. As the base, for example, alkali metal hydride such as sodium hydride, organometallic compound such as butyl lithium, metal amide such as lithium diisopropylamide, or metal alkoxide such as sodium methoxide or potassium t-butoxide can be used. The reaction can be performed at a reaction temperature of -20°C to 150°C, preferably 0°C to 50°C.

The reaction of the twenty-fifth process can be performed at a hydrogen pressure of 98.1kPa to 491kPa in a solvent such as ethanol, methanol, tetrahydrofuran, ethyl acetate or N,N-dimethylformamide in the presence of metallic catalyst such as palladium on carbon, platinum on carbon, platinum oxide or rhodium on alumina. The reaction can be performed at a reaction temperature of 0°C to 100°C, preferably room temperature to 80°C.

The hydrolysis reaction of the twenty-sixth process can be implemented under alkaline condition. For the alkaline condition, lithium hydroxide, sodium hydroxide, potassium hydroxide or the like is used. The reaction can be implemented at a reaction temperature of 0°C to 80°C, preferably room temperature to 60°C.

Generally, compounds of general formula (1h), the binding mode of A portion being -CONHCH₂, in the compounds of said general formula (1) can be prepared, for example, through following processes (Scheme 6).

Moreover, according to the invention, compounds of general formula (1g), the binding mode of A portion being -CH₂NHCH₂-, in the compounds of said general formula (1) can be prepared, for example, through following processes (Scheme 6).

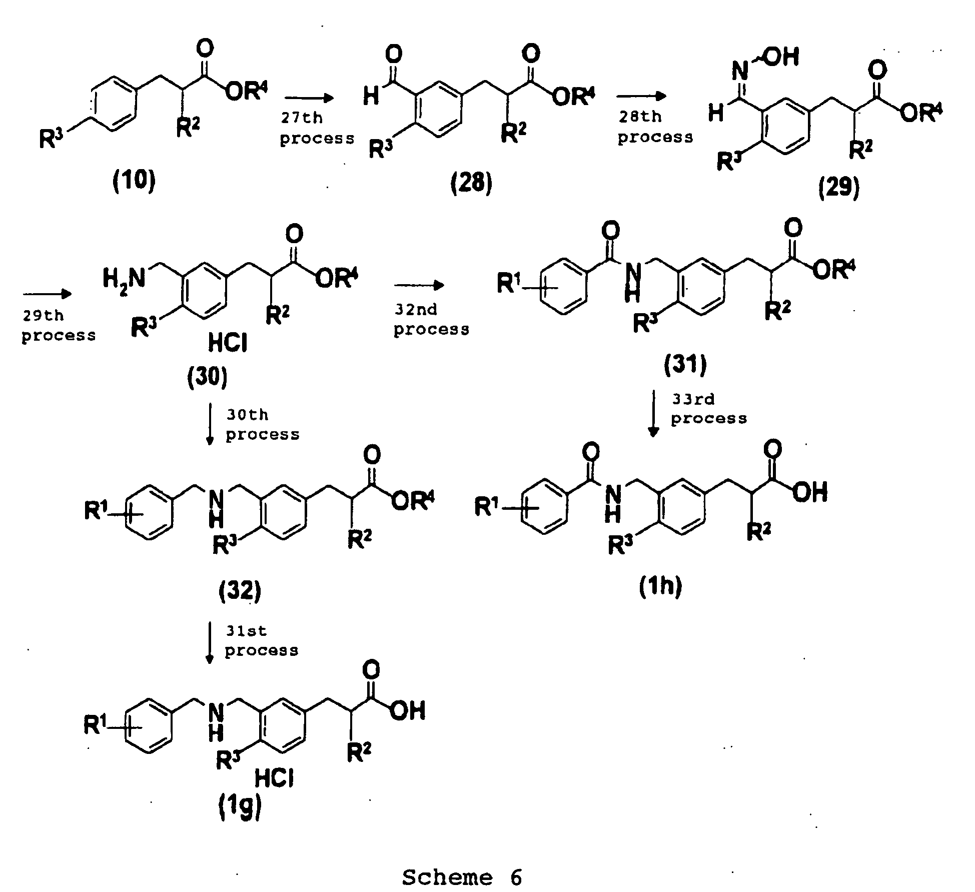

Namely, compounds represented by the general formula (1g) 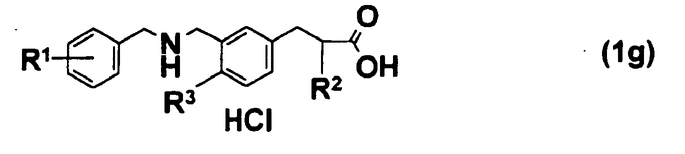 [wherein R¹ denotes a lower alkyl group with carbon atoms of 1 to 4, lower alkoxy group with carbon atoms of 1 to 3, trifluoromethyl group, trifluoromethoxy group, phenyl group which is unsubstituted or may have substituents, phenoxy group which is unsubstituted or may have substituents or benzyloxy group which is unsubstituted or may have substituents, R² denotes a hydrogen atom, lower alkyl group with carbon atoms of 1 to 4 or lower alkoxy group with carbon atoms of 1 to 3, and R³ denotes a lower alkoxy group with carbon atoms of 1 to 3], can be prepared by reacting (twenty-seventh process) compounds represented by the general formula (10) 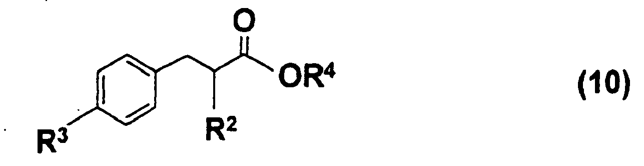 [wherein R² denotes a hydrogen atom, lower alkyl group with carbon atoms of 1 to 4 or lower alkoxy group with carbon atoms of 1 to 3, R³ denotes a lower alkoxy group with carbon atoms of 1 to 3, and R⁴ denotes a lower alkyl group with carbon atoms of 1 to 4], with chloromethyl methyl ether in the presence of Lewis acid, to synthesize compounds represented by a general formula (28) 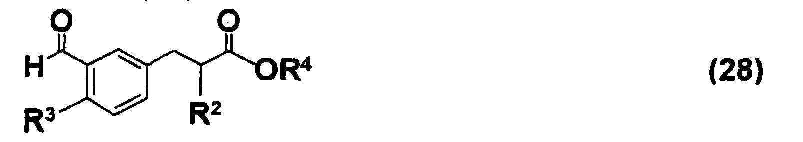 [wherein R² denotes a lower alkyl group with carbon atoms of 1 to 4, trifluoroethyl group or lower alkoxy group with carbon atoms of 1 to 3, R³ denotes a lower alkoxy group with carbon atoms of 1 to 3, and R⁴ denotes a lower alkyl group with carbon atoms of 1 to 4], by reacting (twenty-eighth process) these compounds with hydroxylamine, to synthesize compounds represented by a general formula (29) 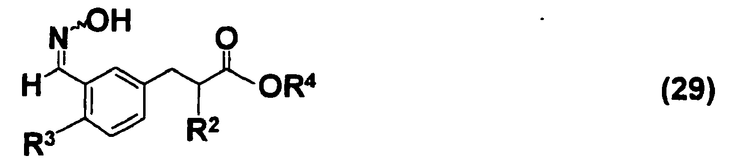 [wherein R², R³ and R⁴ are as described above], by reducing (twenty-ninth process) these compounds to synthesize compounds represented by a general formula (30) 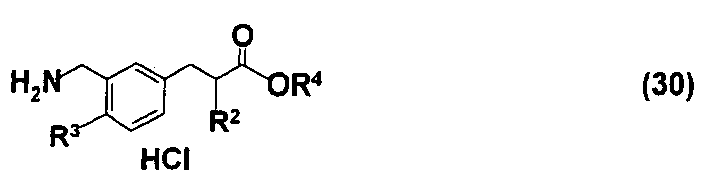 [wherein R², R³ and R⁴ are as described above], by reacting (thirtieth process) these compounds with compounds represented by the general formula (22) 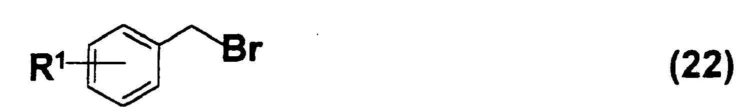 [wherein R¹ is as described above], to synthesize compounds represented by a general formula (32) 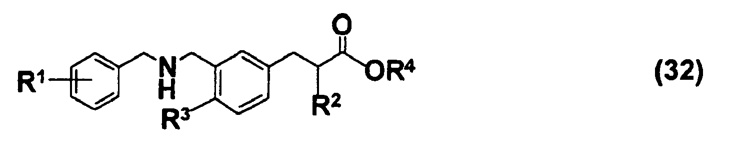 [wherein R¹, R², R³ and R⁴ are as described above], and by hydrolyzing (thirty-first process) COOR⁴ portion of these compounds.

Moreover, compounds represented by the general formula (1h) 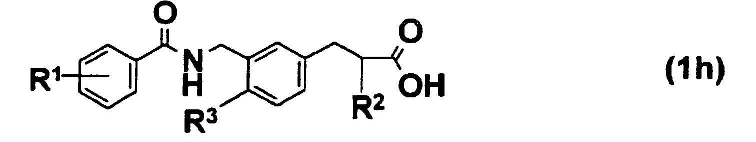 [wherein R¹ denotes a lower alkyl group with carbon atoms of 1 to 4, lower alkoxy group with carbon atoms of 1 to 3, trifluoromethyl group, trifluoromethoxy group, phenyl group which is unsubstituted or may have substituents, phenoxy group which is unsubstituted or may have substituents or benzyloxy group which is unsubstituted or may have substituents, R² denotes a hydrogen atom, lower alkyl group with carbon atoms of 1 to 4 or lower alkoxy group with carbon atoms of 1 to 3, and R3 denotes a lower alkoxy group with carbon atoms of 1 to 3], can be prepared by reacting (thirty-second process) compounds represented by the general formula (30) 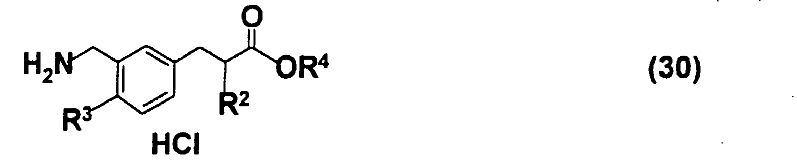 [wherein R², R³ and R⁴ are as described above], with compounds represented by a general formula (33) 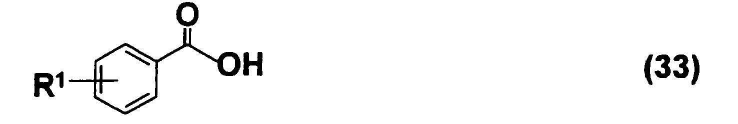 [wherein R¹ denotes a lower alkyl group with carbon atoms of 1 to 4, lower alkoxy group with carbon atoms of 1 to 3, halogen atom, trifluoromethyl group, trifluoromethoxy group, phenyl group which is unsubstituted or may have substituents, phenoxy group which is unsubstituted or may have substituents or benzyloxy group which is unsubstituted or may have substituents], to synthesize compounds represented by a general formula (31) 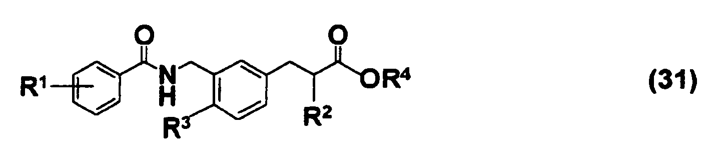 [wherein R¹, R², R³ and R⁴ are as described above], and by hydrolyzing (thirty-third process) COOR⁴ portion of these compounds.

The reaction of the twenty-seventh process can be performed in a solvent such as methylene chloride, chloroform or nitrobenzene. As a Lewis acid, aluminum chloride, tin chloride, boron trifluoride, titanium tetrachloride or the like can be used. The reaction can be performed at a reaction temperature of -50°C to 150°C, preferably -20°C to refluxing temperature of solvent.

The reaction of the twenty-eighth process can be performed in a solvent such as methanol, ethanol, tetrahydrofuran or dioxane in the presence of base of alkali metal hydroxide such as sodium hydroxide, alkali metal carbonate such as potassium carbonate, organic base such as pyridine, or the like. The reaction can be performed at a reaction temperature of -20°C to 150°C, preferably 0°C to 100 °C.

The reaction of the twenty-ninth process can be performed at a hydrogen pressure of 98.1kPa to 491kPa in a solvent such as ethanol, methanol, tetrahydrofuran, ethyl acetate or N,N-dimethylformamide in the presence of metallic catalyst such as palladium on carbon, platinum on carbon, platinum oxide or rhodium on alumina and in the presence or absence of acid such as hydrochloric acid or acetic acid. The reaction can be performed at a reaction temperature of 0°C to 100°C, preferably room tempera-ture to 80°C.

In the reaction of the thirtieth process, as a base, for example, alkali metal hydroxide such as sodium hydroxide, alkali metal carbonate such as potassium carbonate, organic base such as pyridine can be used in a solvent such as ethanol or N,N-dimethylformamide. The reaction can be implemented at a reaction temperature of -20°C to 150°C, preferably 0°C to refluxing temperature of solvent.

The hydrolysis reaction of the thirty-first process can be implemented under alkaline condition. For the alkaline condition, lithium hydroxide, sodium hydroxide, potassium hydroxide or the like is used. The reaction can be implemented at a reaction temperature of 0°C to 80°C, preferably room temperature to 60°C.

The condensation reaction of the thirty-second process can be performed by leaving carboxyl group as it is or converting it to reactive derivatives.

As the "reactive derivative group of carboxyl group", acid chloride, acid bromide, acid anhydride, carbonylimidazole or the like is mentioned. In the case of the reaction using reactive derivative, the reaction can be performed in a solvent such as dioxane or N,N-dimethylformamide in the presence or absence of, for example, alkali metal hydride such as sodium hydride, alkali metal hydroxide such as sodium hydroxide, alkali metal carbonate such as potassium carbonate, or organic base such as pyridine or triethylamine as a base.

In the case of conducting the reaction by leaving carboxylic acid form as it is, the reaction can be performed in a solvent such as methylene chloride, chloroform, dioxane or N,N-dimethylformamide in the presence of condensing agent in the presence or absence of base, and further in the presence or absence of additive.

As the condensing agent, for example, dicyclohexylcarbodiimide,1-[3-(dimethylamino)propyl]-3-ethylcarbodiimide hydrochloride, diethyl cyanophosphate, diphenylphosphoric azide, carbonyldiimidazole or the like can be mentioned. As the base, for example, alkali metal hydroxide such as sodium hydroxide, alkali metal carbonate such as potassium carbonate, or organic base such as pyridine or triethylamine can be mentioned. As the additive, N-hydroxybenzotriazole, N-hydroxysuccinimide, 3,4-dihydro-3-hydroxy-4-oxo-1,2,3-benzotriazine or the like can be mentioned. The reaction can be performed at a reaction temperature of -20°C to 100°C, preferably 0°C to 50°C.

The hydrolysis reaction of the thirty-third process can be performed under alkaline condition. For the alkaline condition, lithium hydroxide, sodium hydroxide, potassium hydroxide or the like is used. The reaction can be performed at a reaction temperature of 0°C to 80°C, preferably room temperature to 60°C.

Moreover, optically active substances (1b') of the compounds of general formula (1b), the binding mode of A portion being -CH₂CONH-, in the compounds of said general formula (1) can be prepared, for example, through following processes (Scheme 7).

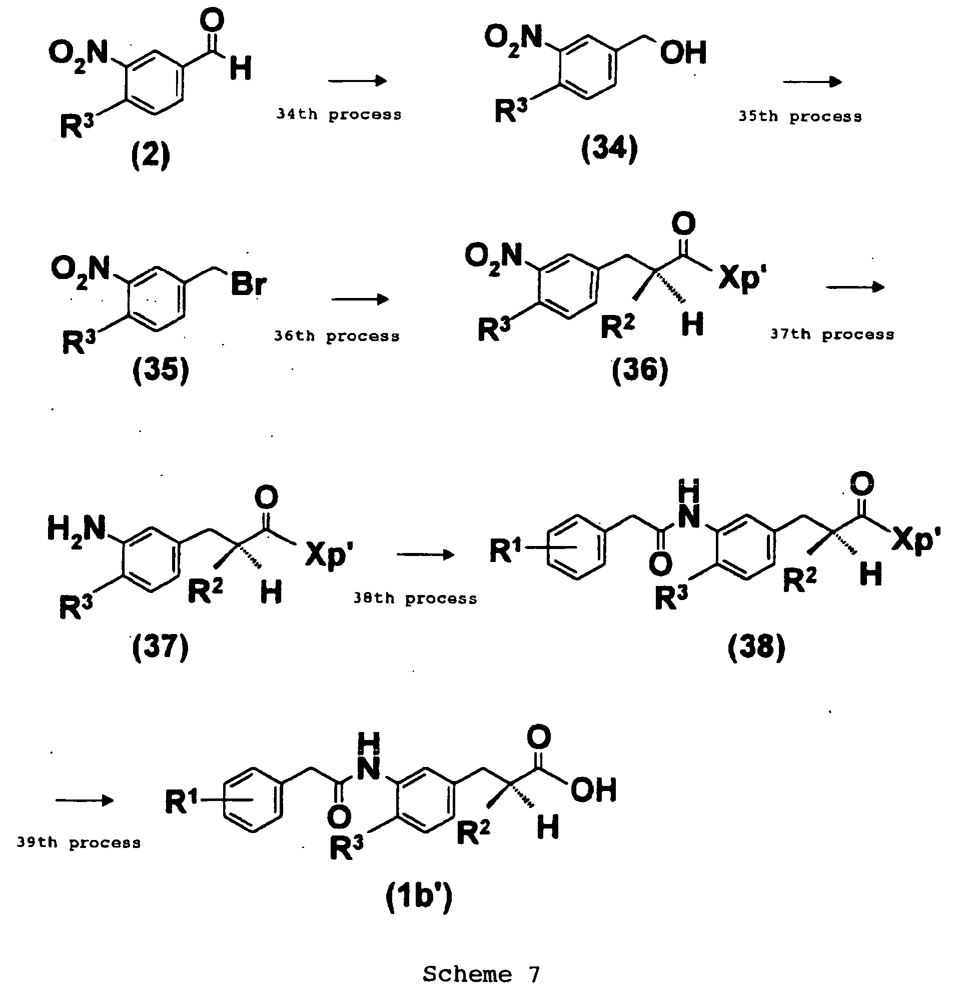

Namely, optically active compounds represented by the general formula (1b') 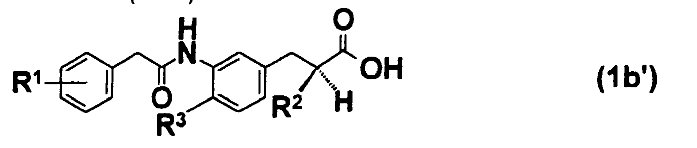 [wherein R¹ denotes a lower alkyl group with carbon atoms of 1 to 4, lower alkoxy group with carbon atoms of 1 to 3, trifluoromethyl group, trifluoromethoxy group, phenyl group which is unsubstituted or may have substituents, phenoxy group which is unsubstituted or may have substituents or benzyloxy group which is unsubstituted or may have substituents, R² denotes a lower alkyl group with carbon atoms of 1 to 4 or lower alkoxy group with carbon atoms of 1 to 3, and R³ denotes a lower alkoxy group with carbon atoms of 1 to 3], can be prepared by reducing (thirty-fourth process) compounds represented by the general formula (2) 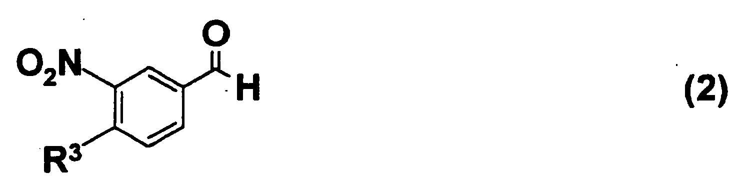 [wherein R³ is as described above], to synthesize compounds represented by a general formula (34) 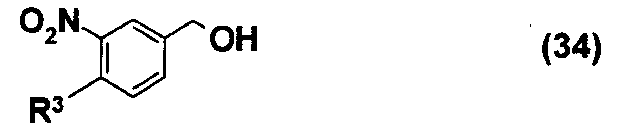 [wherein R³ is as described above], by brominating (thirty-fifth process) these compounds to synthesize compounds represented by a general formula (35) 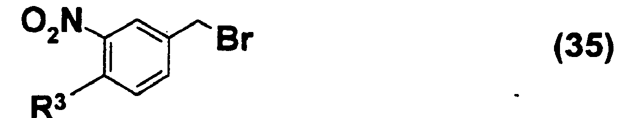 [wherein R³ is as described above], by reacting (thirty-sixth process) these compounds with compounds represented by a general formula (39') 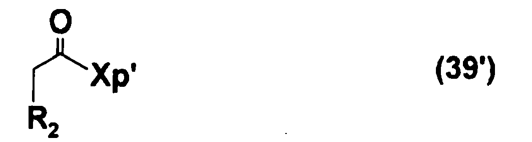 [wherein R² is as described above, and Xp' denotes chiral oxazolidinone, the absolute configuration being (R), such as (R)-4-benzyl-2-oxazolidinone-3-yl group, (R)-4-isopropyl-2-oxazolidinone-3-yl group or (R)-4-phenyl-2-oxazolidinone-3-yl group, chiral imidazolidinone, chiral cyclolactam, chiral sultam or the like], in the presence of base, to synthesize compounds represented by a general formula (36) 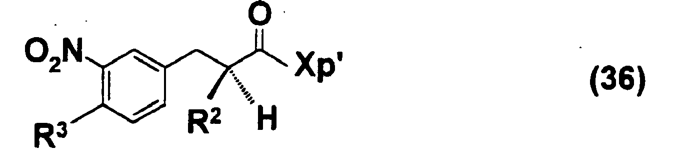 [wherein R², R³ and Xp' are as described above], by reducing (thirty-seventh process) these compounds to synthesize compounds represented by a general formula (37) 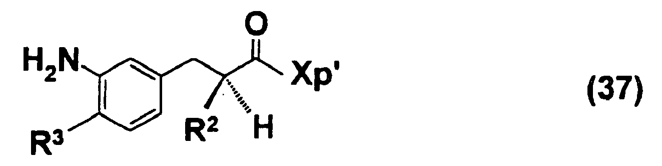 [wherein R², R³ and Xp' are as described above], by reacting (thirty-eighth process) these compounds with compounds represented by the general formula (7) 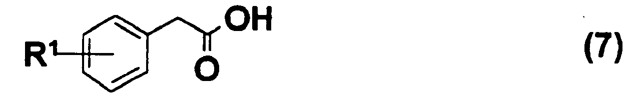 [wherein R¹ denotes a lower alkyl group with carbon atoms of 1 to 4, lower alkoxy group with carbon atoms of 1 to 3, trifluoromethyl group, trifluoromethoxy group, phenyl group which is unsubstituted or may have substituents, phenoxy group which is unsubstituted or may have substituents or benzyloxy group which is unsubstituted or may have substituents], to synthesize compounds represented by a general formula (38) 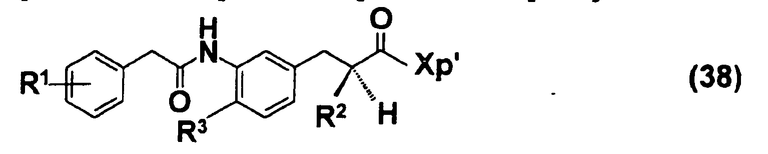 [wherein R¹, R², R³ and Xp' are as described above], and by hydrolyzing (thirty-ninth process) COXp' portion of these compounds.

In the reaction of the thirty-fourth process, as a reducing agent, for example, diisobutylaluminum hydride, borane, alkylborane, sodium borohydride or the like can be used in a solvent such as tetrahydrofuran, diethyl ether or hexane. The reaction can be performed at a reaction temperature of -100°C to 100°C, preferably -80°C to room temperature.

The reaction of the thirty-fifth process can be implemented in a solvent such as methylene chloride, benzene or toluene, using brominating agent such as phosphorus tribromide, thionyl bromide, hydrobromic acid or triphenylphosphine and carbon tetrabromide. The reaction can be performed at a reaction temperature of -20°C to 150°C, preferably 0°C to 100°C.

In the reaction of the thirty-sixth process, as a base, for example, alkali metal hydride such as sodium hydride, organometallic compound such as butyl lithium or metal amide such as lithium diisopropylamide or sodium bis(trimethylsilyl)amide can be used in a solvent such as tetrahydrofuran, diethyl ether or hexane. The reaction can be performed at a reaction temperature of -100°C to room temperature, preferably -80°C to 0°C.

The reaction of the thirty-seventh process can be performed at a hydrogen pressure of 98.1kPa to 491kPa in a solvent such as ethanol, methanol, tetrahydrofuran, ethyl acetate or N,N-dimethylformamide in the presence of metallic catalyst such as palladium on carbon, platinum on carbon, platinum oxide or rhodium on alumina. The reaction can be performed at a reaction temperature of 0°C to 100°C, preferably room temperature to 80°C.

The reaction of the thirty-eighth process can be performed by leaving carboxyl group as it is or converting it to reactive derivatives. As the "reactive derivative group of carboxyl group", acid chloride, acid bromide, acid anhydride, carbonylimidazole or the like is mentioned.

In the case of the reaction using reactive derivative, the reaction can be performed in a solvent such as dioxane or N,N-dimethylformamide in the presence or absence of, for example, alkali metal hydride such as sodium hydride, alkali metal hydroxide such as sodium hydroxide, alkali metal carbonate such as potassium carbonate, or organic base such as pyridine or triethylamine as a base.

In the case of conducting the reaction by leaving carboxylic acid form as it is, the reaction can be performed in a solvent such as methylene chloride, chloroform, dioxane or N,N-dimethylformamide in the presence of condensing agent in the presence or absence of base, and further in the presence or absence of additive.

As the condensing agent, for example, dicyclohexylcarbodiimide, 1-[3-(dimethylamino)propyl]-3-ethylcarbodiimide hydrochloride, diethyl cyanophosphate, diphenylphosphoric azide, carbonyldiimidazole or the like can be mentioned. As the base, for example, alkali metal hydroxide such as sodium hydroxide, alkali metal carbonate such as potassium carbonate, or organic base such as pyridine or triethylamine can be mentioned. As the additive, N-hydroxybenzotriazole, N-hydroxysuccinimide, 3,4-dihydro-3-hydroxy-4-oxo-1,2,3-benzotriazine or the like can be mentioned. The reaction can be performed at a reaction temperature of -20°C to 100°C, preferably 0°C to 50°C.

The reaction of the thirty-ninth process can be performed under alkaline condition. For the alkaline condition, lithium hydroxide, sodium hydroxide, mixture of lithium hydroxide with hydrogen peroxide or the like is used. The reaction can be performed at a reaction temperature of -20°C to 100°C, preferably 0°C to 50°C.

Moreover, optically active substances (1c') of the compounds of general formula (1c), the binding mode of A portion being -NHCOCH₂-, in the compounds of said general formula (1) can be synthesized, for example, through following processes (Scheme 8).

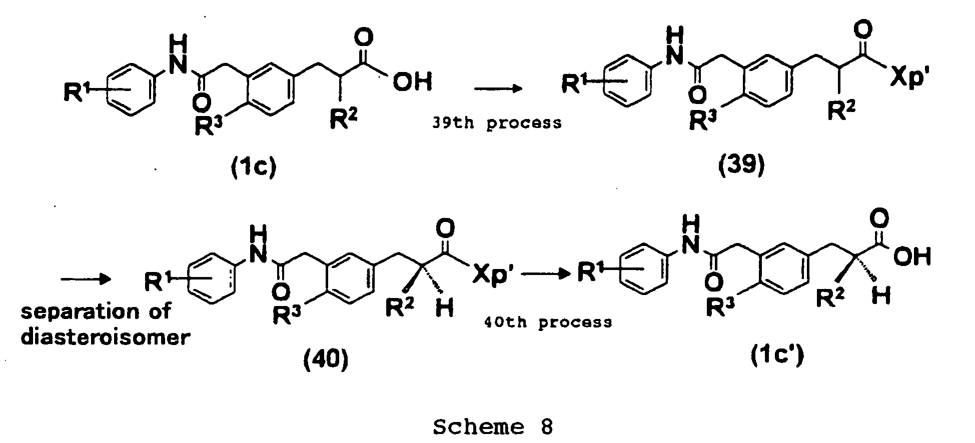

Namely, optically active compounds represented by the general formula (1c') 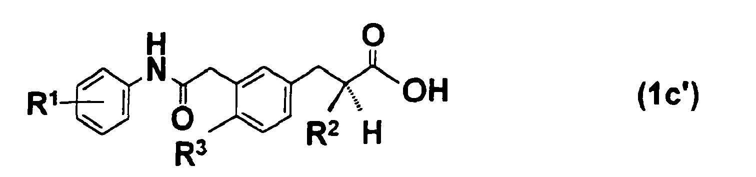 [wherein R¹ denotes a lower alkyl group with carbon atoms of 1 to 4, lower alkoxy group with carbon atoms of 1 to 3, trifluoromethyl group, trifluoromethoxy group, phenyl group which is unsubstituted or may have substituents, phenoxy group which is unsubstituted or may have substituents or benzyloxy group which is unsubstituted or may have substituents, R² denotes a lower alkyl group with carbon atoms of 1 to 4 or lower alkoxy group with carbon atoms of 1 to 3, and R³ denotes a lower alkoxy group with carbon atoms of 1 to 3], can be prepared by reacting compounds represented by the general formula (1c) 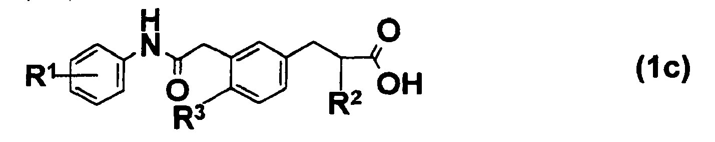 [wherein R¹ denotes a lower alkyl group with carbon atoms of 1 to 4, lower alkoxy group with carbon atoms of 1 to 3, trifluoromethyl group, trifluoromethoxy group, phenyl group which is unsubstituted or may have substituents, phenoxy group which is unsubstituted or may have substituents or benzyloxy group which is unsubstituted or may have substituents, R² denotes a hydrogen atom, lower alkyl group with carbon atoms of 1 to 4 or lower alkoxy group with carbon atoms of 1 to 3, and R³ denotes a lower alkoxy group with carbon atoms of 1 to 3], with pivaloyl chloride in the presence of base, to synthesize compounds represented by a general formula (41) 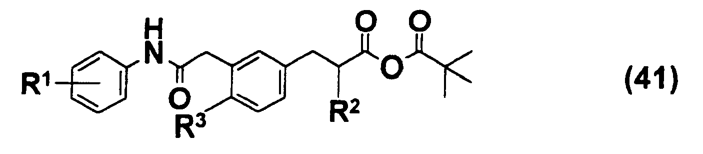 [wherein R¹, R² and R³ are as described above], by reacting (thirty-ninth process) these compounds with compounds represented by a general formula (42)

**Xp"-H** (42)

[wherein Xp" denotes an optically active chiral oxazolidinone derivative such as optically active 4-benzyl-2-oxazolidinone-3-yl group, 4-isopropyl-2-oxazolidinone-3-yl group or 4-phenyl-2-oxazolidinone-3-yl group, amide derivative, sultam derivative or the like], in the presence of base, to synthesize compounds represented by a general formula (39) 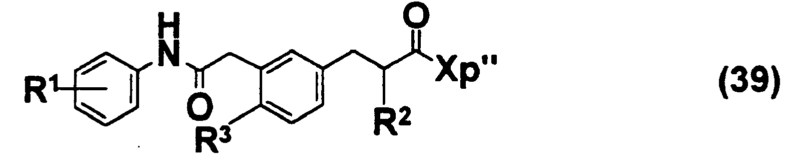 [wherein R¹, R², R³ and Xp" are as described above], by recrystallizing or fractionating by means of column chromatography each diastereomer of these compounds, to obtain compounds represented by a general formula (40) 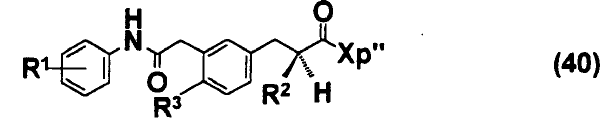 [wherein R¹, R², R³ and Xp'' are as described above], and by hydrolyzing (fortieth process) Xp'' portion of these compounds.

In the reaction of the thirty-ninth process, first, the synthesis of the compounds represented by the general formula (41) 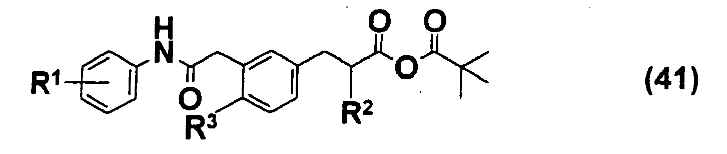 [wherein R¹, R² and R³ are as described above], can be implemented in a solvent such as tetrahydrofuran, methylene chloride or diethyl ether, using tertiary amine such as triethylamine, diisopropylethylamine, ethyldimethylamine or pyridine. The reaction can be performed at a reaction temperature of -100°C to room temperature, preferably -40°C to 0°C.

Next, the reaction of compounds represented by the general formula (41) 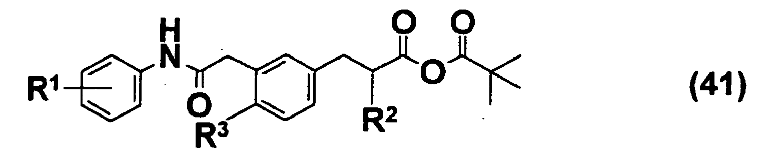 [wherein R¹, R² and R³ are as described above], with general formula (42)

**Xp"-H** (42)

[wherein Xp''is as described above], can be implemented in a solvent such as tetrahydrofuran, methylene chloride or diethyl ether, by reacting base of alkali metal hydride such as sodium hydride, organometallic compound such as butyl lithium, metal amide such as lithium diisopropylamide, metal alkoxide such as sodium methoxide or potassium t-butoxide. The reaction can be performed at a reaction temperature of -100°C to room temperature, preferably -40°C to 0°C.

The reaction of the fortieth process can be performed under alkaline condition. For the alkaline condition, lithium hydroxide, sodium hydroxide, mixture of lithium hydroxide with hydrogen peroxide or the like is used. The reaction can be performed at a reaction temperature of -20°C to 100°C, preferably 0°C to 50°C.

As the forms for administering the inventive novel compounds, solid compositions, liquid compositions and other compositions for oral administration, and injections, external medicines, suppositories, etc. for parenteral administration can be mentioned. The solid compositions for oral administration include tablets, pills, capsules, powders, granules, etc, The liquid compositions for oral administration include aseptic aqueous or nonaqueous solutions, suspe,sions, emulsions, etc.

### Best embodiment to put the invention into practice

In following, the invention will be illustrated based on concrete examples, but the invention is not confined to these examples.

### <Example 1> : Ethyl 3-(3-amino-4-methoxyphenyl)-2-methoxypropanoate

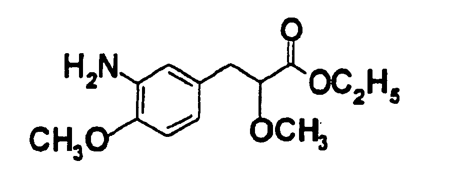

Under an argon atmosphere, stirring and cooling with ice, a solution of triethyl 2-phosphonobutyrate (5.00g, 19.8mmol) in anhydrous tetrahydrofuran (30mL) was added dropwise slowly to a solution of sodium hydride (60% oil dispersion, 795mg, 19.9mmol) in anhydrous tetrahydrofuran (120mL). After stirring for 1 hour at 0°C, a solution of 4-methoxy-3-nitrobenzaldehyde (3.26g, 18.0mmol) in anhydrous tetrahydrofuran (50mL) was added dropwise, and the mixture was stirred for 1 hour at 0°C and for 3 hours at room temperature. The reaction mixture was concentrated and ice water was added to the residue, which was extracted with ethyl acetate. The extract was washed with water, brine and dried over anhydrous sodium sulfate, and then concentrated. The residue was purified by silica gel column chromatography (eluent n-hexane:ethyl acetate=8:1→2:1v/v) to obtain 4.69g (93%) of ethyl 2-ethyl-3-(4-methoxy-3-nitrophenyl)acrylate as colorless crystals.
Mass analysis m/z 281 (M⁺).

Next, ethyl 2-ethyl-3-(4-methoxy-3-nitrophenyl)acrylate (4.50g, 16.0mmol), 10% palladium on carbon (1.50g) and a 1:1 mixed solvent (150mL) of tetrahydrofuran with ethanol were mixed and hydrogenation was carried out at an initial pressure of 294.3kPa at room temperature. After completion of the reaction, the catalyst was removed by filtration and the filtrate was concentrated to afford 3.84g (95%) of the title compound as colorless crystals.
Mass analysis m/z 253 (M⁺).

### <Examples 2 and 3>

Similarly to Example 1, compounds shown in Table 1 were synthesized.

**Table 1**

| | | | | |
|---|---|---|---|---|
| 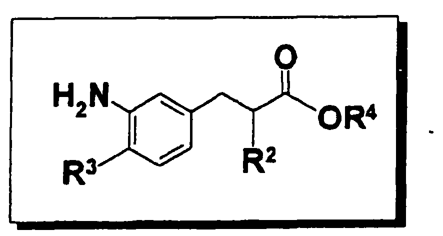 | | | | |

| Example | R² | R³ | R⁴ | Mass analysis (m/z) |
|---|---|---|---|---|
| 2 | C₂H₅ | OCH₃ | C₂H₅ | 251(M⁺) |
| 3 | n-C₃H₇ | OCH₃ | C₂H₅ | 265(M⁺) |

### <Example 4>: Methyl 3-(4-methoxy-3-nitrophenyl)propanoate

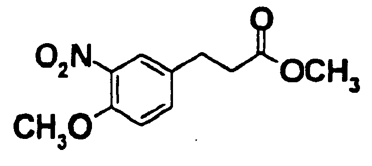

To 100mL of concentrated nitric acid, cooled with ice, publicly known [e.g. Lebedev S. A. et al, J. Organo-met. Chem., 1988, 253] 4-methoxyphenylpropanoic acid (5.00g, 25.7mmol) was added little by little under stirring. After completion of the addition, the mixture was stirred further for 4 hours under cooling with ice. The reaction mixture was poured into ice water, which was extracted with methylene chloride. The extract was dried over anhydrous sodium sulfate and concentrated. The residue was purified by silica gel column chromatography (eluent methylene chloride→methylene chloride:methanol=15:1v/v) to obtain 2.70g (44%) of the title compound as a faintly yellow oil.
¹H-NMR (400MHz, CDCl₃) δ2.64 (2H, t, J=7.3Hz), 2.95 (2H, t, J=7.3 Hz), 3.67 (3H, s), 3.94 (3H, s), 7.02 (1H, d, J=8.3Hz), 7.39 (1H, dd, J=8.3, 2.4Hz), 7.70 (1H, d, J=2.4Hz).

### <Example 5>: Methyl 3-(3-amino-4-methoxyphenyl)propanoate

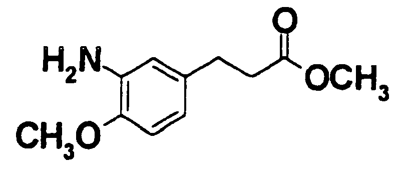

Methyl 4-methoxy-3-nitrophenylpropanoate (2.70g, 11.3mmol), 10% palladium on carbon (200mg) and a mixed solvent (100 mL) of ethyl acetate with ethanol (1:1v/v) were mixed and hydrogenation was carried at an initial pressure of 294.3kPa. After completion of the hydrogenation, the catalyst was removed by filtration and the filtrate was concentrated to obtain 2.35g (95%) of the title compound as a yellow oily product.
1H-NMR (400MHz, CDCl₃) δ2.58 (2H, t, J=8.3Hz), 2.82 (2H, t, J=8.3 Hz), 3.67 (3H, s), 3.82 (3H, s), 6.53-6.57 (2H, m), 6.70 (1H, d, J=8.3Hz).

### <Example 6>: Ethyl 2-methoxy-3-[4-methoxy-3-[2-[4-(trifluoromethyl)phenyl]acetylamino]phenyl]propanoate

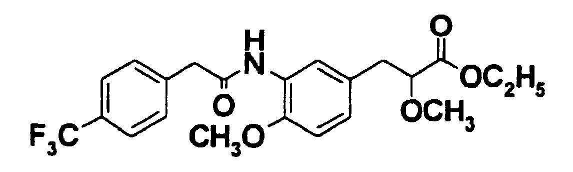

4-Trifluoromethylphenylacetic acid (316mg, 1.50mmol) and methyl 2-methoxy-3-(3-amino-4-methoxyphenyl)propanoate (253mg, 0.999mmol) were dissolved in dichloromethane (10mL) and, after N-cyclohexyl-carbodiimido-N'-methyl polystyrene (1.46g, 2.26mmol) was added, the mixture was stirred for 30.5 hours at room temperature. The insolubles were removed by filtration and the filtrate was concentrated under reduced pressure to afford the title product quantitatively as brown crystals. Mass analysis m/z 439 (M⁺).
¹H-NMR (400MHz, CDCl₃) δ1.25 (3H, t, J=7.3Hz), 2.91 (1H, dd, J=14.2, 7.8Hz), 2.98 (1H, dd, J=14.2, 5.4Hz), 3.33 (3H, s), 3.75 (3H, s), 3.80 (2H, s), 3.91-3.95 (1H, m), 4.19 (2H, q, J=7.3Hz), 6.74 (1H, d, J=8.8 Hz), 6.90 (1H, dd, J=8.3, 2.0Hz), 7.48 (2H, d, J=7.8 Hz), 7.65 (2H, d, J=8.3Hz), 7.74 (1H, s), 8.25 (1H, d, J=2.0Hz).

### <Examples 7 through 38>

Similarly to Example 6, compounds in Table 2 were obtained.

**Table 2**

| | | | | | |
|---|---|---|---|---|---|
| 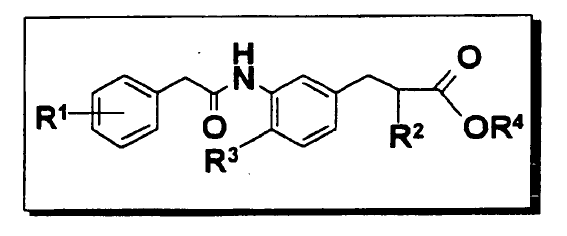 | | | | | |

| Example | R¹ | R² | R³ | R⁴ | Mass analysis (m/z) |
|---|---|---|---|---|---|
| 7 | 4-CF₃ | H | OCH₃ | CH₃ | 395 (M⁺) |
| 8 | 4-OCH₂Ph | H | OCH₃ | CH₃ | 433 (M⁺) |
| 9 | 4-OPh | H | OCH₃ | CH₃ | 419 (M⁺) |
| 10 | 4-OCH₃ | H | OCH₃ | CH₃ | 357 (M⁺) |
| 11 | 4-Ph | H | OCH₃ | CH₃ | 403 (M⁺) |
| 12 | 4-CF₃ | C₂H₅ | OCH₃ | C₂H₅ | 437 (M⁺) |
| 13 | 4-OCH₂Ph | C₂H₅ | OCH₃ | C₂H₅ | 475 (M⁺) |
| 14 | 4-OPh | C₂H₅ | OCH₃ | C₂H₅ | 461 (M⁺) |
| 15 | 4-OCH₃ | C₂H₅ | OCH₃ | C₂H₅ | 399 (M⁺) |
| 16 | 4-Ph | C₂H₅ | OCH₃ | C₂H₅ | 445 (M⁺) |
| 17 | 4-CH₃ | C₂H₅ | OCH₃ | C₂H₅ | 383 (M⁺) |
| 18 | 4-Cl | C₂H₅ | OCH₃ | C₂H₅ | 403 (M⁺) |
| 19 | 4-F | C₂H₅ | OCH₃ | C₂H₅ | 387 (M⁺) |
| 20 | 3-F | C₂H₅ | OCH₃ | C₂H₅ | 387 (M⁺) |
| 21 | 2-F | C₂H₅ | OCH₃ | C₂H₅ | 387 (M⁺) |
| 22 | 4-OPh(4-F) | C₂H₅ | OCH₃ | C₂H₅ | 479 (M⁺) |
| 23 | 4-OPh(2-OCH₃) | C₂H₅ | OCH₃ | C₂H₅ | 491 (M⁺) |
| 24 | 4-CF₃ | n-C₃H₇ | OCH₃ | C₂H₅ | 451 (M⁺) |
| 25 | 4-OCH₂Ph | n-C₃H₇ | OCH₃ | C₂H₅ | 489 (M⁺) |
| 26 | 4-OPh | n-C₃H₇ | OCH₃ | C₂H₅ | 475 (M⁺) |
| 27 | 4-OCH₃ | n-C₃H₇ | OCH₃ | C₂H₅ | 413 (M⁺) |
| 28 | 4-Ph | n-C₃H₇ | OCH₃ | C₂H₅ | 459 (M⁺) |
| 29 | 4-CH₃ | n-C₃H₇ | OCH₃ | C₂H₅ | 397 (M⁺) |
| 30 | 4-Cl | n-C₃H₇ | OCH₃ | C₂H₅ | 417 (M⁺) |
| 31 | 4-F | n-C₃H₇ | OCH₃ | C₂H₅ | 401 (M⁺) |
| 32 | 3-F | n-C₃H₇ | OCH₃ | C₂H₅ | 401 (M⁺) |
| 33 | 2-F | n-C₃H₇ | OCH₃ | C₂H₅ | 401 (M⁺) |
| 34 | 4-OPh(4-F) | n-C₃H₇ | OCH₃ | C₂H₅ | 493 (M⁺) |
| 35 | 4-OPh(2-OCH₃) | n-C₃H₇ | OCH₃ | C₂H₅ | 505 (M⁺) |
| 36 | 4-OCH₂Ph | OCH₃ | OCH₃ | C₂H₅ | 477 (M⁺) |
| 37 | 4-OPh | OCH₃ | OCH₃ | C₂H₅ | 463 (M⁺) |
| 38 | 4-Ph | OCH₃ | OCH₃ | C₂H₅ | 447 (M⁺) |

### <Example 39>: 2-Methoxy-3-[4-methoxy-3-[2-[4-(trifluoromethyl)phenyl]acetylamino]phenyl]propanoic acid

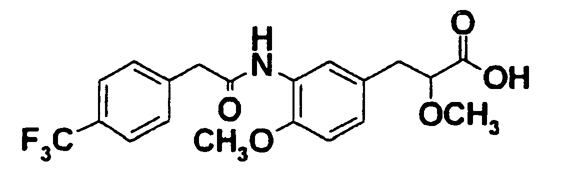

Ethyl 2-methoxy-3-[4-methoxy-3-[2-[4-(trifluoromethyl)-phenyl]acetylamino]phenyl]propanoate (442mg, 0.986mmol), methanol (9mL) and 1mol/L aqueous solution of sodium hydroxide (5mL) were mixed. After stirring for 5 hours at room temperature, the reaction mixture was concentrated under reduced pressure. The residue was dissolved in water, washed with ether, and then made acidic with diluted hydrochloric acid. The precipitates produced were filtered and dried to afford 379mg (93%) of the title compound as pale yellow crystals.
Melting point 124-125°C.
Mass analysis m/z 411(M⁺).
Elemental analysis C₂₀H₂₀F₃NO₅:
Calcd. (%) C, 58.39; H, 4.90; N, 3.40.
Found (%) C, 58.39; H, 4.83; N, 3.52.

### <Examples 40 through 71>

Similarly to Example 39, compounds in Table 3 were obtained.

**Table 3**

| | | | | | | |
|---|---|---|---|---|---|---|
| 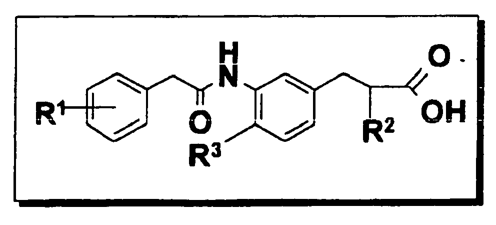 | | | | | | |

| Example | R¹ | R² | R³ | Melting point(°C) | Charac. formula | Elemental analysis(%) |
|---|---|---|---|---|---|---|
| 40 | 4-CF₃ | H | OCH₃ | 186-187 | C₁₉H₁₉F₃NO₄ | Calc.; C 59.84, H 4.76, N 3.67 |
| | | | | | | Found; C 59.73, H 4.77, N 3.67 |
| 41 | 4-OCH₃ | H | OCH₃ | 160-161 | C₁₉H₂₁NO₅ | Calc.; C 66.46, H 6.16, N 4.08 |
| | | | | | | Found; C 66.39, H 6.07, N 4.05 |
| 42 | 4-OCH₂Ph | H | OCH₃ | 151-152 | C₂₅H₂₅NO₅ | Calc.; C 71.58, H 6.01, N 3.34 |
| | | | | | | Found; C 71.29, H 5.99, N 3.44 |
| 43 | 4-OPh | H | OCH₃ | 146-147 | C₂₄H₂₃NO₅ | Calc.; C 71.10, H 5.72, N 3.45 |
| | | | | | | Found; C 71.13, H 5.71, N 3.54 |
| 44 | 4-Ph | H | OCH₃ | 177 | C₂₄H₂₃NO₄ | Calc.; C 74.02, H 5.95, N 3.60 |
| | | | | | | Found; C 73.80, H 5.97, N 3.60 |
| 45 | 4-CF₃ | C₂H₅ | OCH₃ | (amorphous) | C₂₁H₂₂F₃NO₄ | Calc.; C 61.61, H 5.42, N 3.42 |
| | | | | | | Found; C 61.43, H 5.44, N 3.45 |
| 46 | 4-OCH₂Ph | C₂H₅ | OCH₃ | (amorphous) | C₂₇H₂₉NO₅ | Calc.; C 72.46, H 6.53, N 3.13 |
| | | | | | | Found; C 72.41, H 6.56, N 3.06 |
| 47 | 4-OPh | C₂H₅ | OCH₃ | (amorphous) | C₂₅H₂₇NO₅· 1/10H₂O | Calc.; C 71.74, H 6.30, N 3.22 |
| | | | | | | Found; C 71.64, H 6.39, N 3.24 |
| 48 | 4-OCH₃ | C₂H₅ | OCH₃ | 135-136 | C₂₁H₂₅NO₅ | Calc.; C 67.91, H 6.78, N 3.77 |
| | | | | | | Found; C 67.85, H 6.81, N 3.81 |
| 49 | 4-Ph | C₂H₅ | OCH₃ | 142-143 | C₂₆H₂₇NO₄ | Calc.; C 74.80, H 6.52, N 3.35 |
| | | | | | | Found; C 74.75, H 6.64, N 3.38 |
| 50 | 4-CH₃ | C₂H₅ | OCH₃ | 134-135 | C₂₁H₂₅NO₄ | Calc.; C 70.96, H 7.09, N 3.94 |
| | | | | | | Found; C 70.86, H 7.10, N 4.02 |
| 51 | 4 4-Cl | C₂H₅ | OCH₃ | 112-113 | C₂₀H₂₂ClNO₄ | Calc.; C 63.91, H 5.90, N 3.73 |
| | | | | | | Found; C 63.76, H 5.88, N 3.77 |
| 52 | 4-F | C₂H₅ | OCH₃ | 97-98 | C₂₀H₂₂FNO₄ | Calc.; C 66.84, H 6.17, N 3.90 |
| | | | | | | Found; C 66.68, H 6.13, N 3.93 |
| 53 | 3-F | C₂H₅ | OCH₃ | (amorphous) | C₂₀H₂₂FNO₄ | Calc.; C 66.84, H 6.17, N 3.90 |
| | | | | | | Found; C 66.68, H 6.20, N 3.83 |
| 54 | 2-F | C₂H₅ | OCH₃ | 89 | C₂₀H₂₂FNO₄ | Calc.; C 66.84, H 6.17, N 3.90 |
| | | | | | | Found; C 66.85, H 6.17, N 4.01 |
| 55 | 4-OPh(4-F) | C₂H₅ | OCH₃ | 101-102 | C₂₆H₂₆FNO₅· 3/10H₂O | Calc.; C 68.35, H 5.87, N 3.07 |
| | | | | | | Found; C 68.26, H 5.81, N 3.17 |
| 56 | 4-OPh- (2-OCH₃) | C₂H₅ | OCH₃ | (amorphous) | C₂₇H₂₉NO₆ | Calc.; C 69.96, H 6.31, N 3.02 |
| | | | | | | Found; C 69.68, H 6.35, N 3.11 |
| 57 | 4-CF₃ | n-C₃H₇ | OCH₃ | (amorphous) | C₂₂H₂₄F₃NO₄· 2/5H₂O | Calc.; C 61.36, H 5.80, N 3.25 |
| | | | | | | Found; C 61.27, H 5.50, N 3.01 |
| 58 | 4-OCH₂Ph | n-C₃H₇ | OCH₃ | 108-110 | C₂₈H₃₁NO₅ | Calc.; C 72.86, H 6.77, N 3.03 |
| | | | | | | Found; C 72.79, H 6.82, N 3.08 |
| 59 | 4-OPh | n-C₃H₇ | OCH₃ | 116 | C₂₇H₂₉NO₅ | Calc.; C 72.46, H 6.53, N 3.13 |
| | | | | | | Found; C 72.43, H 6.54, N 3.24 |
| 60 | 4-OCH₃ | n-C₃H₇ | OCH₃ | 132-133 | C₂₂H₂₇NO₅ | Calc.; C 68.55, H 7.06, N 3.63 |
| | | | | | | Found; C 68.32, H 7.04, N 3.70 |
| 61 | 4-Ph | n-C₃H₇ | OCH₃ | 145-146 | C₂₇H₂₉NO₄ | Calc.; C 75.15, H 6.77, N 3.25 |
| | | | | | | Found; C 75.02, H 6.87, N 3.26 |
| 62 | 4-CH₃ | n-C₃H₇ | OCH₃ | 115-116 | C₂₂H₂₇NO₄ | Calc.; C 71.52, H 7.37, N 3.79 |
| | | | | | | Found; C 71.31, H 7.35, N 3.80 |
| 63 | 4-Cl | n-C₃H₇ | OCH₃ | 110-111 | C₂₁H₂₄ClNO₄ | Calc.; C 64.69, H 6.20, N 3.59 |
| | | | | | | Found; C 64.62, H 6.13, N 3.61 |
| 64 | 4-F | n-C₃H₇ | OCH₃ | 105-106 | C₂₁H₂₄FNO₄ | Calc.; C 67.54, H 6.48, N 3.75 |
| | | | | | | Found; C 67.46, H 6.43, N 3.84 |
| 65 | 3-F | n-C₃H₇ | OCH₃ | (amorphous) | C₂₁H₂₄FNO₄· 1/5H₂O | Calc.; C 66.90, H 6.52, N 3.72 |
| | | | | | | Found; C 66.98, H 6.50, N 3.80 |
| 66 | 2-F | n-C₃H₇ | OCH₃ | 112-113 | C₂₁H₂₄FNO₄ | Calc.; C 67.54, H 6.48, N 3.75 |
| | | | | | | Found; C 67.46, H 6.40, N 3.78 |
| 67 | 4-OPh-(4-F) | n-C₃H₇ | OCH₃ | 123-124 | C₂₇H₂₈FNO₅· 2/5H₂O | Calc.; C 68.60, H 6.14, N 2.96 |
| | | | | | | Found; C 68.30, H 6.04, N 3.08 |
| 68 | 4-OPh-(2-OCH₃) | n-C₃H₇ | OCH₃ | (amorphous) | C₂₈H₃₁NO₆· 1/10H₂O | Calc.; C 70.16, H 6.56, N 2.92 |
| | | | | | | Found; C 70.16, H 6.57, N 2.93 |
| 69 | 4-OCH₂Ph | OCH₃ | OCH₃ | (amorphous) | C₂₆H₂₇NO₆· 1/5H₂O | Calc.; C 68.92, H 6.10, N 3.09 |
| | | | | | | Found; C 69.03, H 6.30, N 3.12 |
| 70 | 4-OPh | OCH₃ | OCH₃ | (amorphous) | C₂₅H₂₅NO₆ | Calc.; C 68.95, H 5.79, N 3.22 |
| | | | | | | Found; C 68.86, H 5.96, N 3.19 |
| 71 | 4-Ph | OCH₃ | OCH₃ | 112-114 | C₂₅H₂₅NO₅ | Calc.; C 71.58, H 6.01, N 3.34 |
| | | | | | | Found; C 71.48, H 6.09, N 3.35 |

### <Example 72>: Ethyl 2-methylthio-2-(5-formyl-2-methoxyphenyl)acetate

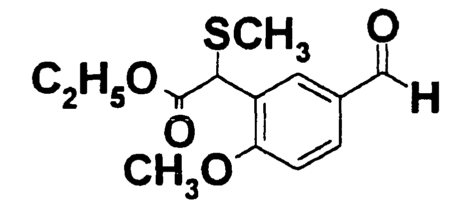

To a solution of 4-methoxybenzaldehyde (7.46g, 57.3mmol) in methylene chloride (250mL), a solution of anhydrous tin chloride (IV) (6.49mL, 54.7mmol) in methylene chloride (100mL) was added dropwise slowly under an argon atmosphere, stirring and cooling with ice. After stirring for 15 minutes at room temperature, a solution of ethyl 2-chloro-2-methylthioacetate (9.24g, 54.8mmol) in a mixed solution (50mL) of methylene chloride with carbon tetrachloride (1:1) was added dropwise. After refluxing for 24 hours, the reaction mixture was allowed to stand for cooling. This was poured in ice water and the organic layer was separated. Then, the aqueous layer was extracted with methylene chloride. Respective organic layers were combined, washed with water, saturated aqueous solution of sodium hydrogencarbonate and brine, then dried over anhydrous sodium sulfate, and concentrated. The residue was purified by silica gel column chromatography (eluent n-hexane:ethyl acetate=5:1v/v) to afford 7.59g (52%) of the title compound as a yellow oil.
Mass analysis m/z 268(M⁺).

### <Example 73>: Benzyl 2-methylthio-2-(5-formyl-2-methoxyphenyl)acetate

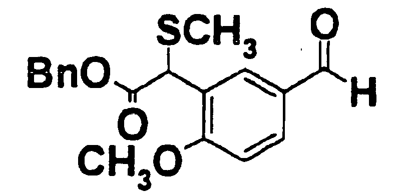

Ethyl 2-methylthio-2-(5-formyl-2-methoxyphenyl)acetate (4.80g, 17.9mmol), ethanol (30mL) and 10% aqueous solution of sodium hydroxide (20mL) were mixed and the mixture was stirred for 3 hours at room temperature. The reaction mixture was poured in ice water and made acidic with concentrated hydrochloric acid, which was then extracted with ethyl acetate. The extract was washed with water, dried over anhydrous sodium sulfate, and then concentrated to afford 4.00g of crude 2-methylthio-2-(5-formyl-2-methoxyphenyl)acetic acid as yellow crystals (this compound was used for next reaction without further purification). Next, 2-methylthio-2-(5-formyl-2-methoxyphenyl)acetic acid (4.00g, 16.4mmol) and N,N-dimethylformamide (50mL) were mixed and, after anhydrous potassium carbonate (3.71g, 26.8mmol) and benzyl bromide (3.06g, 17.9mmol) were added at room temperature under stirring, the mixture was stirred for 5 hours. The reaction mixture was poured in ice water and extracted with ether. The extracted solution was washed with water and brine, and then dried over anhydrous sodium sulfate. The reaction mixture was concentrated and the residue was purified by silica gel column chromatography (eluate n-hexane:ethyl acetate=6:1v/v) to afford 4.50g (76%) of the title compound as a light brown oil. Mass analysis m/z 330(M⁺).

### <Example 74>: Benzyl 2-methylthio-2-[5-[1-(2-ethoxycarbonyl)-phenyl]-2-methoxyphenyl]acetate

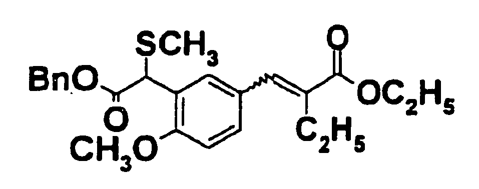

Under an argon atmosphere, triethyl 2-phosphonobutyrate (3.77 g, 14.9mmol) was dissolved in dehydrated tetrahydrofuran (40mL) and potassium *tert*-butoxide (1.68g, 15.0mmol) was added under stirring and cooling with ice. After completion of the addition, the mixture was stirred for 1 hour. Following this, benzyl 2-methylthio-2-(5-formyl-2-methoxyphenyl)acetate (4.49g, 13.6mmol) dissolved in anhydrous tetrahydrofuran (30mL) was added and the mixture was stirred for 6 hours at room temperature. The reaction mixture was concentrated and ice water was added, which was made acidic with 1 mol/L hydrochloric acid. The reaction mixture was extracted with ethyl acetate. The extract was washed with water and brine, dried over anhydrous sodium sulfate, and then concentrated. The residue was purified by silica gel column chromatography (eluent n-hexane:ethyl acetate=9:1v/v) to afford 4.55g (78%) of the title compound as a colorless oil.
Mass analysis m/z 428(M⁺).

### <Example 75>: Benzyl 2-[5-[1-(2-ethoxycarbonyl)-butenyl]-2-methoxyphenyl]acetate

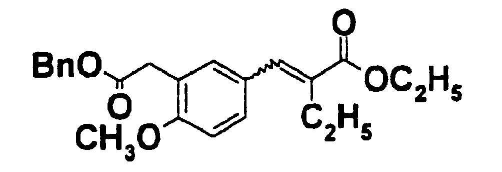

Benzyl 2-methylthio-2-[5-[1-(2-ethoxycarbonyl)butenyl]-2-methoxyphenyl]acetate (2.14g, 4.99mmol) and glacial acetic acid (100mL) were mixed and zinc powder (13.0g, 199mmol) was added under stirring at room temperature. After stirring for 6 hours at room temperature, the insolubles were remove by filtration and the filtrate was concentrated. The residue was dissolved into ethyl acetate (50mL), washed with water and brine, then dried over anhydrous sodium sulfate and concentrated to afford 1.86g (97%) of the title compound as a pale yellow oil.
Mass analysis m/z 382(M⁺).

### <Example 76>: 2-[5-(2-ethoxycarbonyl)butyl-2-methoxyphenyl]-acetic acid

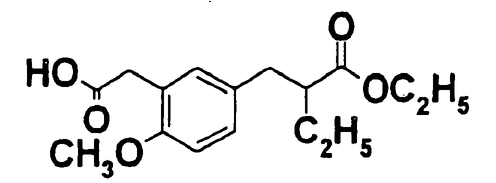

Benzyl 2-[5-[1-(2-ethoxycarbonyl)butenyl]-2-methoxyphenyl]-acetate (1.85g, 4.84mmol), 5% palladium on carbon (400 mg), tetrahydrofuran (50mL) and ethanol (50mL) were mixed and hydrogenation was carried out for 8 hours at room temperature. The catalyst was removed by filtration and the filtrate was concentrated to afford 1.42g (100%) of the title compound as a yellow oily product.
Mass analysis m/z 294(M⁺).

### <Example 77>: Ethyl 2-ethyl-3-[4-methoxy-3-[[N-[4-(trifluoromethyl)phenyl]carbamoyl]methyl]phenyl]propionate

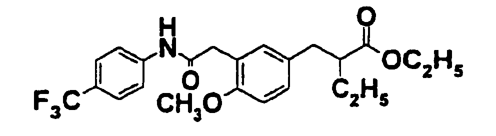

2-[5-(2-Ethoxycarbonyl)butyl-2-methoxyphenyl]acetic acid (500mg, 1.70mmol), 4-trifluoromethylaniline (300mg, 1.86mmol), triethylamine (260µL, 1.87mmol) and anhydrous N,N-dimethylformamide (6mL) were mixed and, under an argon atmosphere, stirring and cooling with ice, diethyl cyanophosphate (283µL, 1.87mmol) was added. After stirring for 6 hours at room temperature, the mixture was allowed to stand overnight. Ice water was added to the reaction mixture, which was then extracted with ethyl acetate. The extract was washed with water and brine, dried over anhydrous sodium sulfate and concentrated. The residue was purified by silica gel column chromatography (eluent n-hexane:ethyl acetate=6:1v/v) to afford 210mg (28%) of the title compound as a yellow oil.
Mass analysis m/z 437(M⁺).

### <Examples 78 through 87>

Similarly to Examples 77, compounds in Table 4 were obtained.

**Table 4**

| | | | | | |
|---|---|---|---|---|---|
| 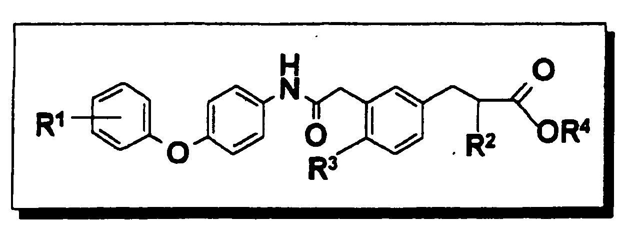 | | | | | |

| Example | R¹ | R² | R³ | R⁴ | Mass analysis (m/z) |
|---|---|---|---|---|---|
| 78 | 2-CH₃ | C₂H₅ | OCH₃ | C₂H₅ | 475 (M⁺) |
| 79 | 3-CH₃ | C₂H₅ | OCH₃ | C₂H₅ | 475 (M⁺) |
| 80 | 4-CH₃ | C₂H₅ | OCH₃ | C₂H₅ | 475 (M⁺) |
| 81 | 2-OCH₃ | C₂H₅ | OCH₃ | C₂H₅ | 491 (M⁺) |
| 82 | 3-OCH₃ | C₂H₅ | OCH₃ | C₂H₅ | 491 (M⁺) |
| 83 | 4-OCH₃ | C₂H₅ | OCH₃ | C₂H₅ | 491 (M⁺) |
| 84 | 2-F | C₂H₅ | OCH₃ | C₂H₅ | 479 (M⁺) |
| 85 | 3-F | C₂H₅ | OCH₃ | C₂H₅ | 479 (M⁺) |
| 86 | 4-F | C₂H₅ | OCH₃ | C₂H₅ | 479 (M⁺) |
| 87 | 4-Cl | C₂H₅ | OCH₃ | C₂H₅ | 495 (M⁺) |

### <Example 88>: 2-Ethyl-3-[4-methooxy-3-[[N-[4-(trifluoromethyl)phenyl]carbamoyl]methyl]phenyl]propanoic acid

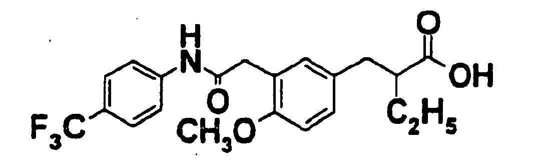

Ethyl 2-ethyl-3-[4-methoxy-3-[[N-[4-(trifluoromethyl)-phenyl]carbamoyl]methyl]phenyl]propanoate (206mg, 0.471mmol), ethanol (5mL) and 10% aqueous solution of sodium hydroxide (3mL) were mixed and the mixture was stirred for 6 hours at room temperature. The reaction mixture was poured into ice water and made acidic with 1mol/L hydrochloric acid, which was then extracted with ethyl acetate. The extract was washed with water and brine, dried over anhydrous sodium sulfate, and then concentrated. The residue was purified by silica gel column chromatography (eluent n-hexane:ethyl acetate=4:1→ 1:1v/v) and recrystallized from a mixed solvent of n-hexane with ethyl acetate to afford 142mg (73%) of the title compound as colorless powder.
Melting point 136-137°C.
Mass analysis m/z 409(M⁺).
Elemental analysis C21H22F3NO4 (409.40):
Calc. C, 61.61; H, 5.42; N, 3.42.
Found C, 61.48; H, 5.39; N, 3.51.
¹H-NMR (400MHz, CDC13) δ0.96 (3H, t, J=7.3Hz), 1.54-1.73 (2H, m), 2.55-2.62 (1H, m), 2.74 (1H, dd, J=14.2, 5.9Hz), 2.88 (1H, dd, J= 14.2, 8.8Hz), 3.68 (2H, s), 3.91 (3H, s), 6.87 (1H, d, J=9.3Hz), 7.11-7.14 (2H, m), 7.51 (2H, d, J=8.8Hz), 7.54 (2H, d, J=8.8Hz), 7.90 (1H, s).

### <Examples 89 through 98)

Similarly to Example 88, compounds in Table 5 were obtained.

**Table 5**

| | | | | | |
|---|---|---|---|---|---|
| 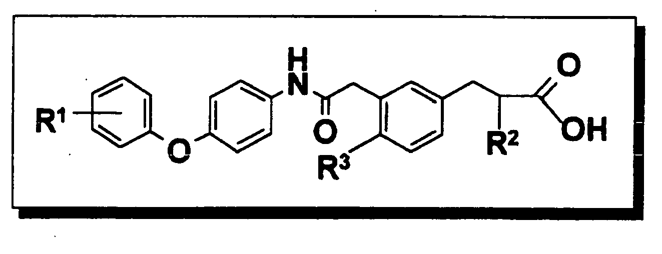 | | | | | |

| Example | R¹ | R² | R³ | Melting point(°C) | Mass analysis(m/z) |
|---|---|---|---|---|---|
| 89 | 2-CH₃ | C₂H₅ | OCH₃ | 130-132 | 447 (M⁺) |
| 90 | 3-CH₃ | C₂H₅ | OCH₃ | 123-125 | 447 (M⁺) |
| 91 | 4-CH₃ | C₂H₅ | OCH₃ | 147-149 | 447 (M⁺) |
| 92 | 2-OCH₃ | C₂H₅ | OCH₃ | 100-102 | 463 (M⁺) |
| 93 | 3-OCH₃ | C₂H₅ | OCH₃ | 120-122 | 463 (M⁺) |
| 94 | 4-OCH₃ | C₂H₅ | OCH₃ | 127-129 | 463 (M⁺) |
| 95 | 2-F | C₂H₅ | OCH₃ | 136-137 | 451 (M⁺) |
| 96 | 3-F | C₂H₅ | OCH₃ | 111-113 | 451 (M⁺) |
| 97 | 4-F | C₂H₅ | OCH₃ | 85-87 | 451 (M⁺) |
| 98 | 4-Cl | C₂H₅ | OCH₃ | 94-96 | 467 (M⁺) |

### <Example 99>: Ethyl 2-ethyl-3-(4-methoxyphenyl)acrylate

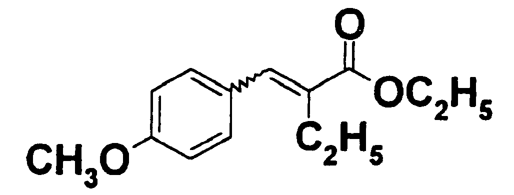

Under an argon atmosphere, stirring and cooling with ice, a solution of triethyl 2-phosphonobutyrate (5.55g, 22.0mmol) in anhydrous tetrahydrofuran (5mL) was added dropwise to a solution of potassium tert-butoxide (2.47g, 22.0mmol) in anhydrous tetrahydrofuran (30mL), and then the mixture was stirred for 20 minutes. Next, after a solution of p-anisaldehyde (2.72g, 20.0mmol) in anhydrous tetrahydrofuran (5mL) was added, the mixture was stirred for 1 hour under cooling with ice and for 4 hours at room temperature. Cooled 0.5 mol/L hydrochloric acid (100mL) was added to the reaction mixture, which was extracted with ethyl acetate. The organic layer was washed with water and brine, then dried over anhydrous sodium sulfate and concentrated. The residue was purified by silica gel column chromatography (eluent n-hexane:ethyl acetate=15:1v/v) to afford 4.58g (98%) of the title compound as a colorless oily product.
Mass analysis m/z 234(M⁺).

### <Example 100>: Ethyl 2-ethyl-3-(4-methoxyphenyl)propanoate

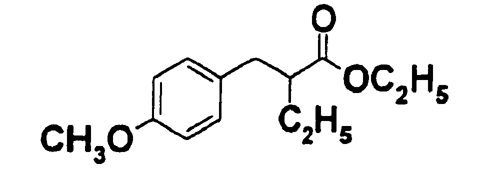

Ethyl 2-ethyl-3-(4-methoxyphenyl)acrylate (4.58g, 19.5mmol), 5% palladium on carbon (500mg) and ethyl acetate (100 mL) were mixed and hydrogenated for 8 hours at room temperature. After the catalyst was removed by filtration, the filtrate was concentrated to afford 4.63g (100%) of the title compound as a pale yellow oil.
Mass analysis m/z 236(M⁺).

### <Example 101>: Ethyl 3-(3-acetyl-4-methoxyphenyl)-2-ethylpropanoate

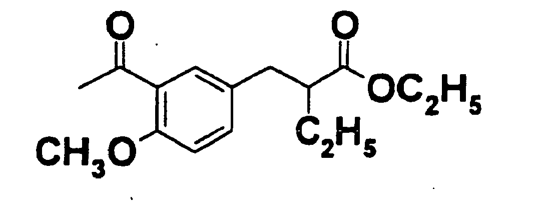

Under stirring and cooling with ice, acetyl chloride (1.00mL, 14.1mmol) wad added dropwise to a suspension of aluminum chloride (III) (1.78g, 13.3mmol) in dichloromethane (30mL), and then the mixture was stirred for 30 minutes. Next, a solution of ethyl 2-ethyl-3-(4-methoxyphenyl)propanoate (2.10g, 8.89mmol) in dichloromethane (10mL) was added dropwise. After stirring for 1 hour under cooling with ice, the mixture was allowed to stand overnight at room temperature. The reaction mixture was poured in ice water, the dichloro-methane layer was separated and the aqueous layer was extracted with dichloromethane. The organic layers were combined and washed with water and brine, dried over anhydrous sodium sulfate, and then concentrated. The residue was-purified by silica gel column chromatography (eluent n-hexane:ethyl acetate=6:1v/v) to afford 1.21g (49%) of the title compound as a yellow oily product.
Mass analysis m/z 278(M⁺).

### <Example 102>: Ethyl 3-[3-(2-ethoxycarbonylacetyl)-4-methoxy-phenyl]-2-ethylpropanoate

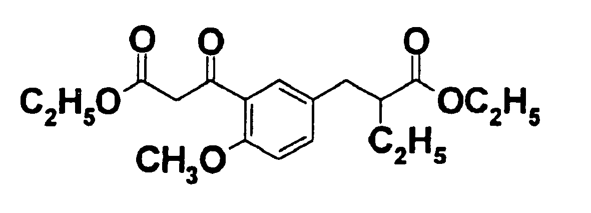

To anhydrous diethyl ether (20mL), sodium hydride (60% oil dispersion, 1.27 g, 52.9mmol) was added under stirring and cooling with ice. Successively, after diethyl carbonate (2.25g, 19.0mmol) was added, the mixture was stirred for 30 minutes at room temperature. Following this, a mixture of ethyl 3-(3-acetyl-4-methoxyphenyl)-2-ethylpropanoate (3.53g, 12.7mmol), anhydrous diethyl ether (10mL) and absolute ethanol (0.24mL) was added dropwise over 20 minutes, and then the mixture was refluxed for 6 hours. After cooling, the reaction mixture was added slowly to a mixed solution of 2mol/L hydrochloric acid (45mL) with ethyl acetate (80mL) under stirring and cooling with ice. The organic layer was separated, and the aqueous layer was extracted with ethyl acetate. The organic layers were combined and washed with water and brine, dried over anhydrous sodium sulfate, and then concentrated. The residue was purified by silica gel column chromatography (eluent n-hexane:ethyl acetate=9:1v/v) to afford 2.59g (58%) of the title compound as a pale yellow oil.
Mass analysis m/z 350(M⁺).

### <Example 103>: Ethyl 3-[3-[2-ethoxycarbonyl-3-[4-(trifluoromethyl)phenyl]propionyl]-4-methoxyphenyl]-2-ethylpropanoate

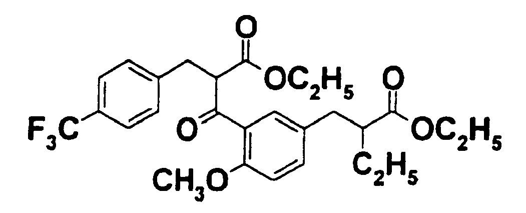

Under an argon atmosphere, stirring and cooling with ice, sodium hydride (60% oil dispersion, 296mg, 7.40mmol) was added little by little to a solution of ethyl 3-[3-(2-ethoxycarbonylacetyl)-4-methoxyphenyl]-2-ethylpropanoate (2.59g, 7.39mmol) in anhydrous tetrahydrofuran (30mL), and then the mixture was stirred for 20 minutes under cooling with ice and for 30 minutes at room temperature. Following this, a solution of 4-(trifluoromethyl)benzylbromide (1.77g, 7.40mmol) in anhydrous tetrahydrofuran (10mL) was added dropwise. After completion of the dropwise addition, the mixture was refluxed for 6 hours. After cooling, a mixture of 1mol/L hydrochloric acid (15mL) with ice water (100mL) was added thereto, which was extracted with ethyl acetate. The organic layer was washed with water and brine, dried over anhydrous sodium sulfate, and then concentrated. The residue was purified by silica gel column chromatography (eluent n-hexane:ethyl acetate=8:1v/v) to afford 3.70g (98%) of the title compound as a pale yellow oil.
Mass analysis m/z 508(M⁺).

### <Example 104>: 2-Ethyl-3-[3-[3-[4-(trifluoromethyl)phenyl]-propionyl]-4-methoxyphenyl]propanoic acid

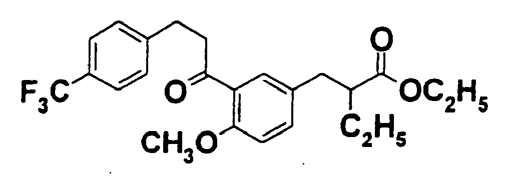

A mixture of ethyl-3-[3-[2-ethoxycarbonyl-3-[4-(trifluoromethyl)phenyl]propionyl]-4-methoxyphenyl]-2-ethylpropanoate (2.62g, 5.15 mmol), glacial acetic acid (10mL) and concentrated hydrochloric acid (5mL) was refluxed for 5 hours. After cooling, the mixture was poured into ice water, which was extracted with ethyl acetate. The organic layer was washed with water and brine, dried over anhydrous sodium sulfate, and then concentrated. The residue was purified by silica gel column chromatography (eluent n-hexane:ethyl acetate:acetic acid=400:100:1.5v/v/v) to afford 1.47g (70%) of the title compound as a pale yellow oil.
Mass analysis m/z 408(M⁺).
Elemental analysis C22H23F3O4 (408.41):
Calc. (%) C, 64.70; H, 5.68.
Found (%) C, 64.42; H, 5.71.
¹H-NMR (400MHz, CDCl3) δ0.95 (3H, t, J=7.3Hz), 1.54-1.69 (2H, m), 2.54-2.61 (1H, m), 2.73 (1H, dd, J=13.7, 6.8Hz), 2.92 (1H, dd, J= 13.7, 7.8Hz), 3.06 (2H, t, J=7.3Hz), 3.31 (2H, t, J=7.3Hz), 3.85 (3H, s), 6.87 (1H, d, J=8.8Hz), 7.28 (1H, dd, J=8.3, 2.4Hz), 7.34 (2H, d, J=7.8Hz), 7.52-7.54 (3H, m).

### <Example 105>: 2-Ethyl-3-[3-[3-[4-(trifluoromethyl)pheyl]-propyl]-4-methoxyphenyl]propanoic acid

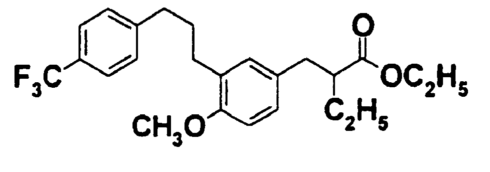

A solution of 2-ethyl-3-[3-[3-[4-(trifluoromethyl)phenyl]-propionyl]-4-methoxyphenyl]propanoic acid (224mg, 0.548mmol) in ethanol (30mL) was hydrogenated for 7 hours with 10% palladium on carbon (250mg) at room temperature under a hydrogen pressure of 392kPa. The catalyst was removed by filtration and the filtrate was concentrated. The residue was purified by means of thin layer silica gel column chromatography (eluent: n-hexane: ethyl acetate:acetic acid=200:100:2v/v/v) to afford 185mg (86%) of the title compound as a yellow oil.
Mass analysis m/z 394(M⁺).
Elemental analysis C22H25F3O3 (394.43):
Calc. (%) C, 66.99; H, 6.39.
Found (%) C, 66.92; H, 6.39.
¹H-NMR (400MHz, CDCl3) δ0.94 (3H, t, J=7.3Hz), 1.52-1.67 (2H, m), 1.87-1.94 (2H, m), 2.53-2.71 (6H, m), 2.88 (1H, dd, J=7.8, 13.7Hz), 3.78 (3H, s), 6.74 (1H, d, J=8.3Hz), 6.92 (1H, d, J=2.4Hz), 6.98 (1H, dd, J=8.3, 2.4Hz), 7.29 (2H, d, J=8.3Hz), 7.52 (2H, d, J=8.3 Hz).

### <Example 106>: Ethyl 2-ethyl-3-(3-formyl-4-methoxyphenyl)-propanoate

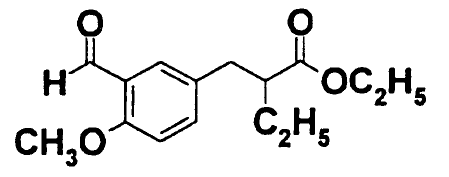

Under an argon atmosphere, stirring and cooling at -20°C, titanium chloride (IV) (35.0mL, 318mmol) and dichloromethyl methyl ether (13.4mL, 148mmol) were added dropwise in sequence to a solution of ethyl 2-ethyl-3-(4-methoxyphenyl)propanoate (10.0g, 42.3 mmol) in dichloromethane (500mL). The mixture was stirred for 6 hours at -20°C to -5°C. The reaction mixture was poured into a mixture of ice (600g) with 3mol/L hydrochloric acid (400mL) and the dichloromethane layer was separated, which was washed with water and brine, dried over anhydrous sodium sulfate, and then concentrated. The residue was purified by silica gel column chromatography (eluent n-hexane:ethyl acetate=4:1v/v) to afford 10.9g (97%) of the title compound as a light brown oily product.
Mass analysis m/z 264(M⁺).

### <Example 107>: Ethyl 2-ethyl-3-[3-(hydroxyimino)-4-methoxyphenyl]propanoate

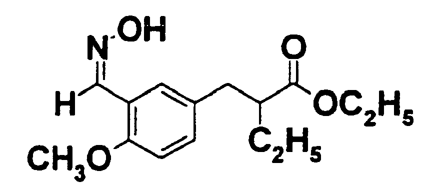

A mixture of ethyl 2-ethyl-3-(3-formyl-4-methoxyphenyl)-propanoate (1.06g, 4.01 mmol), hydroxylamine hydrochloride (293mg, 4.22 mmol), pyridine (1mL) and ethanol (20mL) was refluxed for 6 hours. After cooling, the mixture was concentrated and the residue was dissolved in ethyl acetate, which was washed with 1mol/L hydrochloric acid, saturated aqueous solution of sodium hydrogencarbonate and brine, dried over anhydrous sodium sulfate, and then concentrated. The residue was purified by silica gel column chromatography (eluent: n-hexane:ethyl acetate =6:1v/v) to afford 1.00g (89%) of the title compound as a pale yellow oil.
Mass analysis m/z 279(M⁺).

### <Example 108>: Ethyl 3-[3-(aminomethyl)-4-methoxyphenyl]-2-ethyl-propanoate hydrochloride

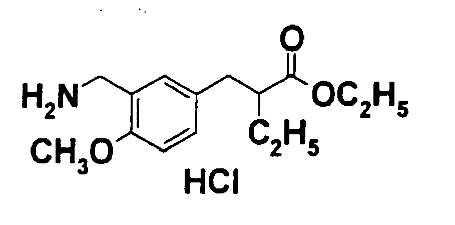

To a solution of ethyl 2-ethyl-3-[3-(hydroxyimino)-4-methoxyphenyl]propanoate (1.00g, 3.58mmol) in ethanol (80mL), 10% palladium on carbon (500mg) and concentrated hydrochloric acid (4mL) were added and hydrogenation was performed for 2 hours at room temperature under a hydrogen pressure of 392kPa. After water (80mL) was added to the reaction mixture, the catalyst was removed by filtration, which was washed with 50% water-containing ethanol. The filtrate was concentrated and the residue obtained was suspended into diethyl ether (20mL). The crystals were collected by filtration, washed with diethyl ether, and then dried to afford 914mg (85%) of the title compound as colorless crystals.
Mass analysis m/z 265(M⁺).

### <Example 109>: Ethyl 2-ethyl-3-[3-[4-(trifluoromethyl) benzoylaminomethyl]-4-methoxyphenyl]propanoate

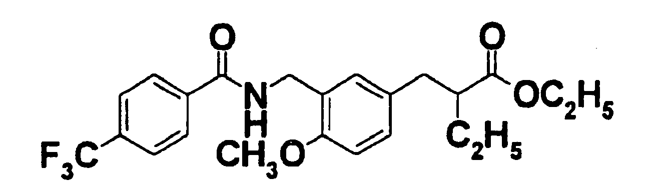

To a solution of 4-(trifluoromethyl)benzoic acid (114mg, 0.600 mmol) and triethylamine (0.21mL, 1.51mmol) in dichloromethane (3mL), ethyl chlorocarbonate (0.063mL, 0.659mmol) was added under stirring and cooling with ice, and the mixture was stirred for 20 minutes. Next, after ethyl 2-ethyl-3-[3-(aminomethyl)-4-methoxyphenyl]propanoate hydrochloride (200mg, 0.663mmol) was added, the mixture was stirred for 1 hour under cooling with ice and for 3 hours at room temperature. Dichloromethane (20mL) was added to the reaction mixture, which was washed with 1mol/L hydrochloric acid, saturated aqueous solution of sodium hydrogencarbonate and brine, dried over anhydrous sodium sulfate, and then concentrated. The residue was purified by silica gel column chromatography (eluent: n-hexane:ethyl acetate=4:1v/v) to afford 245mg (93%) of the title compound as colorless crystals. Mass analysis m/z 437(M⁺).

### <Examples 110 and 111>

Similarly to Example 109, compounds in Table 6 were obtained.

**Table 6**

| | | | | | |
|---|---|---|---|---|---|
| 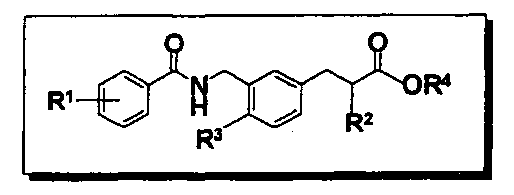 | | | | | |

| Example | R¹ | R² | R³ | R⁴ | Mass analysis (m/z) |
|---|---|---|---|---|---|
| 110 | 4-OPh | C₂H₅ | OCH₃ | C₂H₅ | 461 (M⁺) |
| 111 | 4-OPh(4-F) | C₂H₅ | OCH₃ | C₂H₅ | 479 (M⁺) |

### <Example 112>: 2-Ethyl-3-[3-[4-(trifluoromethyl)benzoylaminomethyl]-4-methoxyphenyl]propanoic acid

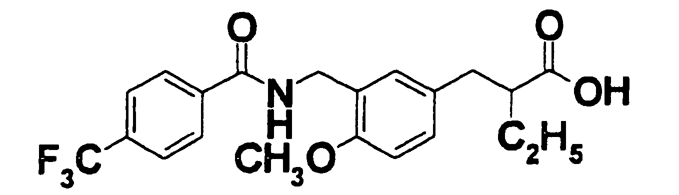

A mixture of ethyl 2-ethyl-3-[3-[4-(trifluoromethyl)-benzoylaminomethyl]-4-methoxyphenyl]propanoate (240mg, 0.549mmol), methanol (6mL) and 2.5mol/L aqueous solution of sodium hydroxide (2mL) was stirred for 4 hours under heating at 50°C. After cooling, water (20 mL) was added and the mixture was made acidic with 1mol/L hydrochloric acid, which was then extracted with ethyl acetate. The organic layer was washed with water and brine, dried over anhydrous sodium sulfate, and then concentrated to afford 222mg (99%) of the title compound as colorless crystals. Further, these were recrystallized from diethyl ether-n-hexane, thus affording 161mg (72%) of purified title compound as colorless needles.
Melting point 132-1330°C.
Mass analysis m/z 409(M⁺).
Elemental analysis C21H22F3NO4 (409.40):
Calc. (%) C, 61.61; H, 5.42; N, 3.42.
Found (%) C, 61.70; H, 5.56; N, 3.47.
¹H-NMR (400MHz, CDCl₃) δ0.95 (3H, t, J=7.3Hz), 1.54-1.70 (2H, m), 2.51-2.59 (1H, m), 2.71 (1H, dd, J=13.7, 6.3Hz), 2.88 (1H, dd, J=13.7, 8.3Hz), 3.85 (3H, s), 4.58 (2H, d, J=5.9Hz), 6.78-6.82 (2H, m), 7.10 (1H, dd, J=8.3, 2.4Hz), 7.16 (1H, d, J=2.4Hz), 7.65 (2H, d, J=8.3 Hz), 7.85 (2H, d, J=8.3 Hz).

### <Examples 113 and 114>

Similarly to Example 112, compounds shown in Table 7 were synthesized.

**Table 7**

| | | | | | | |
|---|---|---|---|---|---|---|
| 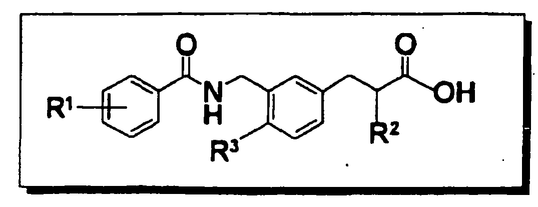 | | | | | | |

| Example | R¹ | R² | R³ | Melting point(°C) | Charac. formula | Elemental analysis(%) |
|---|---|---|---|---|---|---|
| 113 | 4-OPh | H | OCH₃ | 103-104 | C₂₆H₂₇NO₅ | Calc.; C 72.04, H 6.28, N 3.23 |
| | | | | | | Found; C 71.67, H 6.24, N 3.37 |
| 114 | 4-OPh(4-F) | H | OCH₃ | 126-128 | C₂₆B₂₆FNO₅ | Calc.; C 69.17, H 5.80, N 3.10 |
| | | | | | | Found; C 68.91, H 5.80, N 3.13 |

### <Example 115>: Ethyl-2-ethyl-3-[3-[4-(trifluoromethyl)-benzylaminomethyl]-4-methoxyphenyl]proyanoate

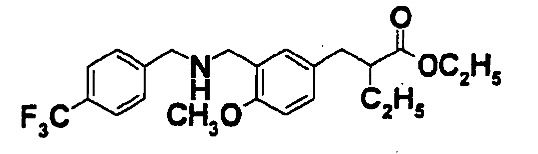

To a solution of ethyl 3-[3-(aminomethyl)-4-methoxyphenyl]-2-ethylpropanoate hydrochloride (203mg, 0.673mmol) in N,N-dimethylformamide (4mL), potassium carbonate (232mg, 1.68mmol) and 4-(trifluoromethyl)benzyl bromide (169mg, 0.707mmol) were added, and then the mixture was stirred for 3 hours at room temperature and for 4 hours under heating at 60°C. After cooling, the mixture was poured in ice water, which was extracted with diethyl ether. The organic layer was washed with water and brine, dried over anhydrous sodium sulfate, and then concentrated. The residue was purified by silica gel column chromatography (eluent: n-hexane:ethyl acetate=2:1v/v) to afford 92.3mg (32%) of the title compound as a colorless oil.
Mass analysis m/z 424(M+H).

### <Example 116>: 2-Ethyl 3-[3-[4-(trifluoromethyl)benzylamino-methyl]-4-methoxyphenyl]propanoic acid hydrochloride

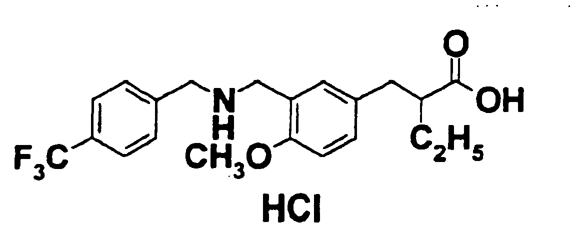

A mixture of ethyl 2-ethyl-3-[3-[4-(trifluoromethyl)-benzylaminomethyl]-4-methoxyphenyl]propanoate (90.2mg, 0.213mmol), methanol (2mL) and 2.5mol/L aqueous solution of sodium hydroxide (2mL) was stirred for 5 hours under heating at 60°C. After cooling, ice water (20mL) was added and the mixture was made acidic with 1mol/L hydrochloric acid, which was then saturated with sodium chloride and extracted with ethyl acetate. The extract was washed with saturated brine, dried over anhydrous sodium sulfate, and then concentrated. The residue was purified by silica gel column chromatography (eluent: dichloromethane:methanol =10:1v/v) to afford 78.0mg (85%) of the title compound as a brown oil.
Mass analysis As C₂₁H₂₄F₃NO₃, m/z 394(M-1)⁺.
Elemental analysis C₂₁H₂₄F₃NO₃·HCl (431.88):
Calc. (%) C, 58.40; H, 5.83; N, 3.24.
Found (%) C, 58.48; H, 6.07; N, 3.03.
¹H-NMR (400MHz, CDCl₃) δ0.99 (3H, t, J=7.3Hz), 1.54-1.61 (1H, m), 1.71-1.78 (1H, m), 2.52-2.57 (1H, m), 2.71-2.83 (2H, m), 3.72 (3H, s), 3.88-3.98 (4H, m), 6.67 (1H, d, J=8.8Hz), 7.10 (1H, dd, J=2.0, 8.8Hz), 7.30 (1H, d, J=2.0Hz), 7.63 (2H, d, J=8.3Hz), 7.71 (2H, d, J=8.3Hz).

### <Example 117>: 4-Methoxy-3-[2-[4-(trifluoromethyl)phenyl]-ethoxy]benzaldehyde

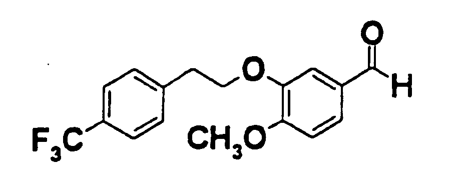

Under an argon atmosphere, stirring and cooling with ice, diethyl azodicarboxylate (40% toluene solution, 3.80mL, 8.38mmol) was added dropwise to a solution of 2-[4-(trifluoromethyl)phenyl]ethanol (1.59g, 8.36mmol), isovanillin (1.28g, 8.41mmol) and triphenylphosphine (2.20g, 8.39mmol) in anhydrous tetrahydrofuran (50 mL). After completion of the dropwise addition, the mixture was stirred for 1 hour under cooling with ice, and then allowed to stand overnight at room temperature. The reaction mixture was concentrated and the residue was purified by silica gel column chromatography (eluent: n-hexane:ethyl acetate=6:1v/v) to afford 1.96g (72%) of the title compound as colorless crystals.
Mass analysis m/z 324(M⁺).

### <Example 118>: Ethyl 2-ethyl-3-[4-methoxy-3-[2-[4-(trifluoromethyl)phenyl]ethoxy]phenyl]acrylate

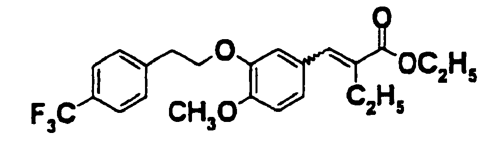

Under an argon atmosphere, stirring and cooling with ice, a solution of triethyl 2-phosphonobutyrate (555mg, 2.20mmol) in anhydrous tetrahydrofuran (5mL) was added dropwise to a solution of potassium tert-butoxide (250mg, 2.23mmol) in anhydrous tetrahydrofuran (10mL), and then the mixture was stirred for 20 minutes. Following this, a solution of 4-methoxy-3-[2-[4-(trifluoromethyl)phenyl]ethoxy]benzaldehyde (650mg, 2.00mmol) in anhydrous tetrahydrofuran (5mL) was added dropwise and then the mixture was stirred for 1 hour under cooling with ice and for 4 hours at room temperature. Cooled 0.5mol/L hydrochloric acid (30mL) was added to the reaction mixture, which was extracted with ethyl acetate. The organic layer was washed with water and brine, dried over anhydrous sodium sulfate, and then concentrated. The residue was purified by silica gel column chromatography (eluent: n-hexane:ethyl acetate=6:1v/v) to afford 842mg (99%) of the title compound as a colorless oily product.
Mass analysis m/z 422(M⁺).

### <Example 119>: Ethyl 2-ethyl-3-[4-methoxy-3-[2-[4-(trifluoromethyl)phenyl]ethoxy]phenyl]propanoate

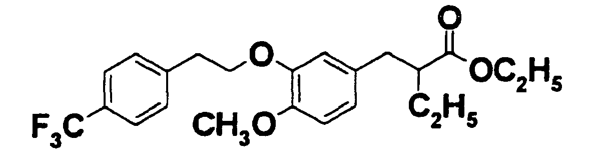

Ethyl 2-ethyl-3-[4-methoxy-3-[2-[4-(trifluoromethyl)-phenyl]ethoxy]phenyl]acrylate (840mg, 1.99mmol), 5% palladium on carbon (100mg) and ethyl acetate (50mL) were mixed, and hydrogenation was carried out for 6 hours at room temperature. The catalyst was removed by filtration, and then the filtrate was concentrated to afford 803mg (95%) of the title compound as colorless crystals.
Mass analysis m/z 424(M⁺).

### <Example 120>: 2-Ethyl-3-[4-methoxy-3-[2-[4-(trifluoromethyl)phenyl]ethoxy]phenyl]propanoic acid

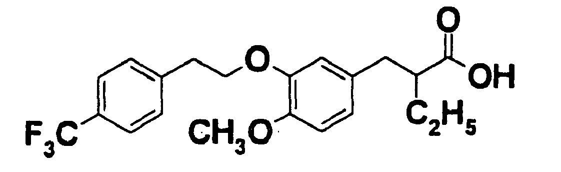

A mixture of ethyl 2-ethyl-3-[4-methoxy-3-[2-[4-(trifluoromethyl)phenyl]ethoxy]phenyl]propanoate (793mg, 1.87mmol), methanol (20 mL) and 1mol/L aqueous solution of sodium hydroxide (10mL) was stirred for 4 hours at 60°C. After cooling, ice water (50 mL) was added and the mixture was made acidic with 1mol/L hydrochloric acid, which was then extracted with ethyl acetate. The organic layer was washed with water and brine, dried over anhydrous sodium sulfate, and then concentrated. The residue was purified by silica gel column chromatography (eluent: n-hexane:ethyl acetate=4:1v/v) to afford 654mg (88%) of the title compound as colorless crystals. Further, these were recrystallized from diethyl ether-n-hexane, thus obtaining 391mg of the purified title compound as colorless needles.
Melting point 64-66°C.
Mass analysis m/z 396(M+).
Elemental analysis C₂₁H₂₃F₃O₄ (396.40):
Calc. (%) C, 63.63; H, 5.85.
Found (%) C, 63.66; H, 5.80.
¹H-NMR (400MHz, CDCl₃) δ0.93 (3H, t, J=7.3Hz), 1.51-1.68 (2H, m), 2.51-2.58 (1H, m), 2.66 (1H, dd, J=13.7, 6.8Hz), 2.87 (1H, dd, J= 13.7, 7.8Hz), 3.18 (2H, t, J=7.3Hz), 3.82 (3H, s), 4.20 (2H, t, J= 7.3Hz), 6.68 (1H, d, J=2.0Hz), 6.73 (1H, dd, J=7.8, 2.0Hz), 6.79 (1H, d, J=8.3Hz), 7.41 (2H, d, J=8.3Hz), 7.56 (2H, d, J=8.3Hz).

### <Example 121>: (+)-2-Ethyl-3-[4-methoxy-3-[[N-[4-(trifluoromethyl)phenyl]carbamoyl]methyl]phenyl]propanoic acid and (-)-2-ethyl-3-[4-methoxy-3-[[N-[4-(trifluoromethyl)phenyl]-methyl]carbamoyl]phenyl]propanoic acid

(±)-2-Ethyl-3-[4-methoxy-3-[[N-[4-(trifluoromethyl)-phenyl]carbamoyl]methyl]phenyl]propanoic acid (compound in Example 88: 14.2g, 34.7mmol) and anhydrous tetrahydrofuran (100mL) were mixed under an argon atmosphere, and triethylamine (4.86ml, 35.0mmol) and pivaloyl chloride (4.27mL, 34.7mmol) were added dropwise under stirring and cooling with ice. Thereafter, the mixture was stirred for 1 hour at room temperature to synthesize mixed acid anhydride. On the other hand, potassium tert-butoxide (4.67g, 41.6mmol) and dehydrated tetrahydrofuran (45mL) were mixed under cooling with ice and argon atmosphere in another vessel, and (S)-4-benzyloxazolidine-2- one (7.38g, 41.6mmol) dissolved into dehydrated tetrahydrofuran (60 mL) was added dropwise. After completion of the dropwise addition, the mixture was stirred for 30 minutes. Next, a suspension of the mixed acid anhydride synthesized previously was added dropwise while filtering under an atmosphere of argon. After completion of the dropwise addition, the reaction mixture was concentrated and then poured in water, which was extracted with ethyl acetate. The extracte was washed with 5% hydrochloric acid, saturated aqueous solution of sodium hydrogencarbonate and brine, then dried over anhydrous magnesium sulfate, and concentrated. The residue was purified by means of silica gel column chromatography (eluent: isopropyl ether) to afford 2.65g (13%) of (4S)-3-[2-ethyl-3-[4-methoxy-3-[[N-[4-(trifluoromethyl)phenyl]carbamoyl]methyl]phenyl]-propanoyl]-4-benzyloxazolidine-2-one with high polarity as a oily product. Also, 3.84g (20%) of (4S)-3-[2-ethyl-3-[4-methoxy-3-[[N-[4-(trifluoromethyl)phenyl]carbamoyl]-methyl]phenyl]propanoyl]-4-benzyloxazolidine-2-one with low polarity were obtained by purifying by silica gel column chromatography, followed by further recrystallization from a mixed solvent of n-hexane with isopropyl ether, as colorless powder.

The (4S)-3-[2-ethyl-3-[4-methoxy-3-[[N-[4-(trifluoromethyl)phenyl]carbamoyl]methyl]phenyl]propanoyl]-4-benzyloxazolidine-2-one with high polarity (2.65g, 4.66mmol) was dissolved into a mixed solvent (4:1v/v) (23mL) of tetrahydrofuran with water, and cooled with ice after replacement with argon. Under stirring, 30% aqueous hydrogen peroxide (1.89mL, 18.6mmol) was added dropwise over 5 minutes. Following this, lithium hydroxide monohydrate (313mg, 7.46 mmol) dissolved into water (7.8mL) was added dropwise over 5 minutes, and the mixture was stirred further for 4 hours under cooling with ice. To the reaction mixture, 64% sodium hydrogensulfite (2.98g, 18.6mmol) dissolved into water (12mL) was added dropwise. The reaction mixture was concentrated and the residue was poured into ice water, which was made acidic by adding 5% hydrochloric acid, and then extracted with methylene chloride. The extract was washed with brine, then dried over anhydrous magnesium sulfate and concentrated. The residue was dissolved into isopropyl ether under heating and allowed to stand. The precipitated crystals were collected by filtration and dried. Further, after concentration, the filtrate was purified by silica gel column chromatography (eluent: methylene chloride:methanol=20:1→15:1v/v) to afford 1.37g (72%) of (+)-2-ethyl-3-[4-methoxy-3-[[N-[4-(trifluoromethyl)-phenyl]carbamoyl]methyl]phenyl]propanoic acid as colorless crystalline powder.
Melting point 119-120°C.
Mass analysis m/z 409(M⁺).
Elemental analysis (%) C₂₁H₂₂F₃NO₄ (409.40):
Calc. C, 61.61; H, 5.42; N, 3.42.
Found C, 61.48; H, 5.46; N, 3.45.
¹H-NMR (400MHz, CDCl₃) δ0.95 (3H, t, J=7.3Hz), 1.55-1.70 (2H, m), 2.56-2.61 (1H, m), 2.73 (1H, dd, J=13.7, 6.3Hz), 2.88 (1H, dd, J=13.7, 8.3Hz), 3.67 (2H, s), 3.90 (3H, s), 6.86 (1H, d, J=8.8Hz), 7.11-7.13 (2H, m), 7.50 (2H, d, J=8.8Hz), 7.53 (2H, d, J=8.8Hz), 7.93 (1H, br s).
Specific rotation [α]D²⁶ +21°(C 0.8, MeOH);
Optical purity 99% e.e.(Chiral Pak AD 0.0046×0.25m, eluate; n-hexane:isopropanol:trifluoroacetic acid=95:5:0.2v/v/v, detecting wavelength; 254nm, column temperature; 40°C, flow rate; 1.00mL/min).

On the other hand, using (4S)-3-[2-ethyl-3-[4-methoxy-3-[[N-[4-(trifluoromethyl)phenyl]carbamoyl]methyl]phenyl]-propionyl]-4-benzyloxazolidine-2-one with low polarity (3.84g, 6.75mmol), similar manipulation to the hydrolysis of (4S)-3-[2-ethyl-3-[4-methoxy-3-[[N-[4-(trifluoromethyl)phenyl]-carbamoyl]methyl]phenyl]propionyl]-4-benzyloxazolidine-2-one with high polarity was conducted to obtain (-)-2-ethyl-3-[4-methoxy-3-[[N-[4-(trifluoromethyl)phenyl]carbamoyl]methyl]-phenyl]propanoic acid (1.55g, 56%) as colorless crystalline powder.
Melting point 121-123°C.
Mass analysis m/z 409(M⁺).
Elemental analysis (%) C₂₁H₂₂F₃NO₄ (409.40):
Calc. C, 61.61; H, 5.42; N, 3.42.
Found C, 61.51; H, 5.47; N, 3.50.
¹H-NMR (400MHz, CDCl3) δ0.95 (3H, t, J=7.3Hz), 1.55-1.70 (2H, m), 2.55-2.61 (1H, m), 2.73 (1H, dd, J=13.7, 6.4Hz), 2.88 (1H, dd, J=13.7, 8.3Hz), 3.67 (2H, s), 3.91 (3H, s), 6.86 (1H, d, J=8.3Hz), 7.11-7.13 (2H, m), 7.50 (2H, d, J=8.8Hz), 7.54 (2H, d, J=8.8Hz), 7.91 (1H, br s).
Specific rotation [α]D²⁶ -22°(C 0.8, MeOH);
Optical purity 98% e.e.(Chiral Pak AD 0.0046×0.25m, eluate; n-hexane:isopropanol:trifluoroacetic acid=95:5:0.2v/v/v, detecting wavelength; 254nm, column temperature; 40°C, flow rate; 1.00mL/min).

### <Example 122>: (4-Methoxy-3-nitrophenyl)methanol

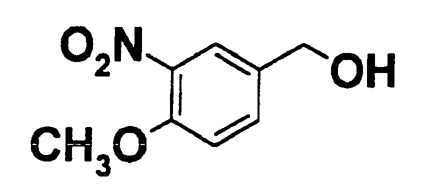

Under an argon atmosphere and cooling with ice, boranetetrahydrofuran complex (1.00mol/L, tetrahydrofuran solution, 100mL, 100 mmol) was added dropwise to a solution of 4-methoxy-3-nitrobenzoic acid (15.3g, 76.1mmol) in anhydrous tetrahydrofuran (200mL) under stirring. After completion of the dropwise addition, the mixture was allowed to stand overnight at room temperature. The reaction mixture was cooled with ice again and 6mol/L hydrochloric acid (20mL) was added dropwise. After stirring for 30 minutes, the mixture was concentrated under reduced pressure. The residue was poured into 1L of ice water and, after stirring for 30 minutes, the precipitates produced were collected by filtration, washed with water and dried to afford 12.5g (90%) of the title compound as pale yellow crystals.
Mass analysis m/z 183(M⁺).

### <Example 123>: 4-(Bromomethyl)-2-nitroanisole

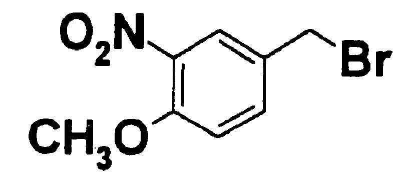

Under an argon atmosphere and cooling with ice, phosphorus tribromide (2.36mL, 24.7mmol) was added dropwise to a solution of (4-methoxy-3-nitrophenyl)methanol (12.2g, 66.6mmol) in anhydrous ether (350mL) under stirring, and the mixture was stirred for 3.5 hours. Ice water (300mL) was added to the reaction mixture. The organic layer was separated, washed with water and brine, dried over anhydrous sodium sulfate, and then concentrated. The residue was purified by means of silica gel column chromatography (eluent: n-hexane:ethyl acetate=2:1→ 1:1v/v) to afford 8.36g (51%) of the title compound as pale yellow crystals.
Mass analysis m/z 245(M⁺)

### <Example 124>: [3(2S),4R]-3-[2-ethyl-3-(4-methoxy-3-nitrophenyl)propionyl]-4-benzyloxazolidine-2-one

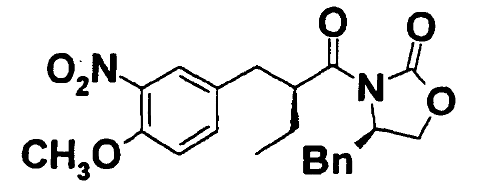

Under an argon atmosphere, sodium bis(trimethylsilyl)-amide (1.00mol/L tetrahydrofuran solution, 11.1mL, 11.1mmol) was added dropwise to a solution of (R)-3-(1-butyryl)-4-benzyloxazolidine-2-one (2.47g, 9.99mmol) in anhydrous tetrahydrofuran (50mL) at -78°C under stirring, and then the mixture was stirred for 1 hour. Following this, a solution of 4-(bromomethyl)-2-nitroanisole (2.72g, 11.1 mmol) in anhydrous tetrahydrofuran (30mL) was added dropwise, and the mixture was stirred for 4 hours at a temperature from -78°C to -60°C. Saturated aqueous solution of ammonium chloride (100mL) was added, which was extracted with ethyl acetate. The extracted solution was washed with water and brine, then dried over anhydrous sodium sulfate and concentrated. The residue was purified by silica gel column chromatography (eluent n-hexane:ethyl acetate=4:1→2:1v/v) to afford 3.40g (82%) of the title compound as pale yellow crystals.
Mass analysis m/z 412(M⁺).

### <Example 125>: [3(2S),4R]-3-[2-ethyl-3-(3-amino-4-methoxyphenyl)propanoyl]-4-benzyloxazolidine-2-one

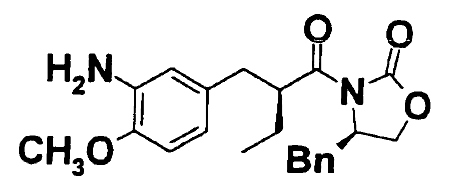

To [3(2S),4R]-3-[2-ethyl-3-(4-methoxy-3-nitrophenyl)-propanoyl]-4-benzyloxazolidine-2-one (3.39g, 8.22mmol), a mixed solvent (100mL) of tetrahydrofuran with ethanol (1:1v/v) and 10% palladium on carbon (0.34g) were added, and the mixture was stirred for 7.5 hours at room temperature under a hydrogen pressure of 294kPa. The catalyst was removed by filtration and the filtrate was concentrated to afford the title compound quantitatively as a yellowish brown amorphous material.
Mass analysis m/z 382(M⁺)

### <Example 126>: [3(2S),4R]-3-[2-ethyl-3-[4-methoxy-3-[2-[4 (trifluoromethyl)phenyl]acetylamino]phenyl]propanoyl]-4-benzyloxazolidine-2-one

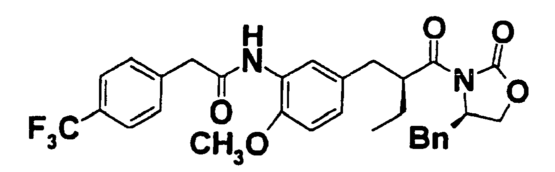

To a solution of 4-(trifluoromethyl)phenylacetic acid (0.889g, 4.22mmol) and [3(2S),4R]-3-[2-ethyl-3-(3-amino-4-methoxyphenyl)propanoyl]-4-benzyloxazolidine-2-one (1.11g, 2.82mmol) in dichloromethane (25mL), N-cyclohexylcarbodiimide-N'-methyl polystyrene HL (1.65mmol/g; 3.84g, 6.34mmol) was added, and the mixture was stirred for 7 hours at room temperature. The insolubles were removed by filtration and the filtrate was concentrated. The residue was purified by silica gel column chromatography (eluent: n-hexane:ethyl acetate =2:1v/v) to afford 1.57g (98%) of the title compound as a pale yellow amorphous material.
Mass analysis m/z 568(M⁺)

### <Example 127>: (S)-(+)-2-ethyl-3-[4-methoxy-3-[2-[4-(trifluoromethyl)phenyl]acetylamino]phenyl]propanoic acid

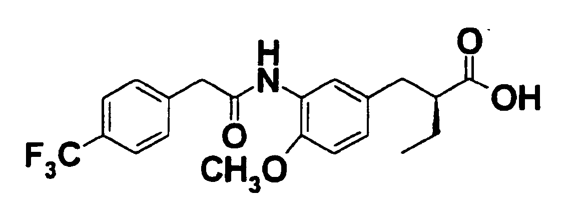

To a solution of [3(2S),4R]-3-[2-ethyl-3-[4-methoxy-3-[2-[4-(trifluoromethyl)phenyl]acetylamino]phenyl]propanoyl]-4-benzyloxazolidine-2-one (1.55g, 2.73mmol) in tetrahydrofuranethanol (4:1) (15mL), 30% aqueous hydrogen peroxide (1.11mL, 11.0mmol) and successively aqueous solution of lithium hydroxide monohydrate (5mL) were added dropwise under stirring and cooling with ice. After stirring for 3.5 hours, an aqueous solution of sodium hydrogensulfite (7mL) was added dropwise, the mixture was stirred for 10 minutes, and then concentrated. The residue was extracted with ethyl acetate, washed with water and brine, then dried over anhydrous sodium sulfate and concentrated. The residue was purified by column chromatography (eluent: methanol→mixed solvent of methanol with 1mol/L hydrochloric acid (9:1) that uses ion exchange resin (Amberlite IRA410) and silica gel column chromatography (eluent: n-hexane:ethyl acetate=2:1→1:2v/v) to afford 672mg (60%) of the title compound as a pale yellow amorphous material.
Mass analysis m/z 409(M⁺).
Elemental analysis C₂₁H₂₂F₃NO₄ (409.40):
Calcd. (%) C, 61.61; H, 5.42; N, 3.42.
Found (%) C, 61.39; H, 5.42; N, 3.46.
¹H-NMR (400MHz, CDCl₃) δ0.93 (3H, t, J=7.3Hz), 1.50-1.69 (2H, m), 2.55-2.64 (1H, m), 2.69 (1H, dd, J=13.7, 6.8Hz), 2.90 (1H, dd, J=13.7, 7.8Hz), 3.74 (3H, s), 3.79 (3H, s), 6.72 (1H, d, J=8.3Hz), 6.84 (1H, dd, J=8.3, 2.0Hz), 7.47 (2H, d, J=8.3Hz), 7.64 (2H, d, J= 8.3Hz), 7.75 (1H, br s), 8.21 (1H, d, J=2.0Hz). Specific rotation [α]D²⁶ +30°(C 0.80, MeCN);
Optical purity 99% e.e.(Chiralcel OJ, 0.0046×0.25m, eluate; n-hexane:isopropanol:trifluoroacetic acid=95:5:0.2, detecting wave-length; 254nm, column temperature; 40°C, flow rate;
1.00ml/min).

### (Biological activity)

### <Test example 1> Test of transcriptional activation on peroxisome proliferator-activated receptor α

To CHO cells cultured in a Dulbecco-modified Eagle's medium containing 10% delipid fetal calf serum (FCS/DMEM), receptor plasmid that expresses fused protein of DNA-binding domain being transcription factor of yeast with ligand-binding domain of human type PPARα (Biochemistry, 1993, 32, 5598), its reporter plasmid (STRATAGENE Corp.) and luciferase plasmid of Renilla (Promega Corp.) as internal standard were cotransfected with lipofectamine in the serum-free state. Thereafter, testing compound was added in the 10% SFCS/DMEM and both luciferase activities were measured after 24 hours, which were corrected with internal standard.

Results are shown in Table 8. From these results, it was shown that the inventive compounds had potent transcriptional activity on human peroxisome prolifertor-activated receptor α.

**Table 8**

| Example | Transcription-activating function EC50(µmol/L) |
|---|---|
| 44 | 0.13 |
| 46 | 0.05 |
| 70 | 0.16 |
| 88 | 0.015 |
| (8S)HETE | 1.30 |

### <Result>

From the results as described above, the inventive substituted phenylpropionic acid derivatives are novel compounds group with excellent binding activity to PPARα and transcriptional activation.

Based on the fact that these inventive compounds have potent working activity on PPARα, it can be said that they are effective compounds as lipid-decreasing drugs aforementioned, in particular, lipid-decreasing drugs for liver, and suppressing drugs for the progress of arterial sclerosis.

## Claims

1. Substituted phenylpropionic acid derivatives represented by a general formula (1) 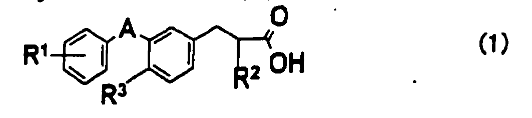 [wherein R¹ denotes a alkyl group with carbon atoms of 1 to 4, alkoxy group with carbon atoms of 1 to 3, trifluoromethyl group, trifluoromethoxy group, phenyl group which is unsubstituted or may have substituents, phenoxy group which is unsubstituted or may have substituents or benzyloxy group which is unsubstituted or may have substituents, R² denotes a hydrogen atom, alkyl group with carbon atoms of 1 to 4 or alkoxy group with carbon atoms of 1 to 3, R3 denotes a alkoxy group with carbon atoms of 1 to 3, and the binding mode of A portion denotes -OCH₂CO-, -COCH₂NH- or -NHCH₂CO-], their pharmaceutically acceptable salts and their hydrates.

2. Substituted phenylpropionic acid derivatives, their pharmaceutically acceptable salts and their hydrates of Claim 1, the steric configuration of R² substituent being as a general formula (1a) 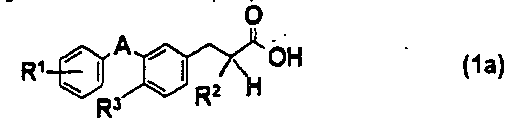 [wherein R¹ denotes a alkyl group with carbon atoms of 1 to 4, alkoxy group with carbon atoms of 1 to 3, trifluoromethyl group, trifluoromethoxy group, phenyl group which is unsubstituted or may have substituents, phenoxy group which is unsubstituted or may have substituents or benzyloxy group which is unsubstituted or may have substituents, R² denotes a alkyl group with carbon atoms of 1 to 4 or alkoxy group with carbon atoms of 1 to 3, R³ denotes a alkoxy group with carbon atoms of 1 to 3, and the binding mode of A portion denotes -CH₂CONH-, -NHCOCH₂-, -CH₂CH₂CH₂-, -CH₂CH₂O-, -CONHCH₂-, -CH₂NHCH₂-, -COCH₂O-, -OCH₂CO-, -COCH₂NH- or -NHCH₂CO-].

3. Substituted phenylpropionic acid derivatives, their pharmaceutically acceptable salts and their hydrates of Claim 1, R¹ being trifluoromethyl group.

4. Substituted phenylpropionic acid derivatives, their pharmaceutically acceptable salts and their hydrates of Claim 1, R¹ being benzyloxy group.

5. Substituted phenylpropionic acid derivatives, their pharmaceutically acceptable salts and their hydrates of Claim 1, R¹ being phenoxy group.

6. Substituted phenylpropionic acid derivatives, their pharmaceutically acceptable salts and their hydrates of Claim 1, R¹ being 4-fluorophenoxy group.

7. Substituted phenylpropionic acid derivatives, their pharmaceutically acceptable salts and their hydrates of Claim 1, R² being ethyl group.

8. Substituted phenylpropionic acid derivatives, their pharmaceutically acceptable salts and their hydrates of Claim 1, R² being n-propyl group.

9. Substituted phenylpropionic acid derivatives, their pharmaceutically acceptable salts and their hydrates of Claim 1, R² being methoxy group.

10. Substituted phenylpropionic acid derivatives, their pharmaceutically acceptable salts and their hydrates of Claim 1, one of them being 2-ethyl-3-[4-methoxy-3-[2-[4-(trifluoromethyl)phenyl]acetylamino]phenyl]propionic acid.

11. Substituted phenylpropionic acid derivatives, their pharmaceutically acceptable salts and their hydrates of Claim 1, one of them being 2-n-propyl-3-[4-methoxy-3-[2-[4-(trifluoromethyl)phenyl]acetylamino]phenyl]propionic acid.

12. Substituted phenylpropionic acid derivatives, their pharmaceutically acceptable salts and their hydrates of Claim 1, one of them being 2-ethyl-3-[4-methoxy-3-[2-(4-phenoxyphenyl)acetylamino]phenyl]propionic acid.

13. Substituted phenylpropionic acid derivatives, their pharmaceutically acceptable salts and their hydrates of Claim 1, one of them being 2-n-propyl-3-[4-methoxy-3-[2-(4-phenoxyphenyl)acetylamino]phenyl]propionic acid.

14. Substituted phenylpropionic acid derivatives, their pharmaceutically acceptable salts and their hydrates of Claim 1, one of them being 2-ethyl-3-[4-methoxy-3-[2-[4-(4-fluorophenoxy)phenyl]acetylamino]phenyl]propionic acid.

15. Substituted phenylpropionic acid derivatives, their pharmaceutically acceptable salts and their hydrates of Claim 1, one of them being 2-n-propyl-3-[4-methoxy-3-[2-[4-(4-fluorophenoxy)phenyl]acetylamino]phenyl]propionic acid.

16. Substituted phenylpropionic acid derivatives, their pharmaceutically acceptable salts and their hydrates of Claim 1, one of them being 2-n-propyl-3-[4-methoxy-3-[[N-[4-(trifluoromethyl)phenyl]carbamoyl]methyl]phenyl]propionic acid.

17. Substituted phenylpropionic acid derivatives, their pharmaceutically acceptable salts and their hydrates of Claim 1, one of them being 2-ethyl-3-[4-methoxy-3-[[N-(4-phenoxyphenyl)carbamoyl]methyl]phenyl]propionic acid.

18. Substituted phenylpropionic acid derivatives, their pharmaceutically acceptable salts and their hydrates of Claim 1, one of them being 2-n-propyl-3-[4-methoxy-3-[[N-(4-phenoxyphenyl)carbamoyl]methyl]phenyl]propionic acid.

19. Substituted phenylpropionic acid derivatives, their pharmaceutically acceptable salts and their hydrates of Claim 1, one of them being 2-ethyl-3-[4-methoxy-3-[[N-[4-(4-fluorophenoxy)phenyl]carbamoyl]methyl]phenyl]propionic acid.

20. Substituted phenylpropionic acid derivatives, their pharmaceutically acceptable salts and their hydrates of Claim 1, one of them being 2-n-propyl-3-[4-methoxy-3-[[N-[4-(4-fluorophenoxy)phenyl]carbamoyl]methyl]phenyl]propionic acid.

21. Substituted phenylpropionic acid derivatives, their pharmaceutically acceptable salts and their hydrates of Claim 1, one of them being 2-ethyl-3-[3-[4-(trifluoromethyl)-benzoylaminomethyl]-4-methoxyphenyl]propionic acid.

22. Substituted phenylpropionic acid derivatives, their pharmaceutically acceptable salts and their hydrates of Claim 1, one of them being 2-ethyl-3-[3-[4-(phenoxy)benzoylaminomethyl]-4-methoxyphenyl]propionic acid.

23. Substituted phenylpropionic acid derivatives, their pharmaceutically acceptable salts and their hydrates of Claim 1, one of them being 2-ethyl-3-[3-[4-(4-fluorophenoxy)-benzoylaminomethyl]-4-methoxyphenyl]propionic acid.

24. A lipid-decreasing drug having at least one or more kinds of substituted phenylpropionic acid derivatives represented by a general formula (1) 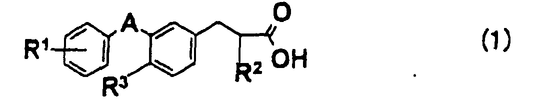 [wherein R¹ denotes a alkyl group with carbon atoms of 1 to 4, alkoxy group with carbon atoms of 1 to 3, trifluoromethyl group, trifluoromethoxy group, phenyl group which is unsubstituted or may have substituents, phenoxy group which is unsubstituted or may have substituents or benzyloxy group which is unsubstituted or may have substituents, R² denotes a hydrogen atom, alkyl group with carbon atoms of 1 to 4 or alkoxy group with carbon atoms of 1 to 3, R³ denotes a alkoxy group with carbon atoms of 1 to 3, and the binding mode of A portion denotes -CH₂NHCH₂-, -COCH₂O-, -OCH₂CO-, -COCH₂NH- or -NHCH₂CO-], their pharmaceutically acceptable salts and their hydrates, as effective ingredients.

25. An agonist against human peroxisome proliferator-activated receptor (PPAR)α having at least one or more kinds of substituted phenylpropionic acid derivatives represented by a general formula (1) 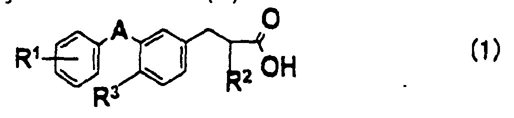 [wherein R¹ denotes a alkyl group with carbon atoms of 1 to 4, alkoxy group with carbon atoms of 1 to 3, trifluoromethyl group, trifluoromethoxy group, phenyl group which is unsubstituted or may have substituents, phenoxy group which is unsubstituted or may have substituents or benzyloxy group which is unsubstituted or may have substituents, R² denotes a hydrogen atom, alkyl group with carbon atoms of 1 to 4 or alkoxy group with carbon atoms of 1 to 3, R³ denotes a alkoxy group with carbon atoms of 1 to 3, and the binding mode of A portion denotes -CH₂NHCH₂-, -COCH₂O-, -OCH₂CO-, -COCH₂NH- or -NHCH₂CO-], their pharmaceutically acceptable salts and their hydrates, as effective ingredients.

26. A therapeutic drug for the arterial sclerosis having at least one or more kinds of substituted phenylpropionic acid derivatives represented by a general formula (1) 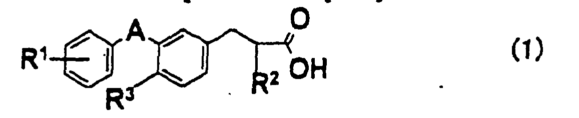 [wherein R¹ denotes a alkyl group with carbon atoms of 1 to 4, alkoxy group with carbon atoms of 1 to 3, trifluoromethyl group, trifluoromethoxy group, phenyl group which is unsubstituted or may have substituents, phenoxy group which is unsubstituted or may have substituents or benzyloxy group which is unsubstituted or may have substituents, R² denotes a hydrogen atom, alkyl group with carbon atoms of 1 to 4 or alkoxy group with carbon atoms of 1 to 3, R³ denotes a alkoxy group with carbon atoms of 1 to 3, and the binding mode of A portion denotes -CH₂NFCH₂-, -COCH₂O-, -OCH₂CO-, -COCH₂NH- or -NHCH₂CO-], their pharmaceutically acceptable salts and their hydrates, as effective ingredients.

27. A lipid-decreasing drug having at least one or more kinds of substituted phenylpropionic acid derivatives, their pharmaceutically acceptable salts and their hydrates of Claim 1, the steric configuration of R² substituent being as a general formula (1a) 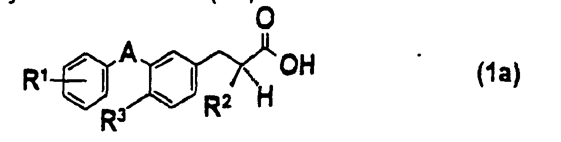 [wherein R¹ denotes a alkyl group with carbon atoms of 1 to 4, alkoxy group with carbon atoms of 1 to 3, trifluoromethyl group, trifluoromethoxy group, phenyl group which is unsubstituted or may have substituents, phenoxy group which is unsubstituted or may have substituents or benzyloxy group which is unsubstituted or may have substituents, R² denotes a alkyl group with carbon atoms of 1 to 4 or alkoxy group with carbon atoms of 1 to 3, R³ denotes a alkoxy group with carbon atoms of 1 to 3, and the binding mode of A portion denotes -CH₂CONH-, -NHCOCH₂-, -CH₂CH₂CH₂-, -CH₂CH₂O-, -CONECH₂-, -CH₂NHCH₂-, -COCH2O-, -OCH₂CO-, -COCH₂NH- or -NECH₂CO-], as effective ingredients.

28. An agonist against human peroxisome proliferator-activated receptor (PPAR)α having at least one or more kinds of substituted phenylpropionic acid derivatives, their pharmaceutically acceptable salts and their hydrates of Claim 1, the steric configuration of R² substituent being as a general formula (1a) 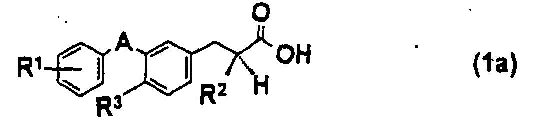 [wherein R¹ denotes a alkyl group with carbon atoms of 1 to 4, alkoxy group with carbon atoms of 1 to 3, trifluoromethyl group, trifluoromethoxy group, phenyl group which is unsubstituted or may have substituents, phenoxy group which is unsubstituted or may have substituents or benzyloxy group which is unsubstituted or may have substituents, R² denotes a alkyl group with carbon atoms of 1 to 4 or alkoxy group with carbon atoms of 1 to 3, R³ denotes a alkoxy group with carbon atoms of 1 to 3, and the binding mode of A portion denotes -CH₂CONH-, -NHCOCH₂-, -CH₂CH₂CH₂-, -CH₂CH₂O-, -CONHCH₂-, -CH₂NHCH₂-, -COCH2O-, -OCH₂CO-, -COCH₂NH- or -NHCH₂CO-] as effective ingredients.

29. A therapeutic drug for the arterial sclerosis having at least one or more kinds of substituted phenylpropionic acid derivatives, their pharmaceutically acceptable salts and their hydrates of Claim 1, the steric configuration of R² substituent being as a general formula (1a) 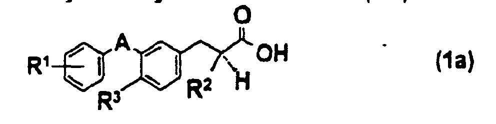 [wherein R¹ denotes a alkyl group with carbon atoms of 1 to 4, alkoxy group with carbon atoms of 1 to 3, trifluoromethyl group, trifluoromethoxy group, phenyl group which is unsubstituted or may have substituents, phenoxy group which is unsubstituted or may have substituents or benzyloxy group which is unsubstituted or may have substituents, R² denotes a alkyl group with carbon atoms of 1 to 4 or alkoxy group with carbon atoms of 1 to 3, R³ denotes a alkoxy group with carbon atoms of 1 to 3, and the binding mode of A portion denotes -CH₂CONH-, -NHCOCH₂-, -CH₂CH₂CH₂-, -CH₂CH₂O-, -CONHCH₂-, -CH₂NHCH₂- -COCH₂O-, -OCH₂CO-, -COCH₂NH- or -NHCH₂CO-] as effective ingredients.

## Patentansprüche

1. Substituierte Phenylpropionsäure-Derivate der allgemeinen Formel (1) 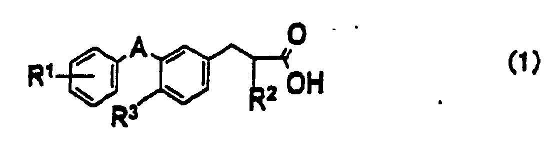 [worin R¹ eine Alkylgruppe mit 1 bis 4 Kohlenstoffatomen, Alkoxygruppe mit 1 bis 3 Kohlenstoffatomen, Trifluormethylgruppe, Trifluormethoxygruppe, Phenylgruppe, welche unsubstituiert ist oder Susbtituenten aufweisen kann, Phenoxygruppe, welche unsubstituiert ist oder Substituenten aufweisen kann, oder Benzyloxygruppe, welche unsubstituiert ist oder Substituenten aufweisen kann, bedeutet, R² ein Wasserstoffatom, eine Alkylgruppe mit 1 bis 4 Kohlenstoffatomen oder Alkoxygruppe mit 1 bis 3 Kohlenstoffatomen bedeutet, R³ eine Alkoxygruppe mit 1 bis 3 Kohlenstoffatomen bedeutet, und der Bindungsmodus des A-Teils -CH₂NHCH₂-, -COCH₂O-, OCH₂CO-, -COCH₂NH- oder -NHCH₂CO- bedeutet], deren pharmazeutisch annehmbaren Salze und deren Hydrate.

2. Substituierte Phenylpropionsäure-Derivate, deren pharmazeutisch annehmbaren Salze und deren Hydrate nach Anspruch 1, wobei die sterische Konfiguration des R²-Substituenten der allgemeinen Formel (1a) entspricht 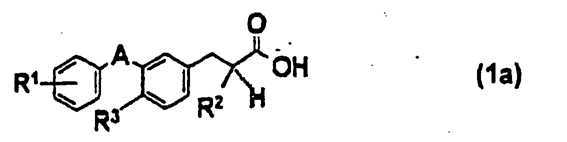 [worin R¹ eine Alkylgruppe mit 1 bis 4 Kohlenstoffatomen. Alkoxygruppe mit 1 bis 3 Kohlenstoffatomen, Trifluormethylgruppe, Trifluormethoxygruppe, Phenylgruppe, welche unsubstituiert ist oder Susbtituenten aufweisen kann, Phenoxygruppe, welche unsubstituiert ist oder Substituenten aufweisen kann, oder Benzyloxygruppe, welche unsubstituiert ist oder Substituenten aufweisen kann, bedeutet, R² eine Alkylgruppe mit 1 bis 4 Kohlenstoffatomen oder Alkoxygruppe mit 1 bis 3 Kohlenstoffatomen bedeutet, R³ eine Alkoxygruppe mit 1 bis 3 Kohlenstoffatomen bedeutet, und der Bindungsmodus des A-Teils -CH₂CONH-, -NHCONH₂-, -CH₂CH₂CH₂-, -CH₂CH₂O-, -CONHCH₂-, -CH₂NHCH₂-, -COCH₂O-, -OCH₂CO-, -COCH₂NH- oder -NHCH₂CO- bedeutet].

3. Substituierte Phenylpropionsäure-Derivate, deren pharmazeutisch annehmbaren Salze und deren Hydrate nach Anspruch 1, wobei R¹ eine Trifluormethylgruppe ist.

4. Substituierte Phenylpropionsäure-Derivate, deren pharmazeutisch annehmbaren Salze und deren Hydrate nach Anspruch 1, wobei R¹ eine Benzyloxygruppe ist.

5. Substituierte Phenylpropionsäure-Derivate, deren pharmazeutisch annehmbaren Salze und deren Hydrate nach Anspruch 1, wobei R¹ eine Phenoxygruppe ist.

6. Substituierte Phenylpropionsäure-Derivate, deren pharmazeutisch annehmbaren Salze und deren Hydrate nach Anspruch 1, wobei R¹ eine 4-Fluorphenoxygruppe ist.

7. Substituierte Phenylpropionsäure-Derivate, deren pharmazeutisch annehmbaren Salze und deren Hydrate nach Anspruch 1, wobei R² eine Ethylgruppe ist.

8. Substituierte Phenylpropionsäure-Derivate, deren pharmazeutisch annehmbaren Salze und deren Hydrate nach Anspruch 1, wobei R² eine n-Propylgruppe ist.

9. Substituierte Phenylpropionsäure-Derivate, deren pharmazeutisch annehmbaren Salze und deren Hydrate nach Anspruch 1, wobei R² eine Methoxygruppe ist.

10. Substituierte Phenylpropionsäure-Derivate, deren pharmazeutisch annehmbaren Salze und deren Hydrate nach Anspruch 1, wobei eines dieser 2-Ethyl-3-[4-methoxy-3-[2-[4-(trifluormethyl)phenyl]acetylamino]phenyl]propionsäure ist.

11. Substituierte Phenylpropionsäure-Derivate, deren pharmazeutisch annehmbaren Salze und deren Hydrate nach Anspruch 1, wobei eines dieser 2-n-Propyl-3-[4-methoxy-3-[2-[4-(trifluormethyl)phenyl]acetylamino]phenyl]propionsäure ist.

12. Substituierte Phenylpropionsäure-Derivate, deren pharmazeutisch annehmbaren Salze und deren Hydrate nach Anspruch 1, wobei eines dieser 2-Ethyl-3-(4-methoxy-3-[2-(4-phenoxyphenyl)acetylamino)phenyl]propionsäure ist.

13. Substituierte Phenylpropionsäure-Derivate, deren pharmazeutisch annehmbaren Salze und deren Hydrate nach Anspruch 1, wobei eines dieser 2-n-Propyl-3-[4-methoxy-3-[2-(4-phenoxyphenyl)acetylamino]phenyl]propionsäure ist.

14. Substituierte Phenylpropionsäure-Derivate, deren pharmazeutisch annehmbaren Salze und deren Hydrate nach Anspruch 1, wobei eines dieser 2-Ethyl-3-[4-methoxy-3-[2-[4-(4-fluorphenoxy)phenyl]acetylamino]phenyl]propionsäure ist.

15. Substituierte Phenylpropionsäure-Derivate, deren pharmazeutisch annehmbaren Salze und deren Hydrate nach Anspruch 1, wobei eines dieser 2-n-Propyl-3-[4-methoxy-3-[2-[4-(4-fluorphenoxy)phenyl]acetylamino]phenyl]propionsäure ist.

16. Substituierte Phenylpropionsäure-Derivate, deren pharmazeutisch annehmbaren Salze und deren Hydrate nach Anspruch 1, wobei eines dieser 2-n-Propyl-3-[4-methoxy-3-[[N-[4-(trifluormethyl)phenyl]carbamoyl]methyl]phenyl]propionsäure ist.

17. Substituierte Phenylpropionsäure-Derivate, deren pharmazeutisch annehmbaren Salze und deren Hydrate nach Anspruch 1, wobei eines dieser 2-Ethyl-3-[4-methoxy-3-[[N-(4-phenoxyphenyl)carbamoyl]methyl]phenyl]propionsäure ist.

18. Substituierte Phenylpropionsäure-Derivate, deren pharmazeutisch annehmbaren Salze und deren Hydrate nach Anspruch 1, wobei eines dieser 2-n-Propyl-3-[4-methoxy-3-[[N-(4-phenoxyphenyl)carbamoyl]methyl]phenyl]propionsäure ist.

19. Substituierte Phenylpropionsäure-Derivate, deren pharmazeutisch annehmbaren Salze und deren Hydrate nach Anspruch 1, wobei eines dieser 2-Ethyl-3-[4-methoxy-3-[[N-[4-(4-fluorphenoxy)phenyl]carbamoyl]methyl]phenyl]propionsäure ist.

20. Substituierte Phenylpropionsäure-Derivate, deren pharmazeutisch annehmbaren Salze und deren Hydrate nach Anspruch 1, wobei eines dieser 2-n-Propyl-3-[4-methoxy-3-[[N-[4-(4-fluorphenoxy)phenyl]carbamoyl]methyl]phenyl]propionsäure ist.

21. Substituierte Phenylpropionsäure-Derivate, deren pharmazeutisch annehmbaren Salze und deren Hydrate nach Anspruch 1, wobei eines dieser 2-Ethyl-3-[3-[4-(trifluormethyl)benzoylaminomethyl]-4-methoxyphenyl]propionsäure ist.

22. Substituierte Phenylpropionsäure-Derivate, deren pharmazeutisch annehmbaren Salze und deren Hydrate nach Anspruch 1, wobei eines dieser 2-Ethyl-3-[3-[4-(phenoxy)benzoylaminomethyl]-4-methoxyphenyl]propionsäure ist.

23. Substituierte Phenylpropionsäure-Derivate, deren pharmazeutisch annehmbaren Salze und deren Hydrate nach Anspruch 1, wobei eines dieser 2-Ethyl-3-[3-[4-(4-fluorphenoxy)benzoylaminomethyl]-4-methoxyphenyl]propionsäure ist.

24. Lipidsenkendes Arzneimittel, das als wirksame Bestandteile mindestens eine oder mehrere Arten substituierter Phenylpropionsäure-Derivate der allgemeinen Formel (1) 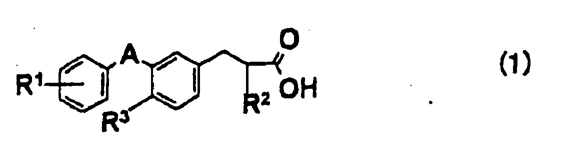 [worin R¹ eine Alkylgruppe mit 1 bis 4 Kohlenstoffatomen, Alkoxygruppe mit 1 bis 3 Kohlenstoffatomen, Trifluormethylgruppe, Trifluormethoxygruppe, Phenylgruppe, welche unsubstituiert ist oder Susbtituenten aufweisen kann, Phenoxygruppe, welche unsubstituiert ist oder Substituenten aufweisen kann, oder Benzyloxygruppe, welche unsubstituiert ist oder Substituenten aufweisen kann, bedeutet, R² ein Wasserstoffatom, eine Alkylgruppe mit 1 bis 4 Kohlenstoffatomen oder Alkoxygruppe mit 1 bis 3 Kohlenstoffatomen bedeutet, R³ eine Alkoxygruppe mit 1 bis 3 Kohlenstoffatomen bedeutet, und der Bindungsmodus des A-Teils -CH₂NHCH₂-, -COCH₂O-, OCH₂CO-, -COCH₂NH- oder -NHCH₂CO- bedeutet], deren pharmazeutisch annehmbaren Salze und deren Hydrate enthält.

25. Agonist gegenüber menschlichem Peroxisom-Proliferator-aktiviertem Rezeptor (PPAR)α, der als wirksame Bestandteile mindestens eine oder mehrere Arten substituierter Phenylpropionsäure-Derivate der allgemeinen Formel (1) 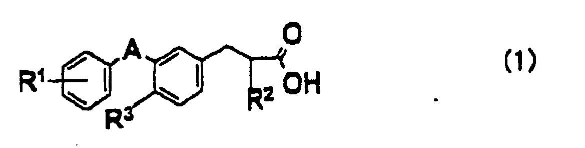 [worin R¹ eine Alkylgruppe mit 1 bis 4 Kohlenstoffatomen, Alkoxygruppe mit 1 bis 3 Kohlenstoffatomen, Trifluormethylgruppe, Trifluormethoxygruppe, Phenylgruppe, welche unsubstituiert ist oder Susbtituenten aufweisen kann, Phenoxygruppe, welche unsubstituiert ist oder Substituenten aufweisen kann, oder Benzyloxygruppe, welche unsubstituiert ist oder Substituenten aufweisen kann, bedeutet, R² ein Wasserstoffatom, eine Alkylgruppe mit 1 bis 4 Kohlenstoffatomen oder Alkoxygruppe mit 1 bis 3 Kohlenstoffatomen bedeutet, R³ eine Alkoxygruppe mit 1 bis 3 Kohlenstoffatomen bedeutet, und der Bindungsmodus des A-Teils -CH₂NHCH₂-, -COCH₂O-, OCH₂CO-, -COCH₂NH- oder -NHCH₂CO- bedeutet], deren pharmazeutisch annehmbaren Salze und deren Hydrate enthält.

26. Therapeutisches Arzneimittel für Arteriosklerose, das als wirksame Bestandteile mindestens eine oder mehrere Arten substituierter Phenylpropionsäure-Derivate der allgemeinen Formel (1) 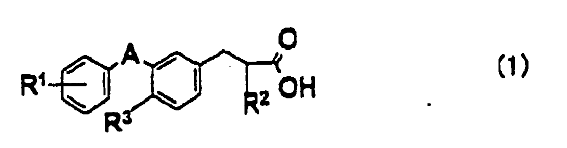 [worin R¹ eine Alkylgruppe mit 1 bis 4 Kohlenstoffatomen, Alkoxygruppe mit 1 bis 3 Kohlenstoffatomen, Trifluormethylgruppe, Trifluormethoxygruppe, Phenylgruppe, welche unsubstituiert ist oder Susbtituenten aufweisen kann, Phenoxygruppe, welche unsubstituiert ist oder Substituenten aufweisen kann, oder Benzyloxygruppe, welche unsubstituiert ist oder Substituenten aufweisen kann, bedeutet, R² ein Wasserstoffatom, eine Alkylgruppe mit 1 bis 4 Kohlenstoffatomen oder Alkoxygruppe mit 1 bis 3 Kohlenstoffatomen bedeutet, R³ eine Alkoxygruppe mit 1 bis 3 Kohlenstoffatomen bedeutet, und der Bindungsmodus des A-Teils -CH₂NHCH₂-, -COCH₂O-, OCH₂CO-, -COCH₂NH- oder -NHCH₂CO- bedeutet], deren pharmazeutisch annehmbaren Salze und deren Hydrate enthält.

27. Lipidsenkendes Arzneimittel, das als wirksame Bestandteile mindestens eine oder mehrere Arten substituierter Phenylpropionsäure-Derivate, deren pharmazeutisch annehmbaren Salze und deren Hydrate gemäß Anspruch 1 enthält, wobei die sterische Konfiguration des R²-Substituenten der allgemeinen Formel (1a) entspricht 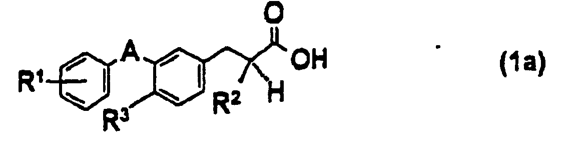 [worin R¹ eine Alkylgruppe mit 1 bis 4 Kohlenstoffatomen, Alkoxygruppe mit 1 bis 3 Kohlenstoffatomen, Trifluormethylgruppe, Trifluormethoxygruppe, Phenylgruppe, welche unsubstituiert ist oder Susbtituenten aufweisen kann, Phenoxygruppe, welche unsubstituiert ist oder Substituenten aufweisen kann, oder Benzyloxygruppe, welche unsubstituiert ist oder Substituenten aufweisen kann, bedeutet, R² eine Alkylgruppe mit 1 bis 4 Kohlenstoffatomen oder Alkoxygruppe mit 1 bis 3 Kohlenstoffatomen bedeutet, R³ eine Alkoxygruppe mit 1 bis 3 Kohlenstoffatomen bedeutet, und der Bindungsmodus des A-Teils -CH₂CONH-, -NHCONH₂-, -CH₂CH₂CH₂-, -CH₂CH₂O-, -CONHCH₂-, -CH₂NHCH₂-, -COCH₂O-, -OCH₂CO-, -COCH₂NH- oder -NHCH₂CO- bedeutet].

28. Agonist gegenüber menschlichem Peroxisom-Proliferator-aktiviertem Rezeptor (PPAR)α, der als wirksame Bestandteile mindestens eine oder mehrere Arten substituierter Phenylpropionsäure-Derivate, deren pharmazeutisch annehmbaren Salze und deren Hydrate gemäß Anspruch 1 enthält, wobei die sterische Konfiguration des R²-Substituenten der allgemeinen Formel (1a) entspricht 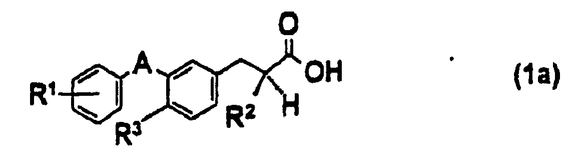 [worin R¹ eine Alkylgruppe mit 1 bis 4 Kohlenstoffatomen, Alkoxygruppe mit 1 bis 3 Kohlenstoffatomen, Trifluormethylgruppe, Trifluormethoxygruppe, Phenylgruppe, welche unsubstituiert ist oder Susbtituenten aufweisen kann, Phenoxygruppe, welche unsubstituiert ist oder Substituenten aufweisen kann, oder Benzyloxygruppe, welche unsubstituiert ist oder Substituenten aufweisen kann, bedeutet, R² eine Alkylgruppe mit 1 bis 4 Kohlenstoffatomen oder Alkoxygruppe mit 1 bis 3 Kohlenstoffatomen bedeutet, R³ eine Alkoxygruppe mit 1 bis 3 Kohlenstoffatomen bedeutet, und der Bindungsmodus des A-Teils -CH₂CONH-, -NHCONH₂-, -CH₂CH₂CH₂-, -CH₂CH₂O-, -CONHCH₂-, -CH₂NHCH₂-, -COCH₂O-, -OCH₂CO-, -COCH₂NH- oder -NHCH₂CO- bedeutet].

29. Therapeutisches Arzneimittel für Arteriosklerose, das als wirksame Bestandteile mindestens eine oder mehrere Arten substituierter Phenylpropionsäure-Derivate, deren pharmazeutisch annehmbaren Salze und deren Hydrate gemäß Anspruch 1 enthält, wobei die sterische Konfiguration des R²-Substituenten der allgemeinen Formel (1a) entspricht 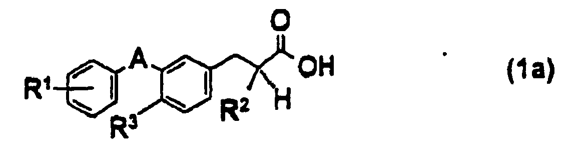 [worin R¹ eine Alkylgruppe mit 1 bis 4 Kohlenstoffatomen, Alkoxygruppe mit 1 bis 3 Kohlenstoffatomen, Trifluormethylgruppe, Trifluormethoxygruppe, Phenylgruppe, welche unsubstituiert ist oder Susbtituenten aufweisen kann, Phenoxygruppe, welche unsubstituiert ist oder Substituenten aufweisen kann, oder Benzyloxygruppe, welche unsubstituiert ist oder Substituenten aufweisen kann, bedeutet, R². eine Alkylgruppe mit 1 bis 4 Kohlenstoffatomen oder Alkoxygruppe mit 1 bis 3 Kohlenstoffatomen bedeutet, R³ eine Alkoxygruppe mit 1 bis 3 Kohlenstoffatomen bedeutet, und der Bindungsmodus des A-Teils -CH₂CONH-, -NHCONH₂-, -CH₂CH₂CH₂-, -CH₂CH₂O-, -CONHCH₂-, -CH₂NHCH₂-, -COCH₂O-, -OCH₂CO-, -COCH₂NH- oder -NHCH₂CO- bedeutet].

## Revendications

1. Dérivés d'acide phénylpropionique substitués représentés par la formule générale (1) : 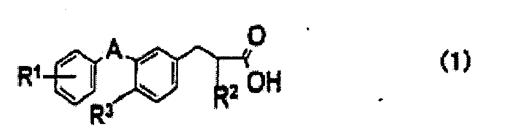 [dans laquelle R¹ représente un groupe alkyle avec 1 à 4 atomes de carbone, un groupe alcoxy avec 1 à 3 atomes de carbone, un groupe trifluorométhyle, un groupe trifluorométhoxy, un groupe phényle qui est non substitué ou qui peut comporter des substituants, un groupe phénoxy qui est non substitué ou qui peut comporter des substituants ou un groupe benzyloxy qui est non substitué ou qui peut comporter des substituants, R² représente un atome d'hydrogène, un groupe alkyle avec 1 à 4 atomes de carbone ou un groupe alcoxy avec 1 à 3 atomes de carbone, R³ représente un groupe alcoxy avec 1 à 3 atomes de carbone, et le mode de liaison de la partie A représente un groupe -CH₂NHCH₂-, -COCH₂O-, -OCH₂CO-, -COCH₂NH- ou -NHCH₂CO-], leurs sels pharmaceutiquement acceptables et leur hydrates.

2. Dérivés d'acide phénylpropionique substitués, leurs sels pharmaceutiquement acceptables et leurs hydrates suivant la revendication 1, la configuration stérique du substituant R² répondant à la formule générale (1a) : 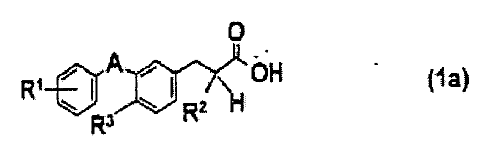 [dans laquelle R¹ représente un groupe alkyle avec 1 à 4 atomes de carbone, un groupe alcoxy avec 1 à 3 atomes de carbone, un groupe trifluorométhyle, un groupe trifluorométhoxy, un groupe phényle qui est non substitué ou qui peut comporter des substituants, un groupe phénoxy qui est non substitué ou qui peut comporter des substituants ou un groupe benzyloxy qui est non substitué ou qui peut comporter des substituants, R² représente un groupe alkyle avec 1 à 4 atomes de carbone ou un groupe alcoxy avec 1 à 3 atomes de carbone, R³ représente un groupe alcoxy avec 1 à 3 atomes de carbone, et le mode de liaison de la partie A représente un groupe -CH₂CONH-, -NHCOCH₂-, -CH₂CH₂CH₂-, -CH₂CH₂O-, -CONHCH₂-, -CH₂NHCH₂-, -COCH₂O-, -OCH₂CO-, -COH₂NH- ou -NHCH₂CO-].

3. Dérivés d'acide phénylpropionique substitués, leurs sels pharmaceutiquement acceptables et leurs hydrates suivant la revendication 1, R¹ représentant un groupe trifluorométhyle.

4. Dérivés d'acide phénylpropionique substitués, leurs sels pharmaceutiquement acceptables et leurs hydrates suivant la revendication 1, R¹ représentant un groupe benzyloxy.

5. Dérivés d'acide phénylpropionique substitués, leurs sels pharmaceutiquement acceptables et leurs hydrates suivant la revendication 1, R¹ représentant un groupe phénoxy.

6. Dérivés d'acide phénylpropionique substitués, leurs sels pharmaceutiquement acceptables et leurs hydrates suivant la revendication 1, R¹ représentant un groupe 4-fluorophénoxy.

7. Dérivés d'acide phénylpropionique substitués, leurs sels pharmaceutiquement acceptables et leurs hydrates suivant la revendication 1, R² représentant un groupe éthyle.

8. Dérivés d'acide phénylpropionique substitués, leurs sels pharmaceutiquement acceptables et leurs hydrates suivant la revendication 1, R² représentant un groupe n-propyle.

9. Dérivés d'acide phénylpropionique substitués, leurs sels pharmaceutiquement acceptables et leurs hydrates suivant la revendication 1, R² représentant un groupe méthoxy.

10. Dérivés d'acide phénylpropionique substitués, leurs sels pharmaceutiquement acceptables et leurs hydrates suivant la revendication 1, l'un d'entre eux étant l'acide 2-éthyl-3-[4-méthoxy-3-[2-[4-(trifluorométhyl)phényl]acétylamino]phényl]propionique.

11. Dérivés d'acide phénylpropionique substitués, leurs sels pharmaceutiquement acceptables et leurs hydrates suivant la revendication 1, l'un d'entre eux étant l'acide 2-n-propyl-3-[4-méthoxy-3-[2-[4-(trifluorométhyl)phényl]acétylamino]phényl]propionique.

12. Dérivés d'acide phénylpropionique substitués, leurs sels pharmaceutiquement acceptables et leurs hydrates suivant la revendication 1, l'un d'entre eux étant l'acide 2-éthyl-3-[4-méthoxy-3-[2-(4-phénoxyphényl)acétylamino]phényl]propionique.

13. Dérivés d'acide phénylpropionique substitués, leurs sels pharmaceutiquement acceptables et leurs hydrates suivant la revendication 1, l'un d'entre eux étant l'acide 2-n-propyl-3-[4-méthoxy-3-[2-(4-phénoxyphényl)acétylamino]phényl]propionique.

14. Dérivés d'acide phénylpropionique substitués, leurs sels pharmaceutiquement acceptables et leurs hydrates suivant la revendication 1, l'un d'entre eux étant l'acide 2-éthyl-3-[4-méthoxy-3-[2-[4-(4-fluorophénoxy)phényl]acétylamino]phényl]propionique.

15. Dérivés d'acide phénylpropionique substitués, leurs sels pharmaceutiquement acceptables et leurs hydrates suivant la revendication 1, l'un d'entre eux étant l'acide 2-n-propyl-3-[4-méthoxy-3-[2-[4-(4-fluorophénoxy)phényl]acétylamino]phényl]propionique.

16. Dérivés d'acide phénylpropionique substitués, leurs sels pharmaceutiquement acceptables et leurs hydrates suivant la revendication 1, l'un d'entre eux étant l'acide 2-n-propyl-3-[4-méthoxy-3-[[N-4-(trifluorométhyl)phényl]carbamoyl]méthyl]phényl]propionique.

17. Dérivés d'acide phénylpropionique substitués, leurs sels pharmaceutiquement acceptables et leurs hydrates suivant la revendication 1, l'un d'entre eux étant l'acide 2-éthyl-3-[4-méthoxy-3-[[N-(4-phénoxyphényl)carbamoyl]méthyl]phényl]propionique.

18. Dérivés d'acide phénylpropionique substitués, leurs sels pharmaceutiquement acceptables et leurs hydrates suivant la revendication 1, l'un d'entre eux étant l'acide 2-n-propyl-3-[4-méthoxy-3-[[N-(4-phénoxyphényl)carbamoyl]méthyl]phényl]propionique.

19. Dérivés d'acide phénylpropionique substitués, leurs sels pharmaceutiquement acceptables et leurs hydrates suivant la revendication 1, l'un d'entre eux étant l'acide 2-éthyl-3-[4-méthoxy-3-[[N-[4-(4-fluorophénoxy)phényl]carbamoyl]méthyl]phényl]propionique.

20. Dérivés d'acide phénylpropionique substitués, leurs sels pharmaceutiquement acceptables et leurs hydrates suivant la revendication 1, l'un d'entre eux étant l'acide 2-n-propyl-3-[4-méthoxy-3-[[N[4-(4-fluorophénoxy)phényl]carbamoyl]méthyl]phényl]propionique.

21. Dérivés d'acide phénylpropionique substitués, leurs sels pharmaceutiquement acceptables et leurs hydrates suivant la revendication 1, l'un d'entre eux étant l'acide 2-éthyl-3-[3-[4-(trifluorométhyl)benzoylaminométhyl]-4-méthoxyphényl]propionique.

22. Dérivés d'acide phénylpropionique substitués, leurs sels pharmaceutiquement acceptables et leurs hydrates suivant la revendication 1, l'un d'entre eux étant l'acide 2-éthyl-3-[3-[4-(phénoxy)benzoylaminométhyl]-4-méthoxyphényl]propionique.

23. Dérivés d'acide phénylpropionique substitués, leurs sels pharmaceutiquement acceptables et leurs hydrates suivant la revendication 1, l'un d'entre eux étant l'acide 2-éthyl-3-[3-[4-(4-fluorophénoxy)benzoylaminométhyl]-4-méthoxyphényl]propionique.

24. Médicament hypolipémiant contenant au moins un ou plusieurs types de dérivés d'acide phénylpropionique substitués représentés par la formule générale (1) : 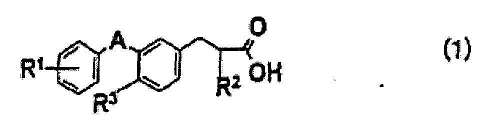 [dans laquelle R¹ représente un groupe alkyle avec 1 à 4 atomes de carbone, un groupe alcoxy avec 1 à 3 atomes de carbone, un groupe trifluorométhyle, un groupe trifluorométhoxy, un groupe phényle qui est non substitué ou qui peut comporter des substituants, un groupe phénoxy qui est non substitué ou qui peut comporter des substituants ou un groupe benzyloxy qui est non substitué ou qui peut comporter des substituants, R² représente un atome d'hydrogène, un groupe alkyle avec 1 à 4 atomes de carbone ou un groupe alcoxy avec 1 à 3 atomes de carbone, R³ représente un groupe alcoxy avec 1 à 3 atomes de carbone, et le mode de liaison de la partie A représente un groupe -CH₂NHCH₂-, -COCH₂O-, -OCH₂CO-, -COCH₂NH- ou -NHCH₂CO-], leurs sels pharmaceutiquement acceptables et leur hydrates, comme ingrédients efficaces.

25. Agoniste contre le récepteur activé par le proliférateur de peroxysome humain (PPAR)-α contenant au moins un ou plusieurs types de dérivés d'acide phénylpropionique substitués représentés par la formule générale (1) : 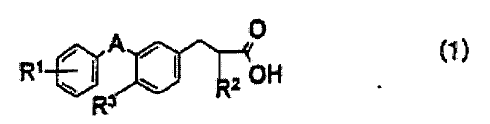 [dans laquelle R¹ représente un groupe alkyle avec 1 à 4 atomes de carbone, un groupe alcoxy avec 1 à 3 atomes de carbone, un groupe trifluorométhyle, un groupe trifluorométhoxy, un groupe phényle qui est non substitué ou qui peut comporter des substituants, un groupe phénoxy qui est non substitué ou qui peut comporter des substituants ou un groupe benzyloxy qui est non substitué ou qui peut comporter des substituants, R² représente un atome d'hydrogène, un groupe alkyle avec 1 à 4 atomes de carbone ou un groupe alcoxy avec 1 à 3 atomes de carbone, R³ représente un groupe alcoxy avec 1 à 3 atomes de carbone, et le mode de liaison de la partie A représente un groupe -CH₂NHCH₂-, -COCH₂O-, -OCH₂CO-, -COCH₂NH- ou -NHCH₂CO-], leurs sels pharmaceutiquement acceptables et leur hydrates, comme ingrédients efficaces.

26. Médicament thérapeutique pour l'artériosclérose contenant au moins un ou plusieurs types de dérivés d'acide phénylpropionique substitués représentés par la formule générale (1) : 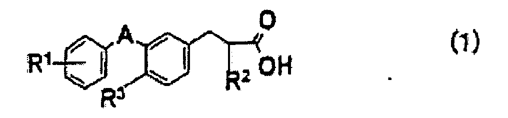 [dans laquelle R¹ représente un groupe alkyle avec 1 à 4 atomes de carbone, un groupe alcoxy avec 1 à 3 atomes de carbone, un groupe trifluorométhyle, un groupe trifluorométhoxy, un groupe phényle qui est non substitué ou qui peut comporter des substituants, un groupe phénoxy qui est non substitué ou qui peut comporter des substituants ou un groupe benzyloxy qui est non substitué ou qui peut comporter des substituants, R² représente un atome d'hydrogène, un groupe alkyle avec 1 à 4 atomes de carbone ou un groupe alcoxy avec 1 à 3 atomes de carbone, R³ représente un groupe alcoxy avec 1 à 3 atomes de carbone, et le mode de liaison de la partie A représente un groupe -CH₂NHCH₂-, -COCH₂O-, -OCH₂CO-, -COCH₂NH- ou -NHCH₂CO-], leurs sels pharmaceutiquement acceptables et leur hydrates, comme ingrédients efficaces.

27. Médicament hypolipémiant contenant au moins un ou plusieurs types de dérivés d'acide phénylpropionique substitués, leurs sels pharmaceutiquement acceptables et leurs hydrates suivant le revendication 1, la configuration stérique du substituant R² répondant à la formule générale (1a) : 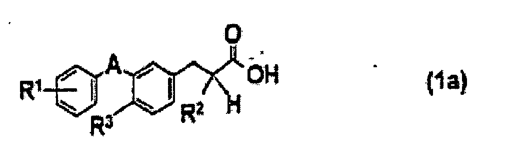 [dans laquelle R¹ représente un groupe alkyle avec 1 à 4 atomes de carbone, un groupe alcoxy avec 1 à 3 atomes de carbone, un groupe trifluorométhyle, un groupe trifluorométhoxy, un groupe phényle qui est non substitué ou qui peut comporter des substituants, un groupe phénoxy qui est non substitué ou qui peut comporter des substituants ou un groupe benzyloxy qui est non substitué ou qui peut comporter des substituants, R² représente un groupe alkyle avec 1 à 4 atomes de carbone ou un groupe alcoxy avec 1 à 3 atomes de carbone, R³ représente un groupe alcoxy avec 1 à 3 atomes de carbone, et le mode de liaison de la partie A représente un groupe -CH₂CONH-, -NHCOCH₂-, -CH₂CH₂CH₂-, -CH₂CH₂O-, -CONHCH₂-, -CH₂NHCH₂-, -COCH₂O-, -OCH₂CO-, -COCH₂NH- ou -NHCH₂CO-], comme ingrédients efficaces.

28. Agoniste contre le récepteur activé par le proliférateur de peroxysome humain (PPAR)-α contenant au moins un ou plusieurs types de dérivés d'acide phénylpropionique substitués, leurs sels pharmaceutiquement acceptables et leurs hydrates suivant la revendication 1, la configuration stérique du substituant R² répondant à la formule générale (1a) : 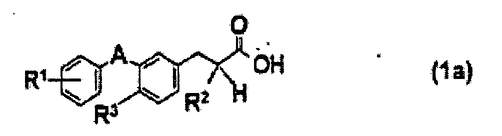 [dans laquelle R¹ représente un groupe alkyle avec 1 à 4 atomes de carbone, un groupe alcoxy avec 1 à 3 atomes de carbone, un groupe trifluorométhyle, un groupe trifluorométhoxy, un groupe phényle qui est non substitué ou qui peut comporter des substituants, un groupe phénoxy qui est non substitué ou qui peut comporter des substituants ou un groupe benzyloxy qui est non substitué ou qui peut comporter des substituants, R² représente un groupe alkyle avec 1 à 4 atomes de carbone ou un groupe alcoxy avec 1 à 3 atomes de carbone, R³ représente un groupe alcoxy avec 1 à 3 atomes de carbone, et le mode de liaison de la partie A représente un groupe -CH₂CONH-, -NHCOCH₂-, -CH₂CH₂CH₂-, -CH₂CH₂O-, -CONHCH₂-, -CH₂NHCH₂-, -COCH₂O-, -OCH₂CO-, -COCH₂NH- ou -NHCH₂CO-], comme ingrédients efficaces.

29. Médicament thérapeutique pour l'artériosclérose contenant au moins un ou plusieurs types de dérivés d'acide phénylpropionique substitués, leurs sels pharmaceutiquement acceptables et leurs hydrates suivant la revendication 1, la configuration stérique du substituant R² répondant à la formule générale (1a) : 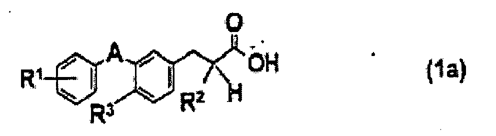 [dans laquelle R¹ représente un groupe alkyle avec 1 à 4 atomes de carbone, un groupe alcoxy avec 1 à 3 atomes de carbone, un groupe trifluorométhyle, un groupe trifluorométhoxy, un groupe phényle qui est non substitué ou qui peut comporter des substituants, un groupe phénoxy qui est non substitué ou qui peut comporter des substituants ou un groupe benzyloxy qui est non substitué ou qui peut comporter des substituants, R² représente un groupe alkyle avec 1 à 4 atomes de carbone ou un groupe alcoxy avec 1 à 3 atomes de carbone, R³ représente un groupe alcoxy avec 1 à 3 atomes de carbone, et le mode de liaison de la partie A représente un groupe -CH₂CONH-, -NHCOCH₂-, -CH₂CH₂CH₂-, -CH₂CH₂O-, -CONHCH₂-, -CH₂NHCH₂-, -COCH₂O-, -OCH₂CO-, -COCH₂NH- ou -NHCH₂CO-], comme ingrédients efficaces.
